# EUROPÄISCHE PATENTSCHRIFT

(11) **EP 3 530 755 B1**
(45) Veröffentlichungstag und Bekanntmachung des Hinweises auf die Patenterteilung: **26.08.2020**
(21) Anmeldenummer: 18158514.2
(22) Anmeldetag: 26.02.2018
(51) Int. Cl.: C12Q 1/6853

(54) **VERFAHREN ZUM ANZEIGEN DES FORTSCHRITTES DER AMPLIFIKATION VON NUKLEINSÄUREN UND KIT ZU DESSEN DURCHFÜHRUNG**
METHOD FOR DISPLAYING THE PROGRESS IN THE AMPLIFICATION OF NUCLEIC ACIDS AND KIT FOR SAME
PROCÉDÉ D'AFFICHAGE DU PROGRÈS DE L'AMPLIFICATION D'ACIDES NUCLÉIQUES ET KIT DE MISE EN UVRE DUDIT PROCÉDÉ

(43) Veröffentlichungstag der Anmeldung: 28.08.2019
(73) Patentinhaber: AGCT GmbH, 23562 Luebeck (DE)
(72) Erfinder: CHERKASOV, Dmitry, 35039 Marburg (DE); BASLER, Norbert, 22927 Großhansdorf (DE); BECKER, Claus, 76470 Ötigheim (DE); HESS, Hans-Jörg, Berlin 13467 (DE); MÜLLER-HERMANN, Andreas, 80796 München (DE); GRUNWALD, Christian, 35392 Gießen (DE)
(74) Vertreter: Keller, Günter

(56) Entgegenhaltungen:
- EP-A1- 3 287 528
- WO-A1-2007/030505
- WO-A1-2009/150467
- WO-A1-2012/058488
- WO-A1-2014/173963
- WO-A1-2015/075198
- WO-A2-2004/076683
- WO-A2-2015/161054
- I. V. SMOLINA: "End invasion of peptide nucleic acids (PNAs) with mixed-base composition into linear DNA duplexes", NUCLEIC ACIDS RESEARCH, Bd. 33, Nr. 17, 25. September 2005 (2005-09-25), Seiten e146-e146, XP055336681, ISSN: 0305-1048, DOI: 10.1093/nar/gni151

### SEQUENZPROTOKOLL

<110> AGCT GmbH
<120> Verfahren zum Anzeigen des Fortschrittes der Amplifikation von Nukleinsäuren und Kit zu dessen Durchführung
<130> PEP03000AGC
<160> 15
<170> PatentIn version 3.5
<210> 1
   <211> 38
   <212> DNA
   <213> Homo sapiens
<400> 1
   gtaagagcag atccctggac aggcaaggaa tacaggta 38
<210> 2
   <211> 45
   <212> DNA
   <213> artificial sequence
<220>
   <223> primer
<400> 2
   aactcagaca agatgtgatt tttttacctg tattacctgt attcc 45
<210> 3
   <211> 70
   <212> DNA
   <213> artificial sequence
<220>
   <223> primer
<400> 3
   ctacagaact cagacaagat gtgaactaca atgttgctca tactacaatg tcacttactg 60
   taagagcaga 70
<210> 4
   <211> 58
   <212> DNA
   <213> artificial sequence
<220>
   <223> activator oligonucleotide
<400> 4
   taatctgtaa gagcagatcc ctggacaggc aaggaataca ggtagaagca tcagagaa 58
<210> 5
   <211> 52
   <212> DNA
   <213> artificial sequence
<220>
   <223> primer
<400> 5
   aagctcgaca aaagaactca gagtgtgctc gacactacct gtattccttg cc 52
<210> 6
   <211> 67
   <212> DNA
   <213> artificial sequence
<220>
   <223> activator oligonucleotide
<400> 6
   gctcatacta caatgtcact tactgtaaga gcagatccct ggacaggcaa ggaatacagg 60
   taaaaaa 67
<210> 7
   <211> 45
   <212> DNA
   <213> artificial sequence
<220>
   <223> primer
<400> 7
   aactcagaca agatgtgatt tttttacctg tattacctgt attcc 45
<210> 8
   <211> 70
   <212> DNA
   <213> artificial sequence
<220>
   <223> primer
<400> 8
   ctacagaact cagacaagat gtgaactaca atgttgctca tactacaatg tcacttactg 60
   taagagcaga 70
<210> 9
   <211> 58
   <212> DNA
   <213> artificial sequence
<220>
   <223> activator oligonucleotide
<400> 9
   taatctgtaa gagcagatcc ctggacaggc aaggaataca ggtagaagca tcagagaa 58
<210> 10
   <211> 48
   <212> DNA
   <213> artificial sequence
<220>
   <223> probe
<400> 10
   tagtatgagc ttttgctcat acacaatgtc acttactgta agagcaga 48
<210> 11
   <211> 67
   <212> DNA
   <213> artificial sequence
<220>
   <223> activator oligonucleotide
<400> 11
   gctcatacta caatgtcact tactgtaaga gcagatccct ggacaggcaa ggaatacagg 60
   taaaaaa 67
<210> 12
   <211> 67
   <212> DNA
   <213> artificial sequence
<220>
   <223> activator oligonucleotide
<400> 12
   gctcatacta caatgtcact tactgtaaga gcagatccct ggacaggcga ggaatacagg 60
   taaaaaa 67
<210> 13
   <211> 45
   <212> DNA
   <213> artificial sequence
<220>
   <223> primer
<400> 13
   aactcagaca agatgtgatt tttttacctg tattacctgt attcc 45
<210> 14
   <211> 70
   <212> DNA
   <213> artificial sequence
<220>
   <223> primer
<400> 14
   ctacagaact cagacaagat gtgaactaca atgttgctca tactacaatg tcacttactg 60
   taagagcaga 70
<210> 15
   <211> 58
   <212> DNA
   <213> artificial sequence
<220>
   <223> activator oligonucleotide
<400> 15
   taatctgtaa gagcagatcc ctggacaggc aaggaataca ggtagaagca tcagagaa 58

## Beschreibung

Die Synthese von Nukleinsäureketten nimmt heute eine zentrale Rolle in der Biotechnologie ein. Verfahren wie PCR haben sowohl die Forschungslandschaft als auch industrielle Applikationsfelder, wie Diagnostik in der Medizin und Lebensmittelindustrie, signifikant weiterentwickelt. Die Verbindung der PCR mit anderen Technologien, wie Sequenzierung, Real-Time-Nachweis, Microarray-Technologie, Mikrofluidic-Managment etc. hat zur technologischen Entwicklung der Basistechnologie beigetragen und einige Barrieren der Grundtechnologie der PCR teilweise überwinden können. Es wurden auch weitere Amplifikations-Verfahren, wie isothermale Amplifikationstechniken (LAMP, HDA, RPA, TMA, SDA etc.) entwickelt. Deren Einsatz wurde besonders für den Bereich von POCT vorgesehen.

Trotz enormer Fortschritte auf diesem Gebiet, nimmt PCR die zentrale Rolle ein und bestimmt somit die einzelnen technologischen Barrieren der Applikationen.

Eine der Eigenschaften von verbreiteten Amplifikationsverfahren, wie PCR, besteht darin, dass während des Amplifikationsvorgangs der Nukleinsäure keine Kontrolle der amplifizierten Sequenzabschnitte zwischen beiden Primern stattfindet. Im Wesentlichen steht die Primer-Bindung im Fokus von Optimierungen von PCR-Amplifikationsreaktionen. Zu Beginn der PCR-Amplifikation und in ihrem Verlauf kommt es kontinuiertlich zu mehr oder weniger spezifischen Primer-Bindungen und der Initiierung der Synthese von Hauptprodukten und Nebenprodukten. Die Nebenprodukte können beispielsweise als Folge eines unspezifischen Primer-Verlängerungsereignisses in einem Synthese-Zyklus generiert werden. Bei ggf. erfolgenden Rücksynthese-Reaktion wird der unspezifisch verlängerte Primer als Matrize abgelesen, was in der Regel zu Ausbildung einer vollständigen Primer-Bindungsstelle führt. Somit erfolgt eine Übertragung einer fehlerhaften Sequenzinformation von einem Synthese-Zyklus auf den nächsten Synthese-Zyklus, was in der Summe von Synthese-Zyklen nicht nur zu initialen Entstehung, sondern vor allem zur exponentiellen Vermehrung von Nebenprodukten führt.

Solche Nebenreaktionen können unter Umständen zu initialen Entstehung und exponentiellen Vermehrung von Fragmenten führen, welche die Hauptreaktion (Amplifikation einer Zielsequenz) stören bzw. zu Interfernzen in nachfolgenden Schritten der Analyse führen. Solche Nebenprodukte umfassen typischerweise Primersequenzen und entsprechende Primersequenzen, so dass ihre Amplifikation parallel zu der Hauptreaktion stattfinden kann. Anstatt einer Zielsequenz umfassen solche Nebenprodukte allerdings eine andere Nukleinsäuresequenz.

Vorrangig wird Spezifität der PCR Amplifikation durch Optimierung der Primer-Bindung an Zielsequenzen erreicht. Dabei können beispielsweise zusätzliche Oligonukleotide verwendet werden, welche an Primer teilweise binden können und somit bei Primer-Bindung an andere Nukleinsäureketten kompetetiv mitwirken. Solche Sonden binden in der Regel an einen Sequenz-Abschnitt des Primers und lassen am Primer einen einzelsträngigen Sequenzabschnitt frei, so dass der Primer mit diesem Abschnitt an die Zielnukleinsäure binden kann und eine Synthese Reaktion initiieren kann. Dabei soll die Spezifität einer Primer-Bindung dadurch verbessert werden, da Primer-Matrizen Mismatches durch solche Oligonukleotide kompetetiv verdrängt werden können. Im Ergebnis kann Spezifität der Initiierung von PCR-Reaktionen in der Regel verbessert werden. Die Wirkung solcher Oligonukleotide ist auf die Interaktion mit Primer-Sequenzen beschränkt. Solche zusätzlichen Oligonukleotide interagieren nicht mit der zu amplifizierenden Nukleinsäurekette in Abschnitten zwischen beiden Primern. Aufgrund eines molaren Überschusses an Primern kann es während einer Amplifikation dennoch zu unspezifischen Interaktionen von Primern mit Matrizen kommen. Falls es zu einem solchen unspezifischen Ereignis der Primer-Verlängerung kommt (Initiierung einer exponentiellen Nebenreaktion), kommt es zur Ausbildung einer vollfunktionsfähigen Primer-Bindungsstelle als Folge bzw. im Rahmen von Rücksynthese eines komplementären Stranges des Nebenproduktes. Das Vorliegen einer solchen vollständigen Primerbindungsstelle im Nebenprodukt führt zu einem Verlust der kompetetiven Wirkung von solchen zusätzlichen Oligonukleotiden auf Primer-Bindung. Kontrolle der Spezifität der Bindung eines Primers an die Matrize durch solche Oligonukleotide macht somit nur Initiierung einer Nebenreaktion unwahrscheinlicher, kann allerdings nach Entstehung eines Nebenproduktes seine expontielle Amplifikation kaum beeinflussen.

Im Allgemeinen kann eine Verbesserung der Spezifität der Synthese eines Amplifikationsverfahrens mit reduzierter Entstehung und Co-Amplifikation von Nebenprodukten, welche sich von Zielsequenz unterscheiden, beispielsweise zu einer Verbesserung von diagnostischen Verfahren beitragen.

Eine Aufgabe der vorliegenden Erfindung ist es, Verfahren und Komponenten bereitzustellen, welche die enzymatische Synthese und Amplifikation von Nukleinsäureketten ermöglichen. Es soll ein neues enzymatisches Verfahren und Komponenten für die Synthese von Nukleinsäureketten, sowie die Amplifikation von Nukleinsäureketten bereitgestellt werden, bei dem eine kontinuierliche (on-line) Signal-Erfassung bereitgestellt wird, wobei die Signal-Erfassung (Monitoring/Detektion) durch sequenzspezifische Oligonukleotid-Sonden vermittelt bzw. unterstützt wird.

Eine weitere Aufgabe der Erfindung ist es, ein Verfahren mit verbesserter Spezifität der Synthese von Zielnukleinsäureketten in einer exponentiellen Amplifikation bereitzustellen. Eine weitere Aufgabe der Erfindung ist es, Mittel für Umsetzung eines exponentiellen Amplifikationsverfahrens mit verbesserter Spezifität der Synthese zur Verfügung zu stellen. Mit dem erfindungsgemäßen Verfahren sollen Nukleinsäureketten mit definierter Sequenz-Zusammensetzung synthetisiert bzw. amplifiziert und detektiert werden können.

Die Aufgabe der Erfindung wird gelöst durch Bereitstellung von Amplifikationsverfahren und entsprechenden Mitteln zu seiner Ausführung. Die Ausführung des Amplifikationsverfahrens wurde in der PCT Anmeldung (PCT/EP2017/071011) und der europäischen Anmeldung (EP-A 16185624.0) bereits beschrieben. Zu Details der Ausführung der Amplifikation wird der Fachmann an diese Anmeldung verwiesen. Vorzugsweise erfolgt die Amplifikation als exponentielle Amplifikation, bei welcher neusynthetisierte Produkte beider Primer (Primer-Verlängerungsprodukte) als Matrizen für weitere Syntheseschritte auftreten. Dabei werden Primersequenzen zumindest teilweise kopiert, so dass komplementäre Primer-Bindungsstellen entstehen, welche unmittelbar nach ihrer Synthese als Sequenzsegmente eines Doppelstranges vorliegen. Im Amplifikatonsverfahren erfolgen Synthese-Schritte von beiden Strängen und Doppelstrang-Öffnungs-Schritten der neusynthetisierten Sequenzabschnitte in gegenseitiger Abwechselung. Eine hinreichende Doppelstrang-Trennung nach einer Synthese stellt eine wichtige Voraussetzung für weitere Synthese dar. Insgesamt kann eine solche Abwechslung aus Synthese- und Doppelstrang-Trennungsschritten zu einer exponentiellen Amplifikation führen.

Im erfindungsgemäßen Amplifikationsverfahren erfolgt die Doppelstrang-Öffnung von Hauptprodukten der Amplifikation (Amplifikation von Zielsequenz umfassenden Nukleinsäureketten) unter anderem mittels eines Oligonukleotides, genannt Aktivator-Oligonukleotid. Das Aktivator-Oligonukleotid umfasst vorzugsweise Sequenzsegmente, welche der Zielsequenz entsprechen.

Im Einzelnen, wird die Strangtrennung erfindungsgemäß durch Einsatz von Aktivator-Oligonukleotiden mit vordefinierten Sequenzen erreicht, welche vorzugsweise mittels einer sequenzabhängigen nukleinsäurevermittelten Strangverdrängung einen neusynthetisierten Doppelstrang bestehend aus beiden spezifischen Primer-Verlängerungsprodukten trennen. Die dabei entstehenden einzelsträngigen Segmente von Primer-Verlängerungsprodukten umfassen die Zielsequenz, sowie entsprechende Primer-Bindungsstellen, welche als Bindungsstellen für weitere Primer-Oligonukleotide dienen können, so dass eine exponentielle Amplifikation von zu amplifizierenden Nukleinsäureketten erreicht wird. Die Primer-Verlängerungsreaktionen und Strangverdrängungsreaktionen finden vorzugsweise im Ansatz gleichzeitig statt. Die Amplifikation erfolgt vorzugsweise unter Reaktionsbedingungen, welche eine spontane Trennung von beiden spezifischen synthetisierten Primer-Verlängerungsprodukten nicht zulassen.

Eine spezifische exponentielle Amplifikation einer Zielsequenz-umfassenden Nukleinsäurekette umfasst eine Wiederholung aus Synthese-Schritten und Doppelstrang-Öffnungs-Schritten (Aktivierungs-Schritten für Primer-Bindungsstellen) als zwingende Voraussetzung für Vermehrung der Nukleinsäurekette.

Die Öffnung von synthetisierten Doppelsträngen wird als Reaktions-Schritt umgesetzt, welcher sequenzspezifisch durch das Aktivator-Oligonukleotid beeinflusst werden soll. Diese Öffnung kann vollständig erfolgen, bis hin zu Dissoziation von beiden komplementären Primer-Verlängerungsprodukten, oder auch partiell sein.

Das Aktivator-Oligonukleotid umfasst erfindungsgemäß Sequenzabschnitte, welche mit der Zielsequenz interagieren können und weitere Sequenzabschnitte, welche diese Interaktion herbeiführen, bzw. ermöglichen, bzw. begünstigen. Im Rahmen der Interaktion mit dem Aktivator-Oligonukleotid werden doppelsträngige Abschnitte der synthetisierten Primer-Verlängerungsprodukte via sequenzspezifischer Strangverdrängung in die einzelsträngige Form überführt. Dieser Vorgang ist sequenzabhängig: erst wenn die Sequenz des synthetisieren Doppelstranges ein gewisses Maß an Komplementarität mit der entsprechenden Sequenz des Aktivator-Oligonukleotides aufweist, kommt es zu einer hinreichenden Doppelstrang-Öffnung, so dass die für die Fortführung der Synthese wesentlichen Sequenzabschnitte, wie z.B. Primer-Bindungsstellen, in einzelsträngige Form überführt werden, was einem "aktiven Zustand" entspricht. Das Aktivator-Oligonukleotid "aktiviert" somit spezifisch die neusynthetisierten Primer-Verlängerungsprodukte, welche die Zielsequenz umfassen, für weitere Synthese-Schritte.

Hingegen werden Sequenzabschnitte, welche keine Zielsequenz umfassen, nicht in einzelsträngigen Zustand überführt und verbleiben als Doppelstrang, was einem "inaktiven" Zustand entspricht. Die potenziellen Primerbindungsstellen sind in einem solchen Doppelstrang an Interaktion mit neuen Primern benachteiligt bzw. vollständig gehindert, so dass weitere Synthese-Schritte an solchen "nicht aktivierten" Strängen im Allgemeinen nicht stattfinden. Diese fehlende oder verminderte Aktivierung (d.h. Überführung in einen einzelsträngigen Zustand) von synthetisierten Nukleinsäuresträngen nach einem Synthese-Schritt führt dazu, dass im darauffolgenden Synthese-Schritt nur eine verminderte Menge an Primern an einer Primer-Verlängerungsreaktion erfolgreich teilnehmen kann.

Aufgrund einer anzustrebenden exponentiellen Amplifikation von Hauptprodukten (einer zu amplifizierenden Nukleinsäurekette, welche Zielsequenzen umfasst) werden mehrere Synthese-Schritte und Aktivierungs-Schritte (Doppelstrang-Öffnungs-Schritte) zu einem Amplifikations-Verfahren zusammengefasst und so lange ausgeführt, bzw. so oft wiederholt, bis die gewünschte Menge der spezifischen Nukleinsäurekette bereitgestellt ist.

Die Reaktionsbedingungen (z.B. Temperatur) werden dabei dermassen gestaltet, dass eine spontane Trennung von komplementären Primer-Verlängerungsprodukten in Abwesenheit eines Aktivator-Oligonukleotdies unwahrscheinlich oder signifikant verlangsamt ist.

Die anzustrebende Erhöhung der Spezifität einer Amplifikation resultiert somit aus der Sequenzabhängigkeit der Trennung von komplementären Primer-Verlängerungsprodukten, welche eine Zielsequenz umfassen: Aktivator-Oligonukleotid ermöglicht bzw. begünstigt diese Doppelstrang-Trennung als Folge der Übereinstimmung seiner Sequenzabschnitte mit vorgegebenen Sequenzabschnitten der Primer-Verlängerungsprodukte. Diese Übereinstimmung wird nach jedem Synthese-Zyklus durch Aktivator-Oligonukleotid überprüft. Die exponentielle Amplifikation resultiert als Folge aus erfolgreichen Wiederholungen aus Synthese-Vorgängen und sequenzspezifischen Strangverdränungen durch Aktivator-Oligonukleotid, d.h. "Aktivierungen" (Doppelstrang-Öffnungen / Doppelstrang-Trennungen / Strangverdrängungs-Vorgängen resultierend in einzelsträngiger Form von entsprechenden Primer-Bindungsstellen) von neusynthetisierten Primer-Verlängerungsprodukten.

Die Aufgaben der Erfindung werden dadurch gelöst, dass eine Kombination eines Amplifikations-Verfahren mit einem Detektions-System bereitgestellt wird, wobei das Detektions-System mindestens eine Oligonukleotid-Sonde und mindestens einen Fluoreszenzreporter umfasst. Weiterhin umfasst das Detektionssystem einen zum Fluoreszenzreporter passenden Fluoreszenzquencher (Quencher genannt), wobei dieser Quencher in der Lage ist, die Fluoreszenzsignale des Fluoreszenzreporters unter bestimmten Umständen zu verringern bzw. die Signal-Intensität zu reduzieren. In einer weiteren Ausführungsform kann ein Detektionssystem ein zum Fluoreszenzreporter passendes Donor-Fluorophor umfassen, so dass dieses Donor-Fluorophor in der Lage ist, die Fluoreszenzsignale des Fluoreszenzreporters unter bestimmten Umständen durch Energie-Übertragung zu ermöglichen. In einer weiteren Ausführungsform kann das Detektions-System das Aktivator-Oligonukleotid umfassen, wobei das Aktivator-Oligonukleotid entweder einen Fluoreszenzreporter oder eine Donor-Fluorophor oder ein Fluoreszenzquencher umfasst.

Die Anordnung einzelner Elemente (Fluoreszenzreporeter, Fluoreszenzquencher, Donor-Fluorophor) an der Oligonukleotid-Sonde und/oder an dem Aktivator-Oligonukleotid soll in Anwesenheit eines ersten Primer-Verlängerungsproduktes unter Ausbildung jeweils komplementärer Komplexe zu Änderung des Fluoreszenzsignals vom Fluoreszenzreporter führen. Diese Änderung kann je nach gewählter Konstellation zwischen einem Fluoreszenzreporter und/oder einer Donor-Fluorophor und/oder eines Fluoreszenzquenchers zu einer Zunahme oder Abnahme der Signal-Intensität des Fluoreszenzrepoters führen. Diese Änderung kann mit bekannten geeigneten Mitteln während oder nach einer abgelaufenen Reaktion detektiert werden. Die Erfassung der Änderungen des Signals kann dabei Rückschlüsse auf Verlauf der Reaktion ermöglichen: z.B. Amplitude des Signals, Kinetik, Zeit- bzw. Konzentrations- Abhängigkeit der Signal-Erscheinung. Bei Verwendung mehrere Target-Sequenzen können Multiplexe Analysen entsprechend durch unterschiedliche spektrale Eigenschaften kodiert werden, so dass auch mehrere Reaktionen parallel zu einander beobachet werden können.

Die vorliegende Erfindung beschreibt einige Ausführungsformen von Oligonukleotid-Sonden, welche zur Erfassung des Reaktionsfortschrittes besonders vorteilhaft sind. Durch geeignete Positionierung von einzelnen Elementen des Detektionssystems an Oligonukleotid-Sonde und/oder Aktivator-Oligonukleotid ist es möglich, Bindungsereignisse dieser Komponenten an das erste Primer-Verlängerungsprodukt durch Signal-Zunahme oder Signal-Abnahme zu erfassen. Eine solche Erfassung kann entwender während der Amplifikation stattfinden (z.B. als On-Line Erfassung) oder in geeigneten zeitlichen Abständen oder auch erst am Ende einer Reaktion erfolgen.

Der Einsatz von vordefiniertem Aktivator-Oligonukleotid ermöglicht somit eine sequenzabhängige Überprüfung der Inhalte von Primer-Verlängerungsprodukten zwischen einzelnen Synthese-Schritten während der exponentiellen Amplifikation und Herbeiführung einer Selektion bzw. einer Auswahl von Sequenzen für nachfolgende Synthese-Schritte. Dabei kann zwischen "aktiven", einzelsträngigen Zuständen von neusynthetisierten spezifischen Primer-Verlängerungsprodukten als Folge einer erfolgreichen Interaktion mit einem Aktivator-Oligonukleotid, und "inaktiven" doppelsträngigen Zuständen von neusynthetisierten unspezifischen Primer-Verlängerungsprodukten als Folge einer mangelhaften und / oder unzureichenden und /oder verminderten und / oder verlangsamten Interaktion mit einem Aktivator-Oligonukleotid unterschieden werden.

Für eine exponentielle Amplifikation ergeben sich daraus folgende Effekte:
Unter nicht-denaturierenden Bedingungen erfolgt die Trennung von spezifisch synthetisierten Strängen unter Mitwirkung von Aktivator-Oligonukleotid.

Exponentielle Amplifikation von Zielsequenz umfassenden Nukleinsäureketten erfolgt sequenzkontrolliert (Hauptreaktion). Diese Sequenz-Kontrolle erfolgt nach jedem Synthese-Schritt und schließt Sequenzsegmente ein, welche zwischen Primern liegen und eine Zielsequenz umfassen. Die erfolgreiche Überprüfung des Ergebnisses der Synthese nach jedem Synthese-Schritt resultiert in Trennng von beiden spezifischen Primer-Verlängerungsprodukten, was die Voraussetzung für weitere spezifische Synthese-Schritte darstellt.

Während der Amplifikation kann eine initiale Entstehung / Generierung von unspezifischen Primer-Verlängerungsprodukten nicht prinziell ausgeschlossen werden (Nebenprodukte). Aufgrund einer Matrizenabhängigkeit liegen solche unspezifischen Primer-Verlängerungsprodukte unmittelbar nach ihrer Synthese in der Regel in doppelsträngiger Form vor. Die Interaktion mit dem Aktivator-Oligonukleotid bleibt allerdings entweder vollständig aus oder ist eingeschränkt, so dass es nicht zu einer Strangtrennung kommt oder die Strangtrennung verlangsamt gegenüber der Hauptreaktion ist. Die Übertragung einer fehlerhaften Sequenzinformation von einem Synthese-Zyklus auf den nächsten findet somit nicht statt.

Durch die Wahl der Reaktions-Bedingungen und die Gestaltung von Aktivator-Oligonukleotid ist es somit möglich, einen gezielten Einfluss auf Effizienz der Regenerierung von korrekten Nukleinsäureketten-Matrizen zwischen einzelnen Synthese-Schritten im Rahmen eines Amplifikationsverfahrens auszuüben. Im Allgemeinen, je höher das Maß der Übereinstimmung der synthetisierten Sequenz mit der vorgegebenen Sequenz des Aktivator-Oligonukleotides, umso erfolgreicher ist die Trennung der synthetisierten Produkte, umso erfolgreicher ist die Regenerierung von korrekten Matrizen von einem Synthese-Schritt zum nächsten. Umgekehrt führt eine Sequenz-Abweichung bei Nebenprodukten zu einer insuffizienten Regenerierung von Matrizensträngen und somit zu einer Verlangsamung der Synthese-Initiierung bzw. zu Reduzierung der Ausbeute in jedem nachfolgenden Zyklus. Die gesamte exponentielle Amplifikation von Nebenprodukten verläuft entweder langsamer oder findet gar nicht statt und / oder bleibt auf einem nicht detektierbaren Niveau.

Das Verfahren ermöglicht somit eine Überprüfung der synthetisierten Sequenzen in Echtzeit, d.h. ohne Reaktions-Unterbrechung und stellt somit Potenzial für Entwicklung von homogenen Assays dar, bei welchen alle Komponenten des Assays bereits zu Beginn einer Reaktion im Reaktionsgemisch vorliegen.

### Begriffe und Definitionen:

Im Rahmen der vorliegenden Erfindung haben die verwendeten Begriffe folgende Bedeutung:
Der Begriff **"Oligonukleotid"** wie er hier bezugnehmend auf Primer, Aktivator-Oligonukleotid, Sonden, zu amplifizierende Nukleinsäurekette verwendet wird, ist als ein Molekül definiert, das zwei oder mehr, bevorzugt mehr als drei Desoxyribonukleotide und/oder Ribonukleotide und/oder Nukleotid-Modifikationen und/oder Nicht-Nukleotid-Modifikationen umfasst. Seine Länge umfasst beispielsweise Bereiche zwischen 3 bis 300 Nukleotid-Einheiten oder ihrer Analoga, bevorzugt zwischen 5 bis 200 Nukleotid-Einheiten oder ihrer Analoga. Seine genaue Größe hängt von vielen Faktoren ab, die ihrerseits von der letztendlichen Funktion oder Verwendung der Oligonukleotide abhängen.

Der Begriff **"Primer",** wie er hier verwendet wird, betrifft ein Oligonukleotid, unabhängig davon, ob es natürlicherweise z. B. in einer gereinigten Restriktionsspaltung vorkommt oder synthetisch produziert wurde. Ein Primer ist fähig, als Initiationspunkt der Synthese zu wirken, wenn er unter Bedingungen eingesetzt wird, bei denen die Synthese eines zu einem Nukleinsäurestrang komplementären Primer-Verlängerungsproduktes induziert wird, d. h. in Gegenwart von Nukleotiden und einem induzierenden Agens, wie z.B. DNA-Polymerase bei einer geeigneten Temperatur und einem geeigneten pH-Wert. Der Primer ist für eine maximale Wirksamkeit bei der Amplifikation bevorzugt einzelsträngig. Der Primer muss genügend lang sein, um in Gegenwart des induzierenden Agens die Synthese des Verlängerungsproduktes zu initiieren. Die genaue Länge des Primers hängt von vielen Faktoren ab, einschließlich der Reaktions-Temperatur und der Primerquelle und der Anwendung des Verfahrens. Beispielsweise beträgt die Länge der Oligonukleotid-Primer bei diagnostischen Anwendungen, je nach Komplexität der Zielsequenz zwischen 5 bis 100 Nukleotide, vorzugsweise 6 bis 40 und besonders bevorzugt 7 bis 30 Nukleotide. Kurze Primer-Moleküle erfordern im Allgemeinen für Ausübung ihrer Primer-Funktion niedrigere Reaktions-Temperaturen, um genügend stabile Komplexe mit der Matrize zu bilden, oder höhere Konzentrationen von anderen Reaktionskomponenten, beispielsweise von DNA-Polymerasen, so dass eine ausreichende Verlängerung von gebildeten Primer-Matrizen-Komplexen erfolgen kann.

Die hier verwendeten Primer sind so ausgewählt, das sie "im Wesentlichen" komplementär zu den verschiedenen Strängen jeder spezifischen zu amplifizierenden Sequenz sind. Das bedeutet, dass die Primer genügend komplementär sein müssen, um mit ihren betreffenden Strängen zu hybridisieren und eine Primer-Verlängerungsreaktion zu initiieren. Somit braucht die Primersequenz beispielsweise nicht die genaue Sequenz der Zielsequenz wiederzuspiegeln. Zum Beispiel kann ein nicht-komplementäres Nukleotidfragment an dem 5'-Ende des Primers angeheftet sein, wobei der Rest der Primersequenz komplementär zu dem Strang ist. In einer anderen Ausführungsform können einzelne nicht-komplementäre Basen oder längere nicht-komplementäre Sequenzen in einem Primer eingefügt sein, vorausgesetzt, dass die Primersequenz eine genügend große Komplementarität mit der zu amplifizierenden Sequenz des Stranges aufweist, um damit zu hybridisieren und so ein Primer-Matrizen-Komplex fähig zur Synthese des Verlängerungsproduktes zu erzeugen.

Im Rahmen der enzymatischen Synthese eines zu Matrize komplementären Stranges wird ein Primer-Verlängerungsprodukt erzeugt, was vollständig zum Matrizen-Strang komplementär ist.

### Tm-Schmelztemperatur

Als Schmelztemperatur eines komplementären oder partiell komplementären Doppelstranges wird im Allgemeinen ein Messwert einer Reaktionstemperatur verstanden, bei welchem ca. die Hälfte der Stränge als Doppelstrang vorliegt und die andere Hälfte als Einzelstrang vorliegt. Das System (Assoziation und Dissoziation von Strängen) befindet sich im Gleichgewicht.

Aufgrund einer Vielzahl von Faktoren, welche die Tm eines Doppelstranges beeinflussen können (z.B. Sequenzlänge, CG-Gehalt der Sequenz, Puffer-Bedingungen, Konzentration von divalenten Metal-Kationen etc.) sollte die Bestimmung der Tm einer zu amplifizierenden Nukleinsäure unter gleichen Bedingungen erfolgen, wie die beabsichtigte Amplifikationsreaktion.

Wegen Abhängigkeit der messbaren Schmelztemperatur von multiplen Reaktions-Parametern, z.B. von jeweiligen Puffer-Bedingungen und jeweiligen Konzentrationen der Reaktionsteilnehmer, wird unter Schmelztemperatur ein Wert verstanden, welcher in gleichem Reaktionspuffer gemessen wurde wie die exponentielle Amplifikation, bei Konzentrationen von beiden komplementären Komponenten eines Doppelstranges von etwa 0,1 µmol/l bis etwa 10 µmol/l, bevorzugt in Konzentration von etwa 0,3 µmol/ bis ca. 3 µmol/l, vorzugsweise bei ca. 1 µmol/l. Bei dem jeweiligen Wert der Schmelztemperatur handelt sich um einen Richtwert, welcher mit der Stabilität eines jeweiligen Doppelstranges korreliert.

Die **Desoxyribonukleosid-Triphosphate** (dNTPs) dATP, dCTP, dGTP und TTP (oder dUTP, oder dUTP/TTP-Gemisch) werden in adäquaten Mengen zum Synthesegemisch gegeben. In einer Ausführungsform können zusätzlich zu dNTPs mindestens eine weitere Art von dNTP-Analoga zum Synthesegemisch zugegeben werden. Diese dNTP-Analoga umfassen in einer Ausführungsform beispielsweise eine charakteristische Markierung (z.B. Biotin oder Fluoreszenzfarbstoff), so dass wenn in Nukleinsäurestrang eingebaut, auch diese Markierung in den Nukleinsäurestrang integriert ist. In einer anderen Ausführungsform umfassen diese dNTP-Analoga mindestens eine Modifikation von Zucker-Phosphat-Anteil des Nukleotids, z.B. alpha-Phosphorothioat-2'-Desoxyribonukleosid-Triphosphate (oder andere Modifikationen, welche einem Nukleinsäurestrang eine Nuklease-Resistenz verleihen), 2',3'-Dideoxy-Ribonukleosid-Triphosphate, Azyklo-Nukleosid-Triphosphate (oder andere zur Termination einer Synthese führende Modifikationen). In einer weiteren Ausführungsform umfassen diese dNTP-Analoga mindestens eine Modifikation von Nukleobase, z.B. Iso-Cytosine, Iso-Guanosine (oder auch andere Modifikationen der Nukleobasen des erweiterten genetischen Alphabets), 2-Amino-Adenosine, 2-Thiouridine, Inosine, 7-deazy-adenosine, 7-deaza-guanosine, 5-Me-Cytosine, 5-Propyl-Uridine, 5-Propyl-Cytosine (oder auch andere Modifikationen von Nukleobasen, welche gegenüber natürlichen Nukleobasen durch eine Polymerase eingebaut werden können und zur Änderung der Strang-Stabilität führen). In einer weiteren Ausführungsform umfasst ein dNTP-Analog sowohl eine Modifikation der Nukleobase als auch eine Modifikation des Zucker-Phosphat-Anteils. In einer weiteren Ausführungsform wird statt mindestens eines natürlichen dNTP-Substrates mindestens eine weitere Art von dNTP-Analoga zum Synthesegemisch zugegeben.

Das die Nukleinsäure-Synthese induzierende Agens kann eine Enzyme einschließende Verbindung oder ein System sein, das so wirkt, dass dadurch die Synthese der Primer-Verlängerungsprodukte bewirkt wird. Geeignete Enzyme für diesen Zweck umfassen z.B. **DNA-Polymerasen** wie Bst Polymerase und ihre Modifikationen, Vent Polymerase und andere - bevorzugt hitzestabile DNA-Polymerasen, die den Einbau der Nukleotide in der korrekten Weise ermöglichen, wodurch die Primer-Verlängerungsprodukte gebildet werden, die zu jedem synthetisierten Nukleinsäurestrang komplementär sind. Im Allgemeinen wird die Synthese am 3'-Ende eines jeden Primers initiiert und schreitet dann in 5'- Richtung entlang des Matrizenstranges fort, bis die Synthese beendet ist oder unterbrochen wird.

Bevorzugt werden Polymerasen verwendet, welche zu Strangverdrängung fähig sind. Dazu zählen beispielsweise das große Fragment der Bst Polymerase oder ihre Modifikationen (z.B. Bst 2.0 DNA Polymerase), Klenow Fragment, Vent exo minus Polymerase, Deepvent exo minus DNA Polymerase, großes Fragment der Bsu DNA Polymerase, großes Fragment der Bsm DNA Polymerase.

In einer Ausführungsform werden bevorzugt Polymerasen eingesetzt, welche keine 5'-3'-Exonuklease-Aktivität aufweisen, bzw. keine 5'-3'-FEN-Aktivität aufweisen.

In einer Ausführungsform werden mindestens zwei verschiedene Polymerasen eingesetzt, beispielsweise Polymerasen welche zu Strangverdrängung fähig sind und solche, welche eine 3'-5'-Proofreading Aktivität aufweisen.

In bevorzugten Ausführungsformen werden Polymerasen mit einer Hotstart-Funktion eingesetzt, welche erst beim Erreichen einer bestimmten Temperatur ihre Funktion entfalten können.

### Erstes Primer-Oligonukleotid:

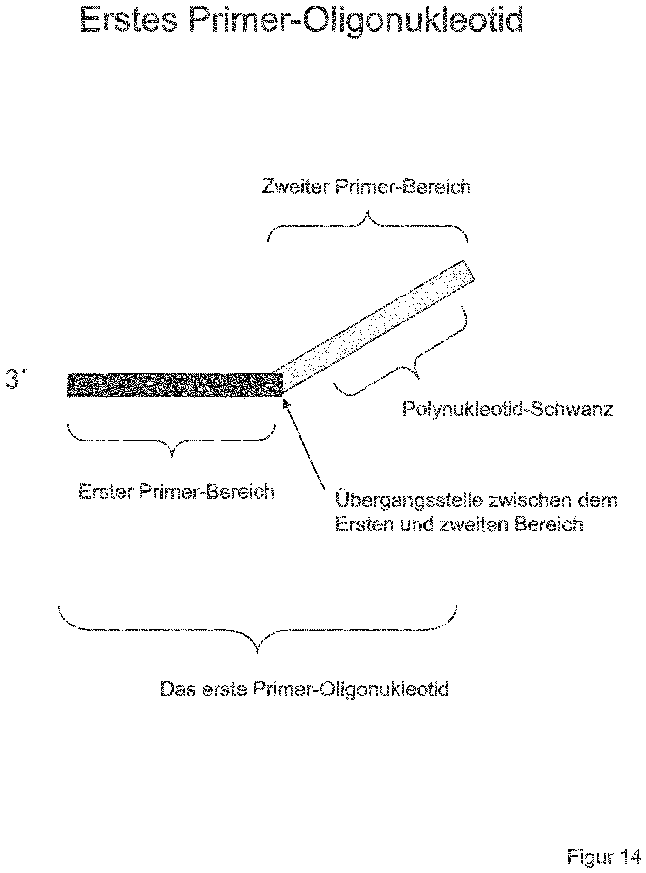

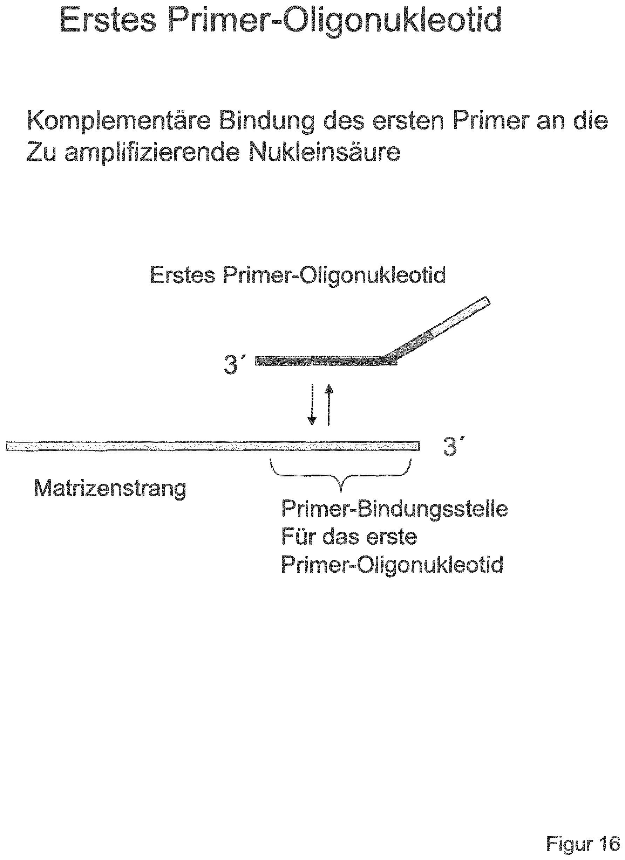

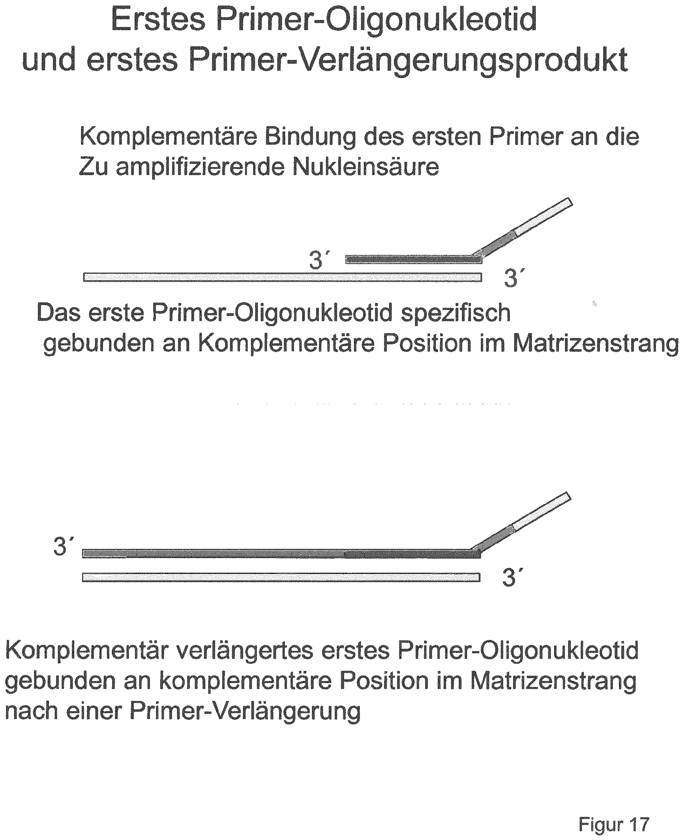

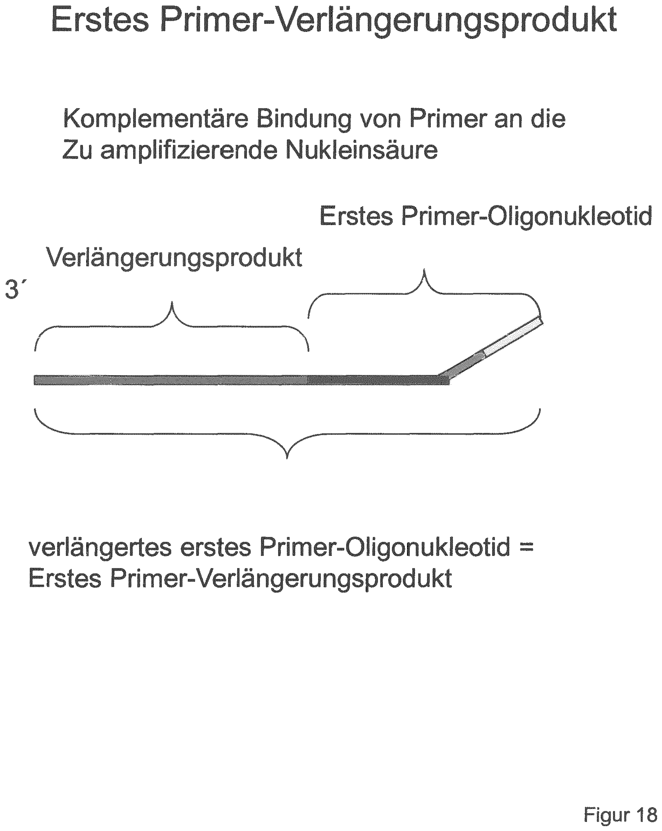

Das erste Primer-Oligonukleotid (Fig. 14 bis 18) umfasst einen ersten Primer-Bereich und einen zweiten Bereich. Der erste Primer-Bereich ist in der Lage, an eine im Wesentlichen komplementäre Sequenz innerhalb der zu amplifizierenden Nukleinsäure oder ihrer Äquivalente zu binden und eine Primer-Verlängerungsreaktion zu initiieren. Der zweite Bereich umfasst einen Polynukleotid-Schwanz, welcher in der Lage ist, ein Aktivator-Oligonukleotid zu binden und damit eine räumliche Nähe zwischen dem Aktivator-Oligonukleotid und den anderen Teilen des ersten Primer-Verlängerungsproduktes zu bewirken, welche ausreichend ist, um eine Strangverdrängung durch das Aktivator-Oligonukleotid zu initiieren. Der zweite Bereich des ersten Primer-Oligonukleotids umfasst weiterhin mindestens eine Modifikation (eine Nukleotid-Modifikation oder eine nicht-Nukleotid-Modifikation), welche die Polymerase am Kopieren des Polynukleotid-Schwanzes hindert, indem die Fortführung der Polymerasenabhängigen Synthese gehemmt wird. Diese Modifikation ist beispielsweise am Übergang zwischen dem ersten und dem zweiten Bereich des ersten Primer-Oligonukleotid lokalisiert. Der erste Primer-Bereich des ersten Primer-Oligonukleotides ist folglich durch eine Polymerase kopierbar, so dass eine komplementäre Sequenz zu diesem Bereich während der Synthese des zweiten Primer-Verlängerungsproduktes von der Polymerase erzeugt werden kann. Der Polynukleotid-Schwanz des zweiten Bereichs des ersten Primer-Oligonukleotides wird vorzugsweise durch die Polymerase nicht kopiert. In einer Ausführungsform wird dies durch die Modifikation im zweiten Bereich erreicht, welche die Polymerase vor dem Polynukleotid-Schwanz stoppt. In einer weiteren Ausführungsform wird dies durch Nukleotid-Modifikationen im zweiten Bereich erreicht, wobei der gesamte Polynukleotid-Schwanz im Wesentlichen aus solchen Nukleotid-Modifikationen besteht und somit für Polymerase unkopierbar ist.

In einer Ausführungsform ist ein jedes erstes Primer-Oligonukleotid spezifisch für je eine zu amplifizierende Nukleinsäure.

In einer Ausführungsform ist ein jedes erstes Primer-Oligonukleotid spezifisch für mindestens zwei der zu amplifizierenden Nukleinsäuren, welche jeweils im Wesentlichen unterschiedliche Sequenzen umfassen.

In einer Ausführungsform ist das erste Primer-Oligonukleotid mit einem charakteristischen Marker markiert, z.B. einem Fluoreszenzfarbstoff (z.B. TAMRA, Fluoreszein, Cy3, Cy5) oder einem Affinitätsmarker (z.B. Biotin, Digoxigenin) oder einem zusätzlichen Sequenzfragment, z.B. zur Bindung einer spezifischen Oligonukleotid-Sonde für Detektion oder Immobilisierung oder zur Barcode-Markierung.

### Zweites Primer-Oligonukleotid:

Ein Oligonukleotid, welches mit seinem 3'-Segment in der Lage ist, an eine im Wesentlichen komplementäre Sequenz innerhalb der zu amplifizierenden Nukleinsäure oder ihrer Äquivalente zu binden und eine spezifische zweite Primer-Verlängerungsreaktion zu initiieren. Dieses zweite Primer-Oligonukleotid ist somit in der Lage, an das 3'-Segment eines ersten spezifischen Primer-Verlängerungsproduktes des ersten Primer-Oligonukleotids zu binden und eine Polymerase-abhängige Synthese eines zweiten Primer-Verlängerungsproduktes zu initiieren.

Die Länge des zweiten Primer-Oligonukleotids kann zwischen 15 und 100 Nukleotiden liegen, bevorzugt zwischen 20 und 60 Nukleotiden, besonders bevorzugt zwischen 30 und 50 Nukleotiden.

In einer Ausführungsform ist ein jedes zweites Primer-Oligonukleotid spezifisch für je eine zu amplifizierende Nukleinsäure.

In einer Ausführungsform ist ein jedes zweites Primer-Oligonukleotid spezifisch für mindestens zwei der zu amplifizierenden Nukleinsäuren, welche jeweils im Wesentlichen unterschiedliche Sequenzen umfassen.

In einer Ausführungsform ist das zweite Primer-Oligonukleotid mit einem charakteristischen Marker markiert, z.B. einem Fluoreszenzfarbstoff (z.B. TAMRA, Fluoreszein, Cy3, Cy5) oder einem Affinitätsmarker (z.B. Biotin, Digoxigenin) oder einem zusätzlichen Sequenzfragment, z.B. zur Bindung einer spezifischen Oligonukleotid-Sonde für Detektion oder Immobilisierung oder zur Barcode-Markierung.

### Primer-Verlängerungsprodukt:

Ein Primer-Verlängerungsprodukt (auch Primer-Extensionsprodukt genannt) entsteht durch enzymatische (Polymerase-abhängige) Verlängerung eines Primer-Oligonukleotides als Ergebnis einer matrizenabhängigen Synthese, welche durch eine Polymerase katalysiert wird.

Ein Primer-Verlängerungsprodukt umfasst die Sequenz des Primer-Oligonukleotid in seinem 5'-Segment und die Sequenz des Verlängerungsproduktes (auch ExtensionsProdukt genannt), welches durch eine Polymerase in matrizenabhängiger Form synthetisiert wurde. Das durch die Polymerase synthetisierte Verlängerungsprodukt ist komplementär zum Matrizenstrang, an welchem es synthetisiert wurde.

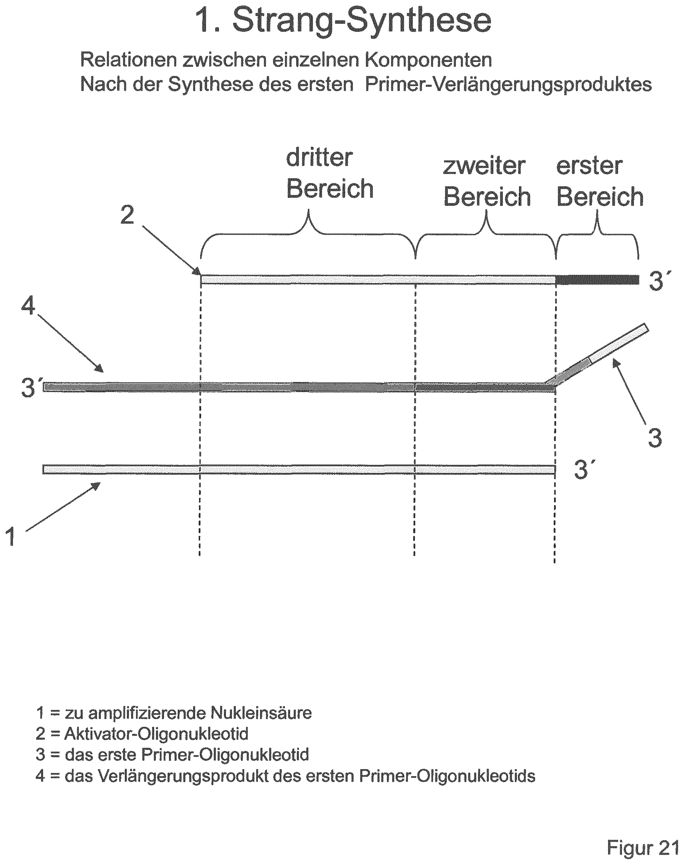

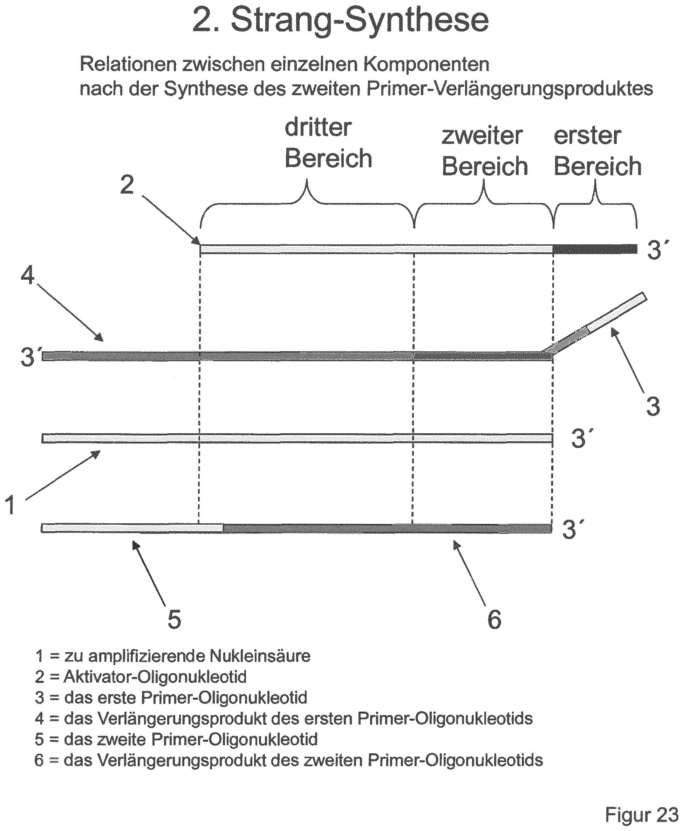

Ein spezifisches Primer-Verlängerungsprodukt (Fig. 21 bis 24) (Hauptprodukt) umfasst Sequenzen der zu amplifizierenden Nukleinsäurekette. Es ist Ergebnis einer spezifischen Synthese bzw. einer korrekten Ausführung einer beabsichtigten Primer-Verlängerungsreaktion bei welcher die spezifisch zu amplifizierende Nukleinsäurekette als Matrize dient. In einer bevorzugten Ausführungsform stimmt die Sequenz der synthetisierten Primer-Verlängerungsprodukte mit der zu erwartenden Sequenz einer zu amplifizierenden Nukleinsäure komplett überein. In einer anderen Ausführungsform können Abweichungen in der erhaltenen Sequenz von der theoretisch zu erwartenden Sequenz toleriert werden. In einer Ausführungsform liegt das Ausmaß der Übereinstimmung der erhaltenen Sequenz infolge einer Amplifikation mit der Sequenz der theoretisch zu erwartenden zu amplifizierenden Nukleinsäure beispielsweise zwischen 90 % und 100 %, bevorzugt liegt die Übereinstimmung bei über 95 %, idealerweise liegt die Übereinstimmung über 98 % (gemessen am Anteil der synthetisierten Basen).

Die Länge des Verlängerungsproduktes eines spezifischen Primer-Verlängerungsproduktes kann zwischen 10 und 300 Nukleotiden, besser zwischen 10 und 180 Nukleotiden liegen, bevorzugt zwischen 20 und 120 Nukleotiden, besonders bevorzugt zwischen 30 und 80 Nukleotiden.

Ein unspezifisches Primer-Verlängerungsprodukt (Nebenprodukt) umfasst beispielsweise Sequenzen, welche als Ergebnis einer unspezifischen bzw. inkorrekten bzw. nicht beabsichtigten Primer-Verlängerungsreaktion entstanden sind. Diese schließen beispielsweise Primer-Verlängerungsprodukte ein, welche als Folge eines falschen Initiierungs-Ereignisses (falsches Primings) oder als Folge anderer Nebenreaktionen, einschließlich polymerase-abhängigen Sequenzveränderungen wie Basen-Substitution, Deletion etc., entstanden sind. Das Ausmaß an Sequenzabweichungen von unspezifischen Primer-Verlängerungsprodukten übersteigt im Allgemeinen die Fähigkeit von Aktivator-Oligonukleotide, solche doppelsträngige Nebenprodukte erfolgreich von ihren Matrizen zu verdrängen, so dass Amplifikation von solchen Nebenprodukten langsamer verläuft oder vollständig ausbleibt. Das Ausmaß der Akzeptanz bzw. die Toleranzgrenze zu Abweichungen sind abhängig beispielsweise von Reaktionstemperatur und Art und Weise der Sequenabweichung. Beispiele für unspezifische Primer-Verlängerungsprodukte bilden Primer-Dimere oder Sequenzvarianten, welche nicht der zu amplifizierenden Nukleinsäure entsprechen, z.B. Sequenzen, welche keine Zielsequenz umfassen.

Die Beurteilung einer hinreichenden Spezifität der Amplifikation hängt häufig mit der Aufgabenstellung zusammen. In vielen Amplifikationsverfahren kann ein gewisses Maß der Unspezifität der Amplifikationsreaktion toleriert werden, solange das gewünschte Ergebnis erreicht werden kann. In einer bevorzugten Ausführungsform beträgt der Anteil von zu amplifizierenden Nukleinsäureketten am Gesamtergebnis der Reaktion mehr als 1 %, besser mehr als 10 %, noch bevorzugter mehr als 30 % gemessen an Gesamtmenge von neusynthetisierten Stränge.

### Die zu amplifizierende Nukleinsäure

Die zu amplifizierende Nukleinsäure stellt eine Nukleinsäurekette dar, welche sequenzspezifisch oder zumindest vorwiegend sequenzspezifisch mit Hilfe der exponentiellen Amplifikation unter Einsatz von Primern und Aktivator-Oligonukleoid durch die Polymerase amplifiziert werden soll.

Die Länge der zu amplifizierenden Nukleinsäure kann zwischen 20 und 300 Nukleotiden, besser zwischen 30 und 200 Nukleotiden liegen, bevorzugt zwischen 40 und 150 Nukleotiden, besonders bevorzugt zwischen 50 und 100 Nukleotiden.

Die zu amplifizierende Nukleinsäurekette kann eine oder mehrere Zielsequenzen oder ihre Äquivalente umfassen. Weiterhin kann eine zu amplifizierende Nukleinsäure die im Wesentlichen zu einer Zielsequenz komplementären Sequenzen umfassen, welche mit ähnlicher Effizienz wie eine Zielsequenz in einer Amplifikationsreaktion vermehrt werden und Zielsequenz oder ihre Abschnitte umfasst. Zusätzlich zu einer Zielsequenz kann die zu amplifizierende Nukleinsäure weitere Sequenzsegmente einschließen, beispielsweise Primer-Sequenzen, Sequenzen mit Primer-Bindungsstellen und/oder Sequenzsegmente für Bindung von Detektions-Sonden, und/oder Sequenzsegmente für Sequenz-Kodierung von Strängen durch Barcode-Sequenzen und/oder Sequenzsegmente für Bindung an eine feste Phase. Die Primer-Sequenzen oder ihre Sequenzanteile, sowie Primer-Bindungsstellen oder ihre Sequenzanteile können beispielsweise zu Sequenzabschnitten einer Zielsequenz gehören.

In einer Ausführungsform entspricht die zu amplifizierende Nukleinsäure der Zielsequenz.

In einer anderen Ausführungsform bildet die Zielsequenz einen Teil der Sequenz der zu amplifizierenden Nukleinsäurekette. Eine solche Zielsequenz kann von 3'-Seite und/oder von 5'-Seite von weiteren Sequenzen flankiert sein. Diese weiteren Sequenzen können beispielsweise Bindungsstellen für Primer oder ihre Anteile umfassen, und/oder Primer-Sequenzen oder ihre Anteile umfassen, und/oder Bindungsstellen für Detektions-Sonden umfassen, und/oder Adaptor-Sequenzen für komplementäre Bindung an eine feste Phase (z.B. Im Rahmen von Microarrays, oder Bead-basierten Analysen) umfassen und/oder Barcoding-Sequenzen für eine digitale Signatur von Sequenzen umfassen.

Damit die Amplifikation starten kann, muss zu Beginn der Reaktion eine Nukleinsäurekette in das Reaktionsgemisch gegeben werden, welche als initiale Matrize für die Synthese der zu amplifizierenden Nukleinsäurekette auftritt. Diese Nukleinsäurekette wird als Start-Nukleinsäurekette bezeichnet. Diese Start-Nukleinsäurekette gibt die Anordnung einzelner Sequenz-Elemente vor, welche für die Bildung / Synthese / exponentielle Amplifikation einer zu amplifizierenden Nukleinsäurekette von Bedeutung sind.

In einer bevorzugten Ausführungsform entspricht die initiale Matrize (Start-Nukleinsäurekette), welche einer Amplifikationsreaktion zu Beginn zugeführt wird, bzw. welche in das Reaktionsgemisch gegeben wird, der Sequenz-Zusammensetzung der zu amplifizierenden Nukleinsäurekette.

In anfänglichen Stadien der Amplifikatons-Reaktion und in ihrem weiteren Verlauf binden die jeweiligen Primer an die entsprechenden Bindungsstellen in der Start-Nukleinsäurekette und initiieren die Synthese von spezifischen Primer-Verlängerungsprodukten. Solche spezifischen Primer-Verlängerungsprodukte akkumulieren im Verlauf der Amplifikation in exponentieller Art und Weise und übernehmen zunehmend die Rolle von Matrizen für Synthese komplementärer Primer-Verlängerungsprodukte bei einer exponentiellen Amplifikation.

Durch die wiederholten matrizen-abhängigen Synthese-Vorgänge im Rahmen einer exponentiellen Amplifikation wird somit die zu amplifizierende Nukleinsäurekette gebildet.

Gegen Ende einer Amplifikationsreaktion kann das Hauptprodukt der Reaktion (die zu amplifizierende Nukleinsäure) vorwiegend einzelsträngig sein oder vorwiegend einen komplementären Doppelstrang bilden. Dies kann beispielsweise durch die relative Konzentrationen von beiden Primern und entsprechende Reaktionsbedingungen bestimmt werden.

Äquivalente der zu amplifizierenden Nukleinsäure umfassen Nukleinsäuren mit im Wesentlichen identischen Informationsgehalt. Beispielsweise haben komplementäre Stränge einer zu amplifizierenden Nukleinsäure identischen Informationsgehalt und können als äquivalent bezeichnet werden.

### Zielsequenz

Eine Zielsequenz ist in einer Ausführungsform ein Segment einer zu amplifizierenden Nukleinsäurekette, welches als charakteristische Sequenz der zu amplifizierenden Nukleinsäure dienen kann. Diese Zielsequenz kann als Marker für die Anwesenheit oder Abwesenheit einer anderen Nukleinsäure dienen. Diese andere Nukleinsäure dient somit als Quelle der Zielsequenz und kann beispielsweise eine genomische DNA oder RNA oder Teile der genomischen DNA oder RNA (z.B. mRNA), oder Äquivalente der genomischen DNA oder RNA eines Organismus sein (z.B. cDNA, modifizierte RNA wie rRNA, tRNA, microRNA etc.), oder definierte Veränderungen der genomischen DNA oder RNA eines Organismus umfassen, beispielsweise Mutationen (z.B. Deletionen, Insertionen, Substitutionen, Additionen, Sequenzvermehrung, z.B. Repeat-Vermehrung im Rahmen von Mikrosatellit-Instabilität), Splice-Varianten, Rearrangement-varianten (z.B. T-Zell-Rezeptor Varianten) usw. Die einzelnen Zielsequenzen können für ein phänotypisches Merkmal stehen, beispielsweise für Antibiotika-Resistenz oder prognostische Information haben und somit für diagnostische Assays/Teste vom Interesse sein. Als Quelle/Ursprung einer Zielsequenz kann eine solche Nukleinsäure beispielsweise die Zielsequenz als Sequenz-Element ihres Stranges umfassen. Eine Zielsequenz kann somit als charakteristischer Marker für einen bestimmten Sequenzinhalt einer anderen Nukleinsäure dienen.

Die Zielsequenz kann einzelsträngig oder doppelsträngig sein. Sie kann mit der zu amplifizierenden Nukleinsäure im Wesentlichen identisch sein oder nur einen Teil der zu amplifizierenden Nukleinsäure darstellen.

Äquivalente der Zielsequenz umfassen Nukleinsäuren mit im wesentlichen identischen Informationsgehalt. Beispielsweise haben komplementäre Stränge einer Zielsequenz identischen Informationsgehalt und können als äquivalent bezeichnet werden, RNA und DNA Varianten einer Sequenz sind ebenfalls Beispiele für äquivalenten Informationsgehalt.

Im Rahmen der Material-Vorbereitung vor Amplifikations-Reaktion kann eine solche Zielsequenz aus ihrer ursprünglichen Sequenzumgebung isoliert werden und für die Amplifikationsreaktion vorbereitet werden.

In einer bevorzugten Ausführungsform umfasst eine zu amplifizierende Nukleinsäure eine Zielsequenz. In einer Ausführungsform entspricht die Zielsequenz der zu amplifizierenden Nukleinsäure. In einer weiteren bevorzugten Ausführungsform umfasst eine Start-Nukleinsäurekette eine Zielsequenz. In einer Ausführungsform entspricht die Zielsequenz einer Start-Nukleinsäurekette.

### Start-Nukleinsäurekette

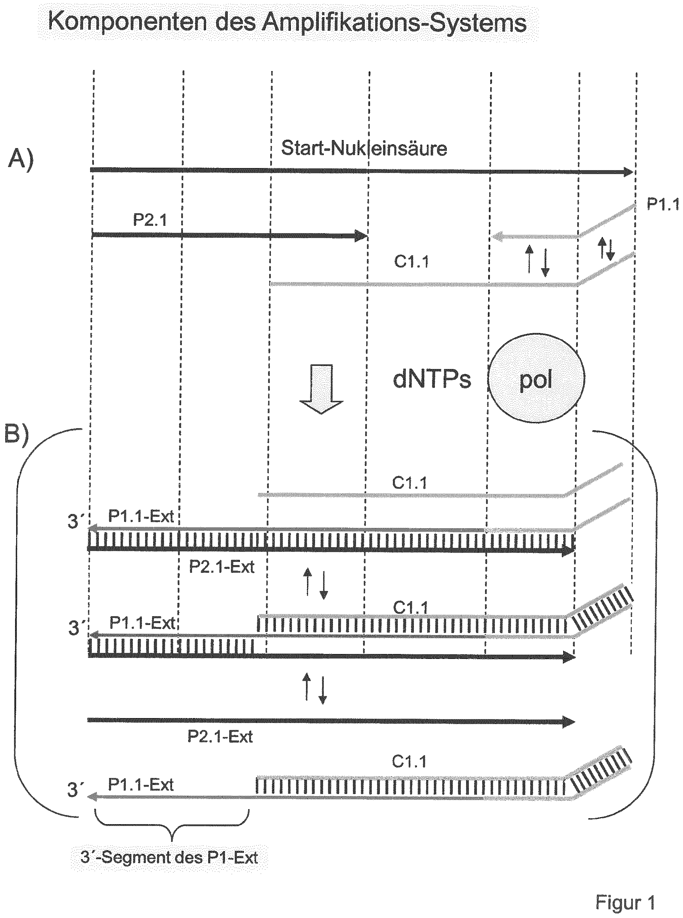

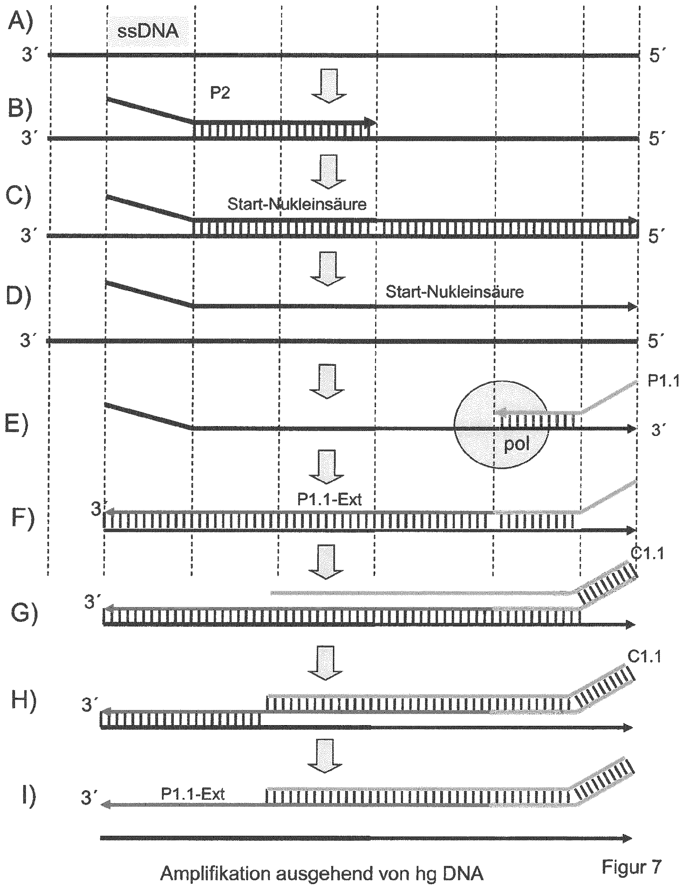

Damit die Amplifikation starten kann, muss zu Beginn der Reaktion eine Nukleinsäurekette in das Reaktionsgemisch gegeben werden, welche als initiale Matrize für die Synthese der zu amplifizierenden Nukleinsäurekette auftritt (Fig. 1 und 7). Diese Nukleinsäurekette wird als Start-Nukleinsäurekette bezeichnet. Diese Start-Nukleinsäurekette gibt die Anordnung einzelner Sequenz-Elemente vor, welche für die Bildung / Synthese / exponentielle Amplifikation einer zu amplifizierenden Nukleinsäurkette von Bedeutung sind.

Eine solche Start-Nukleinsäurekette kann einzelsträngig oder doppelsträngig zu Beginn der Reaktion vorliegen. Wenn die komplementären Stränge der Start-Nukleinsäurekette voneinander getrennt sind, können die Stränge unabhängig davon, ob die Nukleinsäure ursprünglich doppel- oder einzelsträngig vorlag, als Matrize für die Synthese von spezifischen komplementären Primer-Verlängerungsprodukte dienen.

### Aktivator-Oligonukleotid:

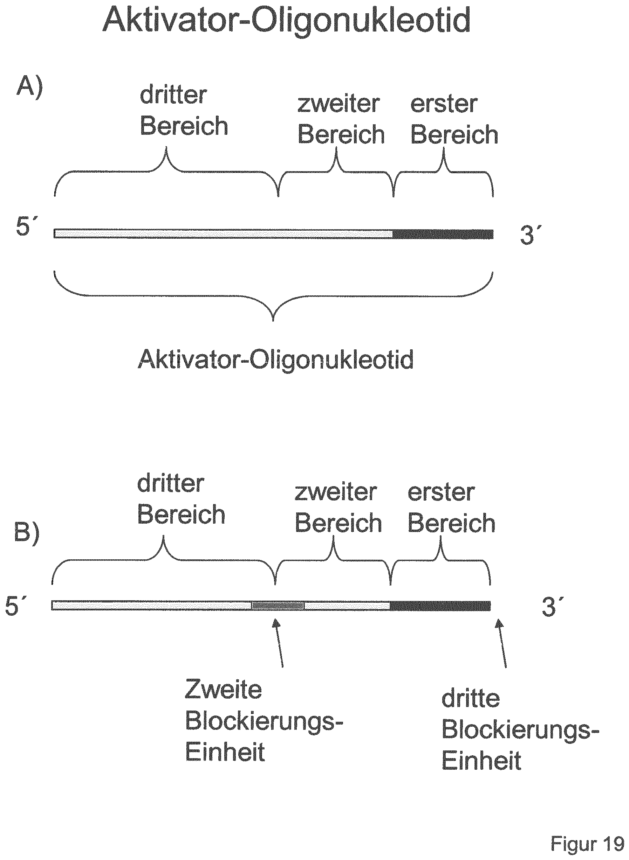

Das Aktivator-Oligonukleotid (Fig. 19) ist eine bevorzugt einzelsträngige Nukleinsäurekette, welche eine vordefinierte im wesentlichen komplementäre Sequenz in einem Teil des ersten Primer-Verlängerungsprodukts einschließt, welches im Rahmen der Amplifikation der zu amplifizierenden Nukleinsäure spezifisch generiert wird. Dadurch kann das Aktivator-Oligonukleotid an das erste Primer-Oligonukleotid und mindestens an das 5'-Segment des spezifischen Verlängerungsprodukts des ersten Primer-Oligonukleotid im Wesentlichen komplementär binden. Das Aktivator-Oligonukleotid umfasst in seinen inneren Sequenzsegment in einer Ausführungsform Nukleotid-Modifikationen, welche die Polymerase daran hindern, einen komplementären Strang unter Verwendung des Aktivator-Oligonukleotides als Matrize zu synthetisieren, wenn das erste Primer-Oligonukleotid an das Aktivator-Oligonukleotid komplementär gebunden ist. Das Aktivator-Oligonukleotid ist weiterhin in der Lage, unter den gewählten Reaktionsbedingungen das zweite spezifische Primer-Verlängerungsprodukt vollständig oder teilweise aus der Bindung mit dem ersten spezifischen Primer-Verlängerungsprodukt via Strangverdrängung zu verdrängen. Dabei lagert sich das Aktivator-Oligonukleotid mit seinen komplementären Bereichen an das erste spezifische Primer-Verlängerungsprodukt an. Bei erfolgreicher Bindung zwischen dem Aktivator-Oligonukleotid und dem ersten spezifischen Primer-Verlängerungsprodukt führt dies zu Wiederherstellung eines einzelsträngigen Zustandes des 3'-ständigen Segments des zweiten spezifischen Primer-Verlängerungsproduktes, welches für die Bindung des ersten Primer-Oligonukleotides geeignet ist, so dass eine neue Primer-Verlängerungsreaktion stattfinden kann. Während der Synthese des zweiten Primer-Verlängerungsproduktes kann das Aktivator-Oligonukleotid aus der Bindung mit dem ersten Primer-Verlängerungsprodukt mittels Strangverdrängung beispielsweise durch Polymerase und/oder durch das zweite Primer-Oligonukleotid getrennt werden.

### Detektions-System:

Ein Detektionssystem umfasst mindestens eine Oligonukleotid-Sonde und mindestens einen Fluoreszenz-Reporter (ein Fluorophor). Das Detektionssystem soll in der Lage sein, die Synthese des ersten Primer-Verlängerungproduktes zu erfassen. Dies geschieht durch Verwendung von Oligonukleotiden, welche in der Lage sind an das Primer-Verlängerungsprodukt zu binden und dabei ein spezifisches Signal zu generieren, bzw. eine Änderung des Signals herbeizuführen. Diese Änderung kann dabei eine Zunahme oder Abnahme der Fluoreszenzintensität sein. Ein Detektions-System kann weiterhin zusätzliche Komponenten umfassen. Zu solchen Komponenten gehören bevorzugt Fluoreszenzquencher und/oder Donor-Fluorophor. Weiterhin kann das Detektions-System auch das Aktivator-Oligonukleotid umfassen. Die Anordnung von Fluoreszenz-Reporter, Fluoreszenz-Quencher, Donor-Phluorophore an der Oligonukleotid-Sonde oder an dem Paar umfassend Oligonukleotid-Sonde und Aktivator-Oligonukleotid ermöglicht es die Bindungsereignisse an das erste Primer-Verlängerungsprodukt zu erfassen.

**Fluoreszenz-Reporter** - ein Fluorophor ist eine chemishe Verbindung bzw. ein Molekül, welches bei Anregung durch elektromagnetische Strahlung in der Lage ist, eine elektromagnetische Strahlung (Licht) abzugeben (Emission). Diese vom Fluorophor abgegebene Strahlung (Emission) kann als Fluoreszenzsignal mit geeigneten technischen Mitteln erfasst werden. Ein solcher Reporter kann an einem Oligonukleotid kovalent gebunden werden. Viele Fluorophore sind bekannt, welche an Oligonukleotide gekoppelt werden können (z.B. FAM, TAMRA, HEX, ROX, Cy-Farbstoffe, Alexa-Farbstoffe)

**Fluoreszenz-Quencher** - Ein Quencher ist eine chemische Verbindung / ein Molekül, welches in der Lage ist durch direkten Kontakt (contact quenching) oder durch Energie-Übertragung (z.B. als FRET), die Emission eines Fluorophores zu verringern. In der Regel muss ein Quencher in eine räumliche Nähe zum Fluorophor gebracht werden, damit diese Signal-Minderung im signifikanten Ausmaß erfolgen kann.

Vorteilhaft für eine deutliche Signal-Minderung eines Fluoreszenzreporters durch einen Quencher sind Distanzen zwischen dem Reporter und Quencher von weniger als 25 Nukleotide, besser von weniger als 15 Nukleotide, besonders vorteilhaft von weniger als 5 Nukleotide.

Zur Überwindung einer Signal-Minderung eines Fluoreszenzreporters durch einen Quencher muss die Distanz zwischen diesen Komponenten entsprechend vergrößert werden, vorteilhaft ist dabei wenn die Distanz zwischen dem Reporter und Quencher von auf mehr als 15 Nukleotide, besser auf mehr als 20 Nukleotide, besonders vorteilhaft auf mehr als 40 Nukleotide vergrößert wird. In diesem Fall muss die durch die Nukleotidsequenz bewirkte Entfernung auf eine ausgestreckte Nukleotidsequenz zurückzuführen sein. Wenn sich beispielsweise Hairpin-Strukturen bilden, ist die räumliche Entfernung möglicherweise nicht mehr gegeben.

Besonders bei FRET-basierten Quenchern ist eine hinreichende spektrale Überlappung zwischen dem Emissions-Spektrum eines Fluorophors und dem Absorbtionsspektrum eines Quenchers vorteilhaft. Daher werden Fluorophor-Quencher-Paare bevorzugt, welche mehr als 25% spektrale Überlappung aufweisen. (z.B. FAM / TAMRA).

**Donor-Phluorophore** - sind chemische Verbindungen / Moleküle, welche in der Lage sind, elektromagnetische Strahlung zu absorbieren und durch Energie-Übertragung (z.B. als FRET) auf ein anderes Fluorophor (Akzeptor) dermassen zu übertragen, dass dieses Fluorophor angeregt wird und als Folge davon selbst Licht-Emission generiert. Bei der Emission wird ein Fluoreszenz-Signal generiert. In der Regel bilden Donor und Akzeptor ein Fluoreszenz-Resonanz-Energie-Transfer-Paar (ein FRET-Paar). In der Regel muss ein Donor in eine räumliche Nähe zum Akzeptor (Fluorophor) gebracht werden, damit diese Signal-Generierung im signifikanten Ausmaß erfolgen kann.

Vorteilhaft für Signal-Generierung eines Fluoreszenzreporters infolge eines FRET von einem Donor-Fluorophor sind Distanzen zwischen dem Reporter und Donor von weniger als 25 Nukleotide, besser von weniger als 15 Nukleotide, besonders vorteilhaft von weniger als 5 Nukleotide.

Die Aufhebung der FRET-Wirkung vom Donor zum Akzeptor wird in der Regel durch Vergrößerung der Distanz zwischen beiden Partnern eines FRET-Paares erreicht. Vorteilhaft ist dabei wenn die Distanz zwischen dem Reporter und Donor von auf mehr als 15 Nukleotide, besser auf mehr als 20 Nukleotide, besonders vorteilhaft auf mehr als 40 Nukleotide vergrößert wird.

Weiterhin ist in der Regel eine hinreichende spektrale Überlappung zwischen dem Emissions-Spektrum eines Donors und dem Absorbtionsspektrum eines Akzeptors vorteilhaft. Daher werden FRET-Paare bevorzugt, welche mehr als 25% in spektraler Überlappung aufweisen (z.B. FAM / Cy3).

Erfindungsgemäß wird die räumliche Entfernung zwischen den einzelnen Komponenten bevorzugt dadurch bewirkt, dass zwei Komponenten über eine Nukleotidsequenz verbunden sind, die mit einem bestimmten Teil der Zielsequenz hybridisieren kann.

### Strangverdrängung (Strand Displacement):

Damit wird ein Vorgang bezeichnet, welcher durch Einwirkung eines geeigneten Mittels zu einer vollständigen oder partiellen Trennung eines ersten Doppelstranges (bestehend beispielsweise aus A1 und B1-Strang) führt, und zur gleichzeitigen/parallelen Ausbildung eines neuen zweiten Doppelstranges, wobei mindestens einer der Stränge (A1 oder B1) an der Ausbildung dieses neuen zweiten Stranges beteiligt sind. Man kann hier zwei Formen der Strangverdrängung unterschieden.

In einer ersten Form der Strangverdrängung kann die Ausbildung eines neuen zweiten Doppelstranges unter Verwendung eines bereits existierenden komplementären Stranges erfolgen, welcher zu Beginn der Reaktion im Allgemeinen in einzelsträngiger Form vorliegt. Dabei wirkt das Mittel der Strangverdrängung (beispielsweise ein vorgebildeter einzelsträngiger Strang C1, welcher eine komplementäre Sequenz zum Strang A1 aufweist, auf den ersten bereits ausgebildeten Doppelstrang (A1 und B1) und geht mit dem Strang A1 eine komplementäre Bindung ein, wodurch der Strang B1 aus der Bindung mit dem Strang A1 verdrängt wird. Falls die Verdrängung des B1 vollständig abläuft, so ist das Ergebnis der C1-Wirkung ein neuer Doppelstrang (A1:C1) und ein einzelsträngiger Strang B1. Falls die Verdrängung von B1 unvollständig abläuft, so hängt das Ergebnis von mehreren Faktoren ab. Beispielsweise kann ein Komplex aus partiell doppelsträngigen A1:B1 und A1:C1 als Zwischenprodukt vorliegen.

In einer zweiten Form der Strangverdrängung kann die Ausbildung eines neuen zweiten Doppelstranges unter einer gleichzeitig ablaufenden enzymatischen Synthese des komplementären Stranges erfolgen, wobei ein Strang des ersten vorgebildeten Doppelstranges als Matrize für die Synthese durch die Polymerase auftritt. Dabei wirkt das Mittel der Strangverdrängung (beispielsweise Polymerase mit einer Strangverdrängungs-Fähigkeit), auf den ersten bereits vorgebildeten Doppelstrang (A1 und B1) und synthetisiert einen zu Strang A1 neuen komplementären Strang D1, wobei gleichzeitig der Strang B1 aus der Bindung mit dem Strang A1 verdrängt wird.

Unter dem Begriff "nukleinsäurevermittelte Strang-Verdrängung" wird eine Summe / Reihe von Zwischenschritten zusammengefasst, welche untereinander im Gleichgewicht stehen können, und im Ergebnis zur vorübergehenden oder dauerhaften Öffnung einer ersten vorgeformten Duplex (bestehend aus komplementären Strängen A1 und B1) und Ausbildung einer neuen zweiten Duplex (bestehend aus komplementären Strängen A1 und C1), wobei A1 und C1 einander komplementär sind.

Es ist bekannt, dass eine wesentliche strukturelle Voraussetzung für die Initiierung einer Strangverdrängung die Herbeiführung einer räumlichen Nähe zwischen einem Duplex-Ende (vorgeformter erster Duplex aus A1 und B1) und einem einzelsträngigen Strang (C1) ist, welcher die Strangverdrängung initiiert (wobei A1 und C1 einen komplementären Strang bilden können). Eine solche räumliche Nähe kann vorzugsweise mittels eines einzelsträngigen Überhanges (in der Literatur sind Beispiele mit kurzen Überhängen bekannt, im Englischen genannt "Toehold", siehe Literatur oben) herbeigeführt werden, welcher den einzelsträngigen Strang (C1) vorübergehend oder dauerhaft komplementär bindet, und somit komplementäre Seqmente des Stranges C1 und A1 in hinreichende Nähe bringt, so dass eine erfolgreiche Strangverdrängung des Stranges B1 initiiert werden kann. Die Effizienz der Initiierung der nukleinsäurevermittelten Strang-Verdrängung ist im Allgemeinen um so höher, je näher die komplementäre Segmente des Stranges A1 und C1 zueinander positioniert werden.

Eine weitere wesentliche strukturelle Voraussetzung für die effiziente Fortführung einer nukleinsäurevermittelten Strangverdrängung in inneren Segmenten liegt in hoher Komplementarität zwischen Strängen (z.B. zwischen A1 und C1), welche einen neuen Doppelstrang ausbilden müssen. So können beispielsweise einzelne Nukleiotid-Mutationen (in C1) zur Unterbrechung einer Strangverdrängung (beschrieben z.B. für Branch-Migration) führen.

Die vorliegende Erfindung macht Gebrauch von der Fähigkeit komplementärer Nukleinsäuren zu einer sequenzabhängigen nukleinsäurevermittelten Strangverdrängung.

Bevorzugte Ausführungsformen der Erfindung werden in den Figuren und Beispielen näher erläutert.
Fig. 1A
   Zeigt schematisch Komponenten des Amplifikationssystems im Ansatz vor Amplifikationsstart:
   Start-Nukleinsäure (Start-NS), welche eine Zielsequenz umfasst.
   Primer 1 (P1.1), Primer 2 (P2.1), ein Aktivator-Oligonukleotid (C1.1).
   Komponenten für Primer-Verlängerungsreaktion: Polymerase (Pol) und dNTPs
Fig. 1B
   Zeigt schematisch Ergebnis der Amplifikation:
   Primer-Verlängerungsprodukt P1.1-Ext ausgehnd von P1.1, Primer-Verlängerungsprodukt 2.1-Ext. Diese Produkte (P1.1-Ext, P2.1-Ext) können untereinader und mit Aktovator-Oligonukleotid unterschiedliche Komplexformen ausbilden (je nach Konzentrations-Verhältnis und Reaktionsbedingugnen). Im Einzelnen, können diese Formen Komplese aus P1.1-Ext / C1.1 und / oder P1.1-Ext und / oder P1.1-Ext / C1.1/ P2.1-Ext umfassen.
   Das P1-Ext umfasst ein 3'-Segment, welches nicht vom Aktivator-Oligonukleotid komplementär gebunden wird. Dieses Segment dient als Bindungspartner für die Oligonukleotid-Sonde.
Fig. 2A
   Zeigt schematisch Komponenten eines Detektionssystems
   Eine Oligonukleotid-Sonde (S1.1) ist ein Oligonukleotid, welches einen Fluoreszenz-Reporter (R) und einen Quencher (Q) umfasst. Im nicht an die komplementäre Sequenz gebundenen, vorwiegend einzelsträngigen Zustand der Sonde sind Reporter und Quencher räumlich nicht hinreichend getrennt, so dass Fluoreszenzsignal vom Reporter durch den Quencher vermindert wird. Bei einer vorwiegend komplementären Bindung an das 3'-Segment eines P1-Ext erfolgt räumliche Trennung von Quencher und Reporter, so dass Fluoreszenzsignal zunimmt.
Fig. 2B
   Zeigt schematisch Komponenten eines Detektionssystems
   Eine Oligonukleotid-Sonde (S2.1) ist ein Oligonukleotid, welches einen Fluoreszenz-Reporter (R) und einen Quencher (Q) umfasst. Die Sonde umfasst selbstkomplementäre Sequenzelemente (Stem-Segment 1 und Stem-Segment 2), welche durch ein Loop-Segment 3 voneinder getrennt sind. Bei einer komplementären Bindung des "Loop-Segment" 3 an ein Sequenz-Anteils des 3'-Segments eines P1-Ext erfolgt unter verwendeten Reaktionsbedigungen des Detektions-Schrittes eine räumliche Trennung von Quencher und Reporter, was zu einer Zunahme des Fluoreszenzsignal führt.
Fig. 3A
   Zeigt schematisch Komponenten eines Detektionssystems
   Eine Oligonukleotid-Sonde (S3.1) ist ein Oligonukleotid, welches einen Reporter und einen Quencher umfasst. Die Sonde umfasst selbstkomplementäre Sequenzelemente (Stem-Segment 1 und Stem-Segment 2), welche durch ein Loop-Segment-3 voneinder getrennt sind. Bei einer gleichzeitigen vorwiegend komplementären Bindung des Stem-Segmentes 1 und Sequenz-Segmentes 4 an das 3'-Segment eines P1-Ext erfolgt unter Reaktionsbedigungen des Detektionsschrittes die räumliche Trennung von Quencher und Reporter, so dass Fluoreszenzsignal des Reporters zunimmt.
Fig. 3B
   Zeigt schematisch Komponenten eines Detektionssystems
   Eine Oligonukleotid-Sonde (S4.1) ist ein Oligonukleotid, welches einen Reporter und einen Quencher umfasst. Die Sonde umfasst selbstkomplementäre Sequenzelemente (Stem-Segment 1 und Stem-Segment 2), welche durch ein Loop-Segment-3 voneinander getrennt sind. Die Sonde umfasst ein weiteres Sequenz-Segment 4, welches mit dem 3'-Segment des P1-Ext einen komplementären Strang bilden kann und als Primer von Polymerase verlängert werden kann. Bei Verlängerung durch die Polymerase kommt es zu Ausbildung eines Primer-Verlängerungsproduktes S4.1-Ext.
Fig. 4A und 4B
   Zeigt schematisch Komponenten eines weiteren Detektionssystems
   Ein Oligonukleotid (S5.1) und mindestens ein Aktivator-Oligonukleotid (A5.1), ein FRET-Paar, Donor und Reporter sind jeweils getrennt auf einem der beiden Oligonukleotide gekoppelt und dermassen angeordnet, dass es bei einer komplementären Bindung von beiden Oligonukleotiden an dasselbe P1-Ext zu einer räumlichen Nähe zwischen dem Donor und dem Reporter kommt, so dass Reporter vom Donor angeregt werden kann.
Fig. 5A und 5B
   Zeigt schematisch Komponenten eines weiteren Detektionssystems
   Ein Oligonukleotid (S7.1 bzw. S8.1) und mindestens ein Aktivator-Oligonukleotid (A7.1 bzw. A8.1) und ein FRET-Paar. Das Oligonukleotid S7.1 oder S8.1 kann bei einer komplementären Bindung an das P1-Ext von einer Polymerase unter Verdrängung des
   Aktivator-Oligonukleotides verlängert werden, so dass dabei ein S7.1-Verlängerungsprodukt (S7.1-Ext) oder S8.1-Verlängerungsprodukt (S8.1-Ext) generiert werden.
Fig. 6
   Zeigt schematisch Bindungs-Positionen der Oligonukleotid-Sonde am ersten Primer-Verlängerungsprodukt
Fig. 7 bis 10 zeigen schematisch das Zusammenwirken von Komponenten bei einer exponentiellen Amplifikation einer zu amplifizierenden Nukleinsäurekette.
Fig. 11 zeigt Ergebnisse aus Beispiel 1
Fig. 12 zeigt Ergebnisse aus Beispiel 2
Fig. 13 zeigt Ergebnisse aus Beispiel 3
Figur 14 - 18 zeigt schematisch die Struktur des ersten Primer-Oligonukleotids.
Figur 16 - 17 zeigt schematisch die Interaktion zwischen dem ersten Primer-Oligonukleotid und der Matrize, sowie die Synthese des ersten Primer-Verlängerungsprodukts.
Figur 18 zeigt schematisch die Struktur des Primer-Verlängerungsprodukts des ersten Primer-Oligonukleotids.
Figur 19 zeigt schematisch die Struktur des Aktivator-Oligonukleotids.

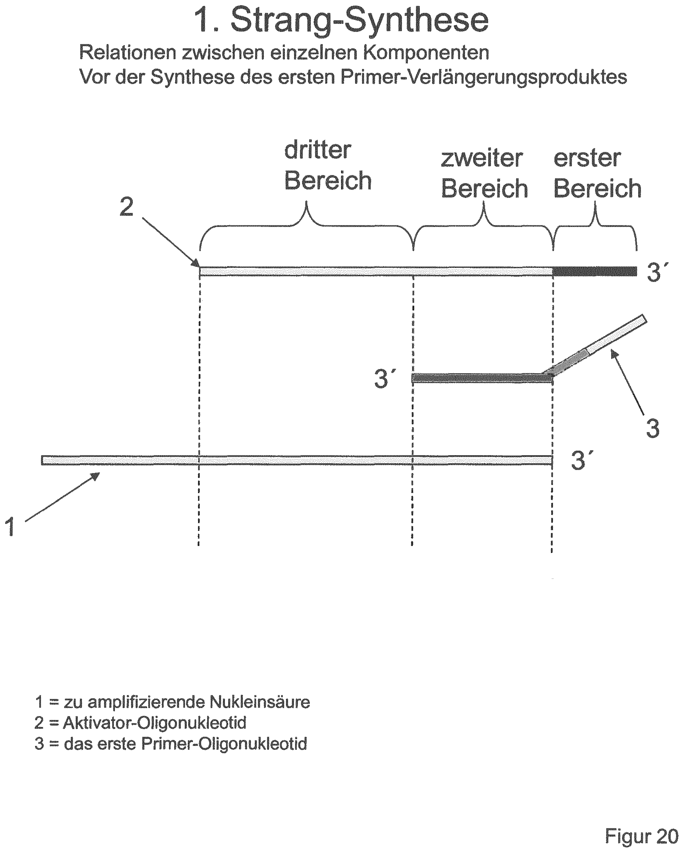

Figur 20 - 21 zeigt schematisch die Interaktion zwischen Strukturen während der Primer-Verlängung des ersten Primer-Oligonukleotids.
Figur 22 - 25 zeigt schematisch die Interaktion zwischen Strukturen während der Primer-Verlängerung des zweiten Primer-Oligonukleotids.

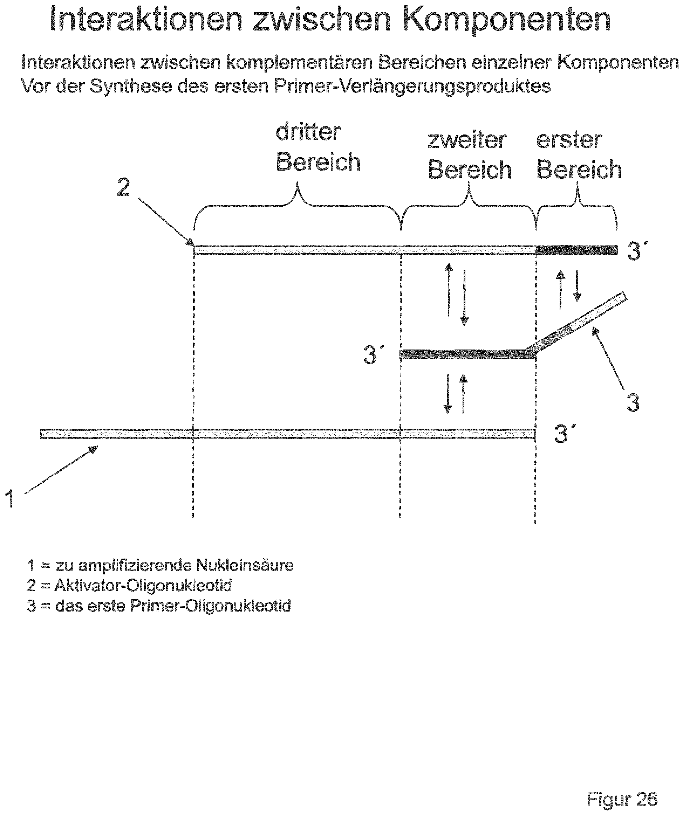

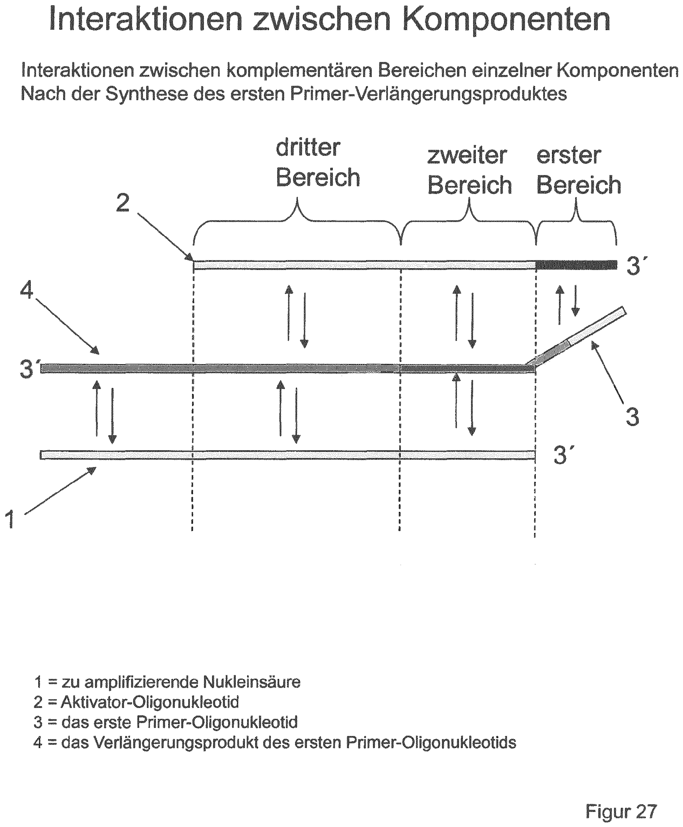

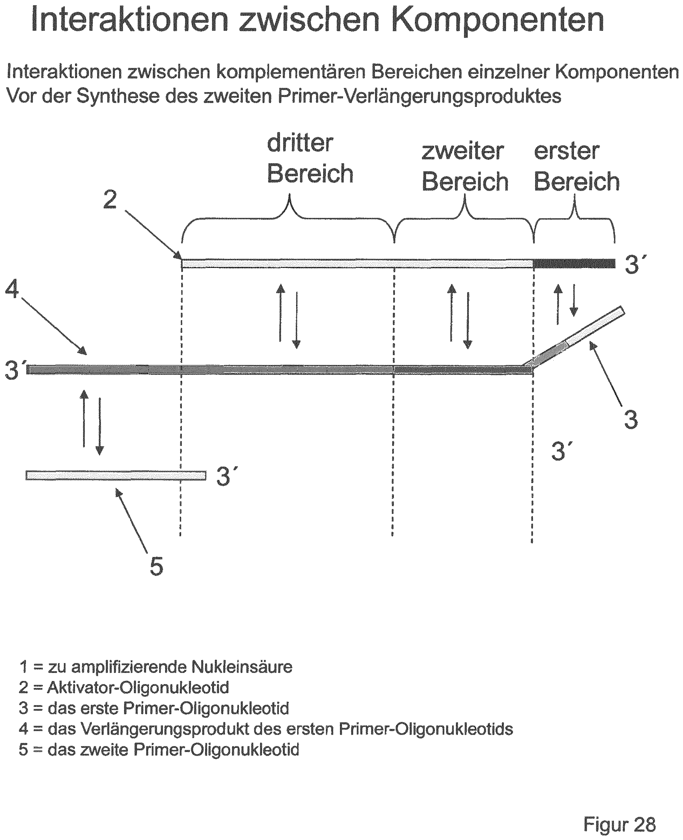

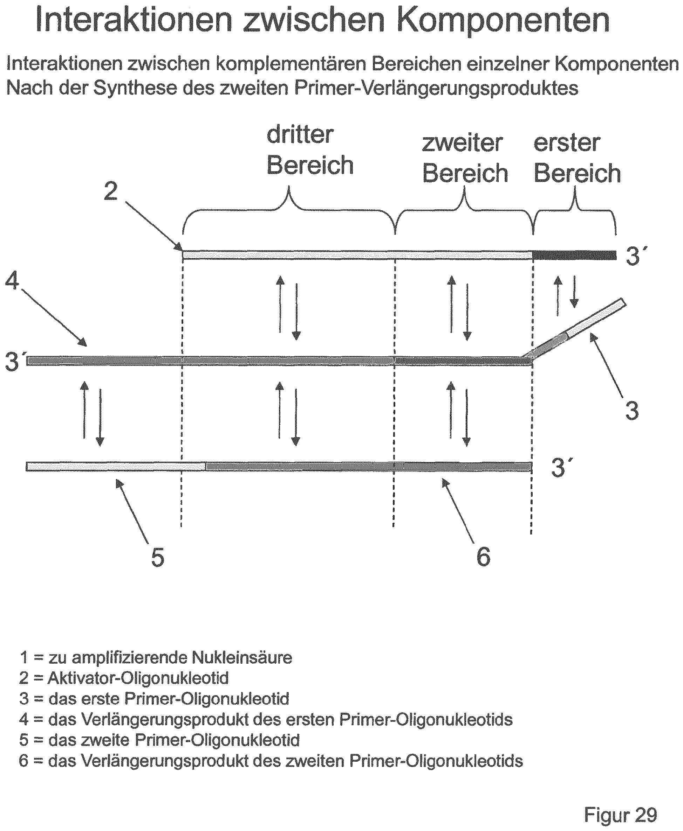

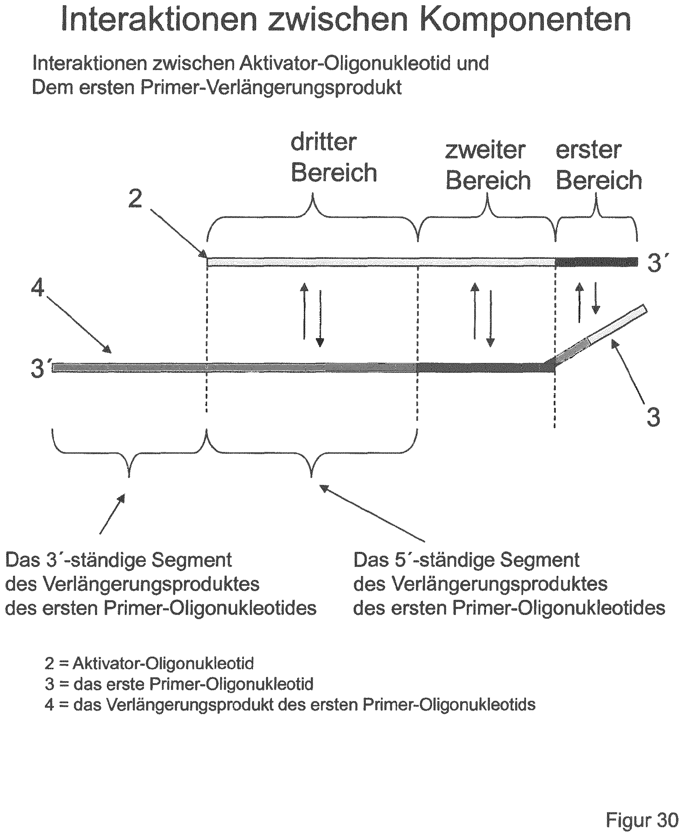

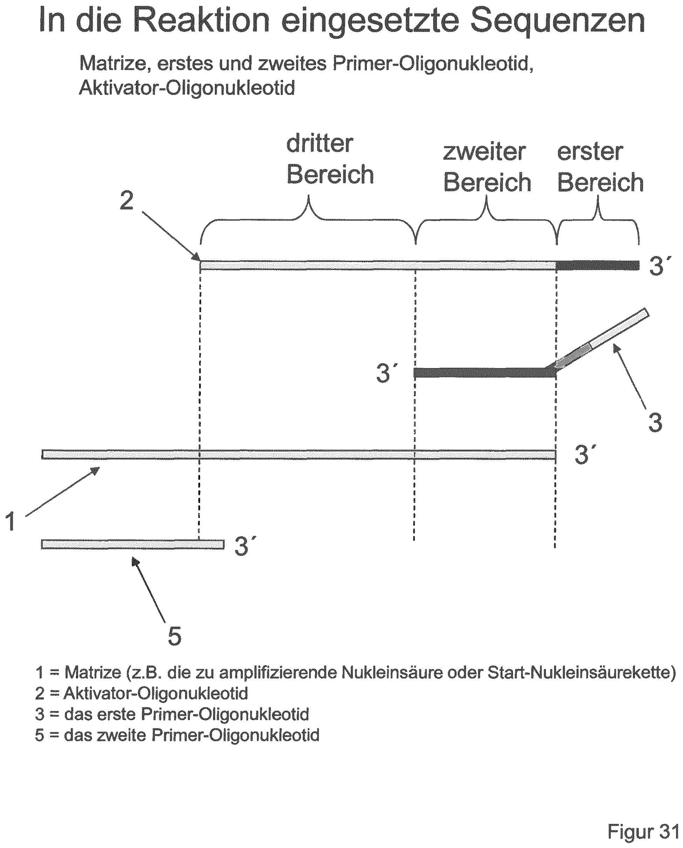

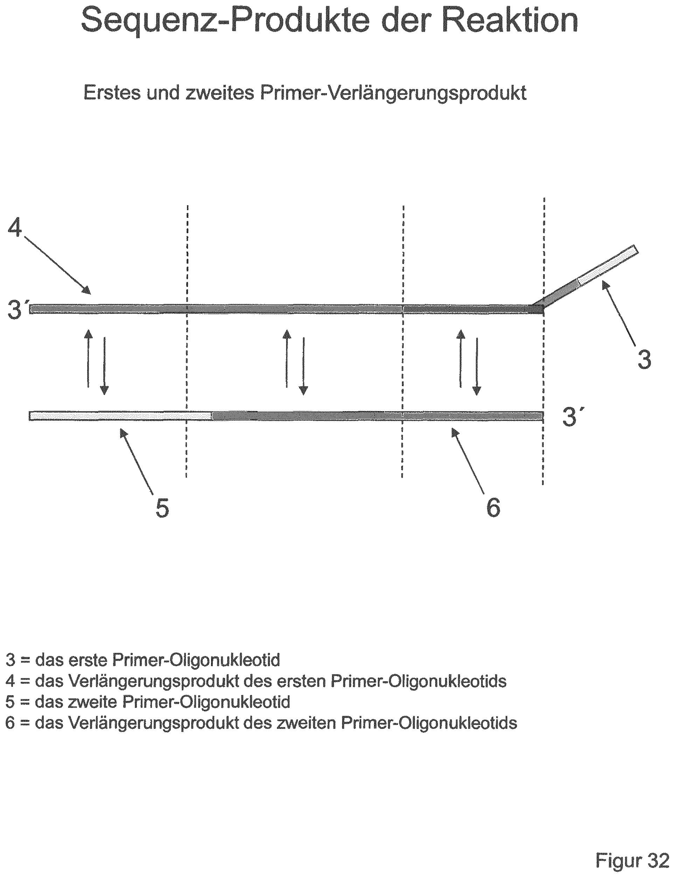

Figur 26 - 32 zeigt schematisch die Interaktion zwischen einzelnen Bereichen von Komponenten während der Amplifikation.

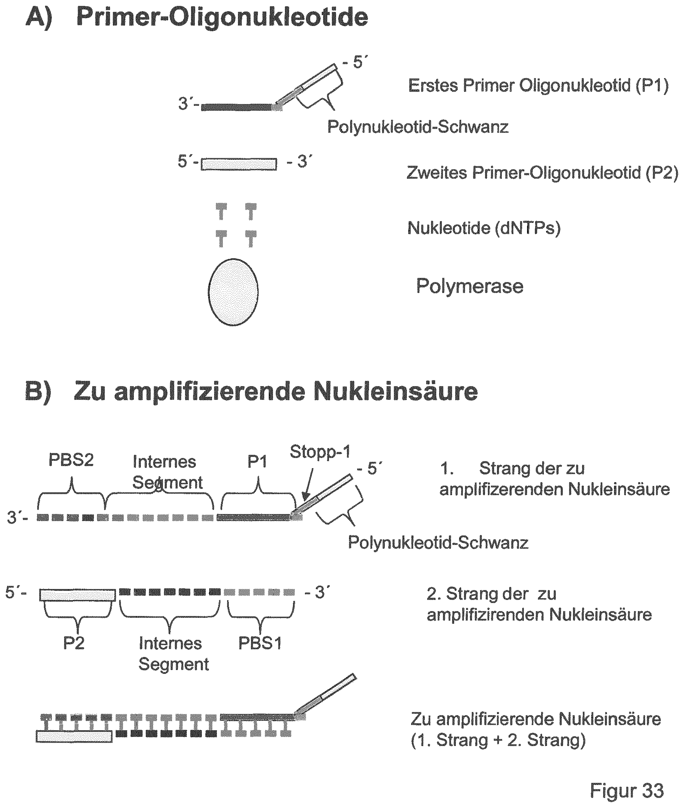

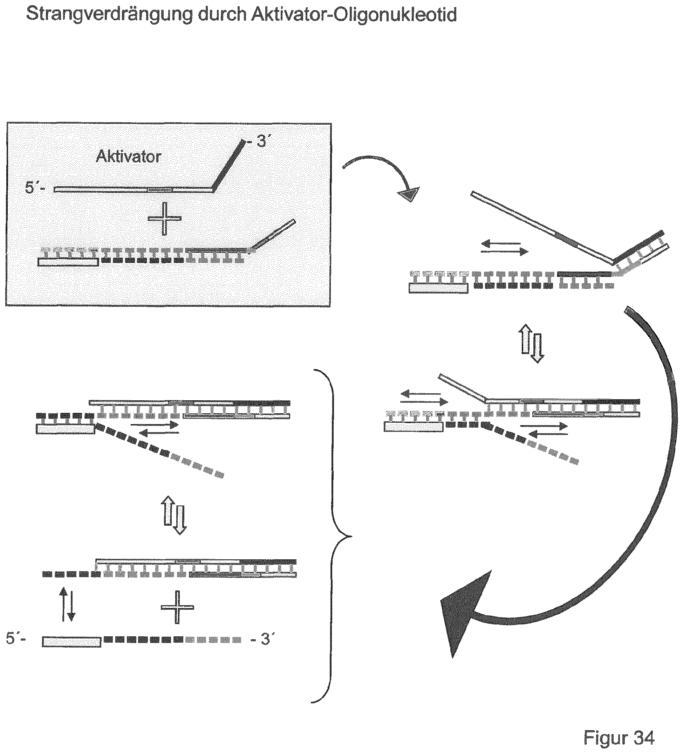

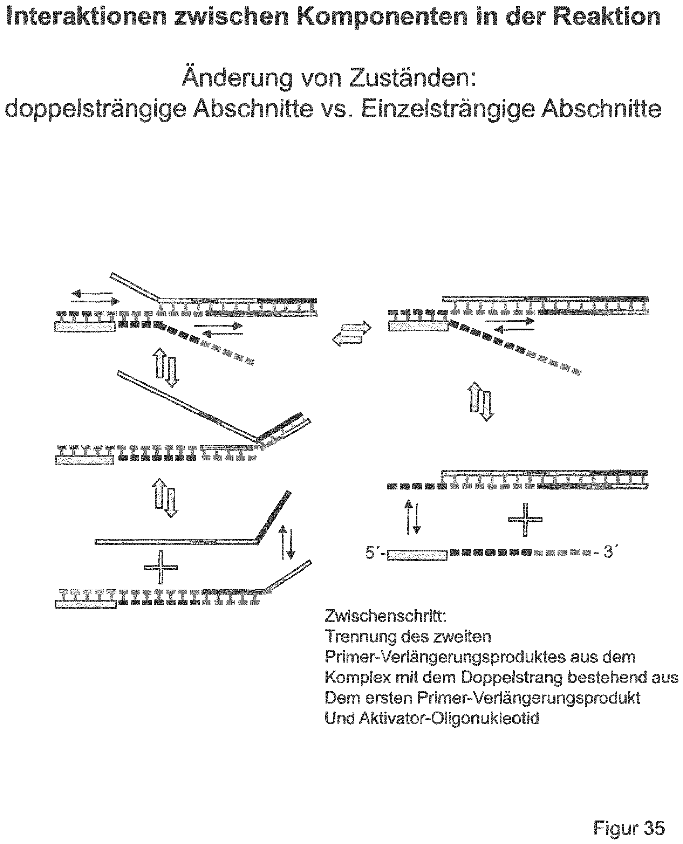

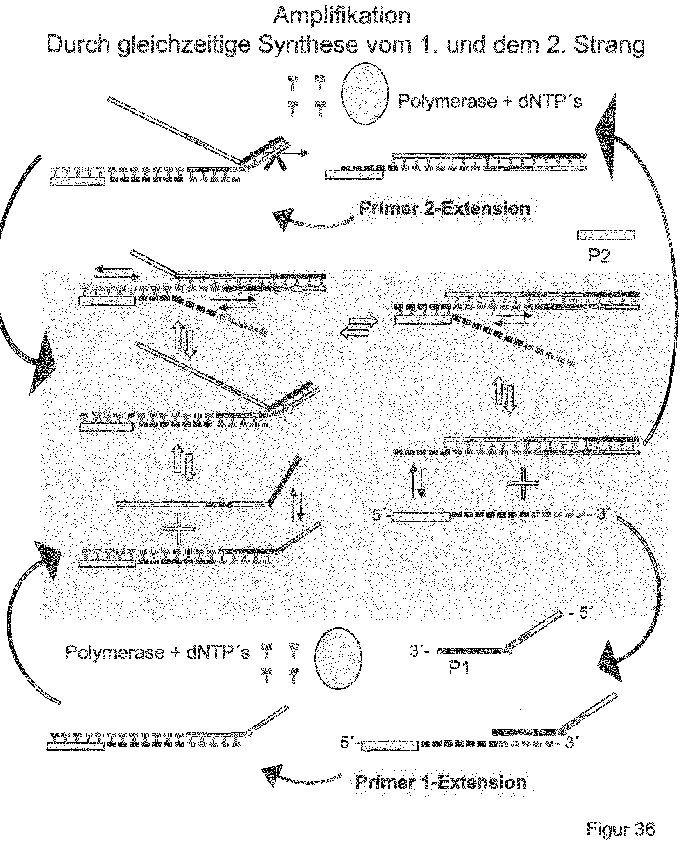

Figur 33 beschreibt mehr im Detail die Komponenten der in Fig. 34 - 36 dargestellten Strukturen.
Figur 34 zeigt schematisch den Strangverdrängungsmechanismus.
Figur 35 zeigt schematisch das Zusammenwirken der Strukturen bei Strangverdrängung.
Figur 36 zeigt schematisch das Zusammenwirken der Strukturen während der Nukleinsäureamplifikation durch einerseits die Amplifikation von erstem Primer-Oligonukleotid und zweitem Primer-Oligonukleotid und weiterhin die Einwirkung des Aktivator-Oligonukleotids und dabei resultierende Strangverdrängung.

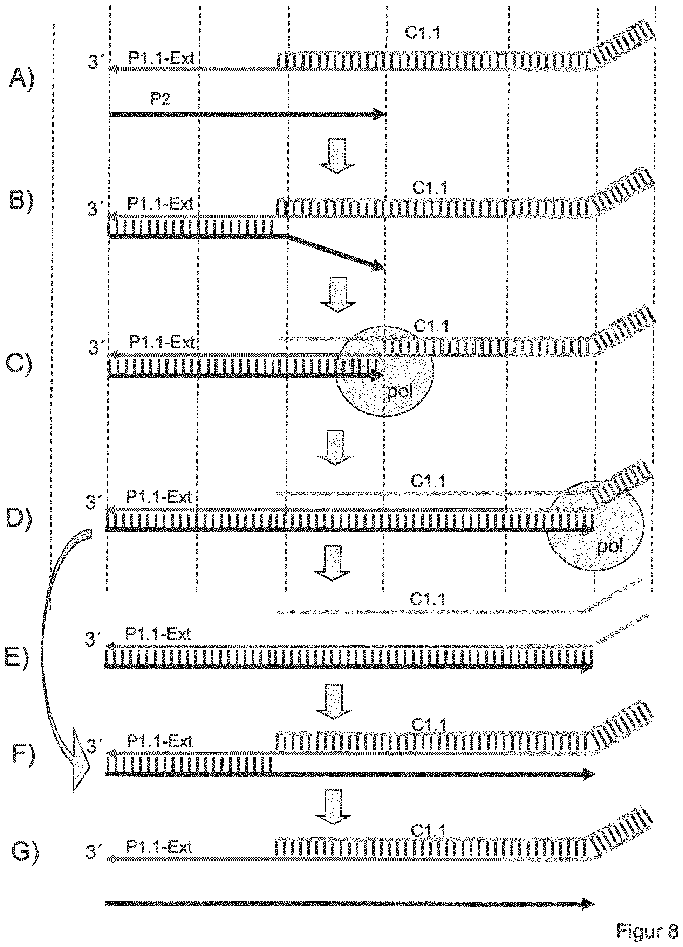

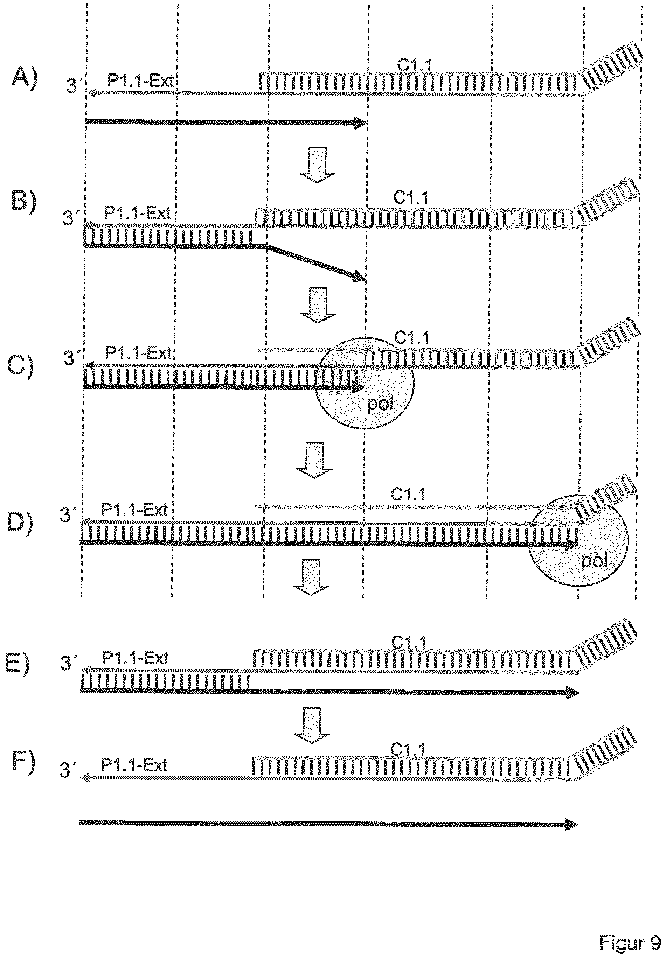

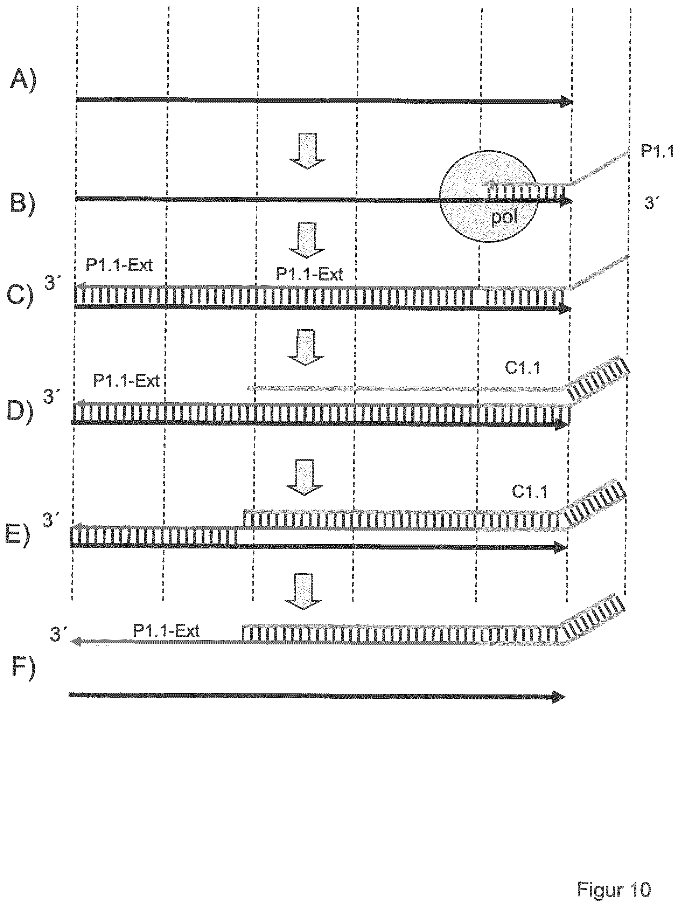

In dem erfindungsgemäßen Verfahren (Fig. 7 bis 10) kann als Ausgangsmaterial eine einzelsträngige Nukleinsäurekette dienen oder eine doppelsträngige Nukleinsäure, welche in einzelsträngige Form überführt wurde. Die Amplifikation erfolgt bevorzugt sequenzspezifisch, d.h. vorzugsweise wird die zu amplifizierende Nukleinsäure vervielfältigt.

Als Komponenten des Amplifikationssystems dienen eine zu amplifizierende Nukleinsäurekette, ein erstes spezifisches Primer-Oligonukleotid, ein zweiter Primer und ein Aktivator-Oligonukleotid, welches bei der Trennung der neu synthetisierten Stränge mitwirkt, sowie eine geeignete Polymerase und Substrate wie dNTP's. Die Amplifikation verläuft in einer Puffer-Lösung unter Bedingungen, welche eine Primer-Verlängerungsreaktion von beiden Primern zulassen, sowie welche eine Strangverdrängung durch Aktivator-Oligonukleotid zur Trennung von beiden Primer-Verlängerungsprodukten unterstützen.

In einer Ausführungsform sollen alle Verfahrensschritte unter Bedingungen verlaufen, welche keine Trennung von synthetisierten Primer-Verlängerungsproduken in Abwesenheit eines geeigneten Aktivator-Oligonukleotides zulassen. Beispielsweise ist die Temperatur der Reaktionslösung dermaßen gewählt, dass die Tm eines Doppelstranges aus beiden Primer-Verlängerungsprodukten signifikant über der Reaktionstemperatur liegt.

Unter diesen Voraussetzungen erfolgt eine Trennung von beiden Primer-Verlängerungsprodukten in Abhängigkeit von der Wirkung des Aktivator-Oligonukleotides. Dieses Aktivator-Oligonukleotid ist in der Lage, komplementär an das erste Primer-Verlängerungsprodukt zu binden und dadurch das zweite Primer-Verlängerungprodukt aus seiner Bindung mit dem ersten Primerverlängerungsprodukt zu verdrängen. Um diese Strangverdrängungsreaktion zu initiieren, ist das erste Primer-Oligonukleotid mit einem Polynukleotid-Schwanz in seinem zweiten Bereich versehen, welcher in der Lage ist, das Aktivator-Oligonukleotid unter Reaktionsbedingungen vorübergehend zu binden und damit eine räumliche Nähe zu anderen Bereichen des ersten Primer-Verlängerungsnukleotides herbeizuführen. Nach der Initiierung der Strangverdrängung durch das Aktivator-Oligonukleotid wird das zweite Primer-Verlängerungsprodukt aus seiner Bindung mit dem ersten Primer-Verlängerungsprodukt verdrängt. Sein 3'-ständiges Segment wird damit frei und steht für weitere Bindung eines ersten Primer-Oligonukleotides zur Verfügung.

Der Polynukleotid-Schwanz des ersten Primer-Oligonukleotides ist vorzugsweise nicht von einer Polymerase kopierbar. Das kann entweder durch Verwendung entsprechender Modifikationen in diesem Bereich erreicht werden oder durch Einführung einer ersten Blockierungseinheit zwischen dem ersten Primer-Bereich und dem zweiten Primer-Bereich des ersten Primer-Oligonukleotides.

Die Synthese des zweiten Primer-Verlängerungsproduktes erfolgt nach der Bindung des zweiten Primer-Oligonukleotides an das erste Primer-Verlängerungsprodukt in seinem 3'-ständigen Segment. Dieses Segment geht vorzugsweise keine Bindung mit dem Aktivator-Oligonukleotid ein und ist hinreichend lang, um das zweite Primer-Oligonukleotid zu binden und eine erfolgreiche Primer-Verlängerungsreaktion zu unterstützen. Die Synthese des zweiten Primer-Verlängerungsproduktes erfolgt unter Verdrängung des Aktivator-Oligonukleotides aus der Bindung mit dem ersten Primer-Verlängerungsprodukt. Dies kann beispielsweise durch Polymerase-abhängige Strangverdrängung oder auch durch Strangverdrängung mittels des zweiten Primers erfolgen.

Beide Primer-Verlängerungsprodukte schließen kopierbare Bereiche ein und dienen gegenseitig als Matrize. Das Aktivator-Oligonukleotid dient nicht als Matrize. Dies kann bevorzugt durch Verwendung von Nukleotid-Modifikationen erreicht werden, welche zwar eine komplementäre Bindung mit dem ersten Primer-Verlängerungsprodukt eingehen können, nicht aber von der Polymerase als Matrize akzeptiert werden. Beispiele für solche Nukleotid-Modifikationen stellen Nukleotid-Verbindungen mit modifizierten Phosphat-Zucker-Rückgrat-Anteilen, z.B. 2'-O-Alkyl-RNA Modifikationen (z.B. 2'-OMe), LNA-Modifikationen oder Morpholino-Modifikationen dar. Im Allgemeinen hindert die Anwesenheit solcher Modifikationen in einem Strang eine DNA-abhängige Polymerase beim Ablesen eines solchen Stranges. Die Anzahl von solchen Modifikationen kann unterschiedlich groß sein, in der Regel können wenige Modifikationen (zwischen 1 und 20) hinreichend sein, um eine Polymerase beim Ablesen eines solchen Stranges zu hindern. Solche Nukleotid-Modifikationen können beispielsweise an oder um die Bindungsstelle des ersten Primer-Oligonukleotides an das Aktivator-Oligonukleotid verwendet werden und/oder als Bestandteile des zweiten Bereichs des ersten Primer-Oligonukleotides.

Dank der Verwendung solcher Modifikationen kommt es zu einer lokalen Hinderung der Polymerase-Funktion, so dass bestimmte Segmente der verwendeten Strukturen nicht durch die Polymerase kopiert werden können und vorwiegend einzelsträngig bleiben. In dieser einzelsträngigen Form können sie weitere Reaktionskomponenten binden und somit ihre Funktion ausführen.

Unter Reaktionsbedingungen, welche einen Doppelstrang nicht denaturieren, führt die Verwendung der sequenzabhängigen nukleinsäurevermittelten Strangverdrängung zur sequenzspezifischen Trennung der beiden Primer-Verlängerungsprodukte während der Amplifikationsreaktion im beschriebenen Verfahren: eine hinreichende Komplementarität zwischen neusynthetisierten Verlängerungsfragmenten der Primer-Oligonukleotide mit der zu Beginn einer Amplifikation vorgegebenen Sequenz eines Aktivator-Oligonukleotides stellt eine Voraussetzung für eine erfolgreiche Strangverdrängung dar und kann somit Einfluss auf die Effizienz der Strangtrennung eines Doppelstranges (bestehend aus dem ersten und dem zweiten Primer-Verlängerungsprodukt) haben. Bei geringfügigen Abweichungen kommt es zu einer Verlangsamung der Strangverdrängung und somit auch zur Verlangsamung der Strangtrennung. Diese kann sich in einer Verlangsamung der Gesamtreaktion auswirken. Mit steigender Differenz in der Sequenz der neusynthetisierten Verlängerungsprodukte und der zu Beginn der Reaktion vorgegebenen Sequenz des Aktivator-Oligonukleotides kommt es zu einer immer höheren Hinderung der Strangverdrängung, welche im Endeffekt keine ausreichende Trennung von beiden Primer-Verlängerungsprodukten mehr leisten kann. Die beiden neu synthetisierten Stränge können nicht mehr ausreichend voneinander getrennt werden, so dass ihre Bindungsstellen für Primer-Oligonukleotide nicht mehr zugängig sind. Das führt im Allgemeinen zum Abbruch einer Amplifikation von Sequenzen mit Sequenzabweichungen.

Zusammengefasst können nicht nur die Spezifitäten der Bindung von beiden Primern mit ihren Matrizen, sondern auch die Beschaffenheit der Sequenzsegmente zwischen den Primern auf die Amplifikation Auswirkung haben, nämlich indem diese Abschnitte eine hinreichende Strangverdrängung zulassen oder nicht, je nach ihrer Übereinstimmung in der zu Beginn der Reaktion vorgegebenen Sequenz des Aktivator-Oligonukleotides. Das beschrieben Verfahren kann dadurch unter Umständen insgesamt eine höhere Spezifität vorweisen, als die herkömmlichen Amplifikations-Verfahren.

Die spezifische Amplifikation resultiert weiterhin durch Verwendung der Komponenten bei Reaktionsbedingungen, welche bevorzugt eine spontane Trennung von neu synthetisierten Primer-Verlängerungsprodukte nicht zulassen.

Das Verfahren umfasst mehrere Vorgänge, welche nachfolgend beschrieben sind. Diese Vorgänge können in einem Ansatz oder in getrennten Ansätzen ausgeführt werden. Wenn die Vorgänge in einem Ansatz durchgeführt werden sollen, so können diese unter gleichen Bedingungen, z.B. isothermal, oder unter unterschiedlichen Bedingungen, z.B. beim Thermocycling, ausgeführt werden. Vorzugsweise sind Primer-Oligonukleotide und Aktivator-Oligonukleotid zu Beginn der Reaktion präsent. Eine sequenzielle Zugabe einzelner Reagenzien ist allerdings auch möglich.

Ebenfalls sind Kombinationen mit anderen Amplifikationsverfahren möglich, z.B. mit PCR, wobei die PCR beispielsweise erst über 1 bis 10 Zyklen durchgeführt werden und anschließend wird beispielsweise unter isothermalen Bedingungen weitergearbeitet.

In einer vorteilhaften Form umfasst das Verfahren zum Monitoring/Detektion einer spezifischen Amplifizierung von Nukleinsäureketten folgende Schritte:
Ausführung einer Amplifikation einer zu amplifizierenden Nukleinsäurekette, welche folgende Schritte umfasst:
a) Hybridisierung eines ersten Primer-Oligonukleotids an das 3'-Segment eines Stranges einer zur amplifizierenden Nukleinsäurekette, wobei die zu amplifizierende Nukleinsäurekette eine Zielsequenz umfasst, wobei das erste Primer-Oligonukleotid umfasst:
   - Einen ersten Primer-Bereich im 3'-Segment des ersten Primer-Oligonukleotides, der an einen Strang einer zu amplifizierenden Nukleinsäurekette sequenzspezifisch binden kann,
   - einen zweiten Bereich, gekoppelt direkt oder via einen Linker an das 5'-Ende des ersten Primer-Bereichs des ersten Primer-Oligonukleotides, welcher einen Polynukleotid-Schwanz umfasst, welcher zur Bindung eines Aktivator-Oligonukleotids und Unterstützung der Strangverdrängung (Schritt c) durch Aktivator-Oligonukleotids geeignet ist, wobei der Polynukleotid-Schwanz von Polymerase unter den gewählten Reaktionsbedingungen im Wesentlichen unkopiert bleibt.
b) Extension des ersten Primer-Oligonukleotids mit Hilfe einer Polymerase zur Bildung eines ersten Primer-Verlängerungsproduktes, das zur Zielsequenz und/oder zu der zu amplifizierenden Nukleinsäurekette (a) komplementäre Sequenzabschnitte umfasst,
c) Bindung des Aktivator-Oligonukleotides an den Polynukleotid-Schwanz des zweiten Bereichs des ersten verlängerten Primer-Oligonukleotids, wobei das Aktivator-Oligonukleotid umfasst:
   - Einen ersten einzelsträngigen Bereich, welcher an den Polynukleotid-Schwanz des zweiten Bereichs des ersten Primer-Oligonukleotides binden kann,
   - einen zweiten einzelsträngigen Bereich, welcher zu dem ersten Bereich des ersten Primer-Oligonukleotides im Wesentlichen komplementär ist und an diesen binden kann,
   - einen dritten einzelsträngigen Bereich, welcher zumindest zu einem Segment des von Polymerase synthetisierten Verlängerungsproduktes des ersten Primer-Verlängerungsprodukt im Wesentlichen komplementär ist,
      wobei das Aktivator-Oligonukleotid nicht als Matrize für eine Primerverlängerung des ersten Primer-Oligonukleotid dient,
d) Bindung des Aktivator-Oligonukleotides an den ersten Primer-Bereich des ersten verlängerten Primer-Oligonukleotids, unter Verdrängung des zu diesem ersten Primer-Bereich komplementären Stranges der zur amplifizierenden Nukleinsäurekette,
e) Bindung des Aktivator-Oligonukleotides an das komplementäre Segment des Verlängerungsproduktes des ersten verlängerten Primer-Oligonukleotids, unter Verdrängung des zu diesem Verlängerungsprodukt komplementären Stranges der zur amplifizierenden Nukleinsäurekette, wobei das 3'-Segment des ersten Primer-Verlängerungsproduktes einzelsträngig wird,
f) Hybridisierung eines zweiten Oligonukleotid-Primers an das erste Primer-Verlängerungsprodukt, wobei das 3'-Segment des zweiten Oligonukleotid-Primers eine Sequenz umfasst, die an das erste Primer-Verlängerungsprodukt hybridisieren kann, und
g) Verlängerung des zweiten Oligonukleotid-Primers mit Polymerase zur Bildung eines zweiten Primer-Verlängerungsproduktes, wobei Verlängerung bis einschließlich des ersten Primer-Bereichs des ersten Primer-Oligonukleotids erfolgt und dieser erste Primer-Bereich von der Polymerase kopiert wird, wobei der Polynukleotid-Schwanz des zweiten Bereichs unkopiert bleibt,
h) Wiederholung der Schritte a)-g) bis das gewünschte Ausmaß der Amplifikation erreicht ist.
wobei der Reaktionsmischung ein Detektionssystem zugegeben wird.

Die Oligonukleotid-Sonde umfasst ein zum 3'-Segment des ersten Primer-Verlängerungsproduktes im Wesentlichen komplementäres Sequenzsegment, welches an das 3'-Segment des ersten Primer-Verlängerungsproduktes unter geeigneten Bedingungen hybridisieren kann (Hybridisierungsbedingungen), wobei zumindest ein Teil dieses komplementären Segmentes nicht mit dem dritten Bereich des Aktivator-Oligonukleotides in der Sequenzzusammensetzung identisch ist und die Bindung dieses genannten komplementären Segmentes der Oligonukleotid-Sonde erfolgt am 3'-Segment des ersten Primer-Verlängerungsproduktes in 3'-Richtung relativ zum gebundenen Aktivator-Oligonukleotid. In dieser Ausführungsform umfasst die Oligonukleotid-Sonde zumindest einen Fluoreszenz-Reporter und /oder einen Fluoreszenz-Donor und /oder einen Fluoreszenz-Quencher.

Die Bindung der Oligonukleotid-Sonde an das 3'-Segment des synthetisierten ersten Primer-Verlängerungsproduktes unter geeigneten Hybridisierungsbedingungen führt zu Ausbildung eines Doppelstranges. Die Hybridisierungsbedingungen liegen während des Verfahrens vor.

Zu geeigneten Zeitpunkten während des Verfahrens erfolgt eine Beleuchtung der Reaktion mit Licht einer geeigneten Wellenlänge für Generierung eines Fluoreszenzsignals und Detektion des Fluoreszenzsignals vom Fluoreszenzreporter, wobei die Intensität des Fluoreszenzsignals gemessen wird.

Die Detektion des Fluoreszenzsignals erfolgt mit geeigneten optisch/physikalischen Mitteln, bei denen entweder die Zunahme des Fluoreszenzsignals oder die Abnahme des Fluoreszenzsignals gemessen wird. Durch geeignete Sensoren kann die Intensität des Fluoreszenzsignals umgewandet werden in Messwerte, die nach entsprechender Kalibrierung eine Bestimmung dahingehend ermöglichen, ob eine gewünschte Zielsequenz in der Reaktionsmischung vorhanden ist oder nicht.

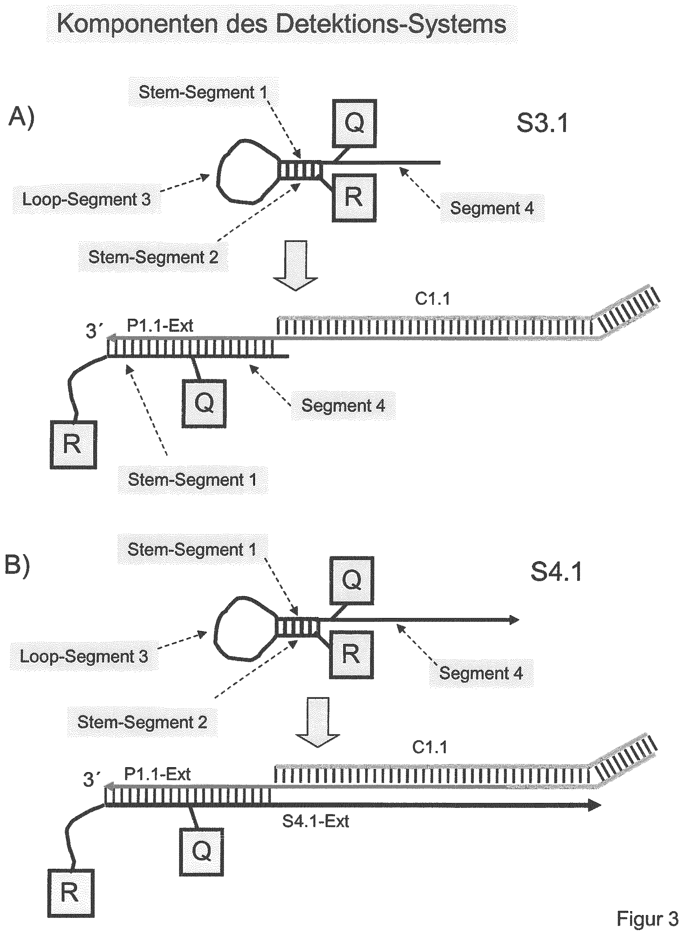

Ein wesentlicher Aspekt des Fluoreszenz-Detektionssystems ist das Reporter-Quencher-Paar. Wenn sich der Quencher in räumlicher Nähe zum Reporter befindet, wird bei Belichtung mit dem Anregungslicht kein Signal emittiert. Wenn, wie beispielsweise in Figur 3A dargestellt, Reporter- und Quenchermoleküle aufgrund einer komplementären Stem-Sequenz in naher räumlicher Nähe gehalten werden, tritt auch bei Beleuchtung kein Fluoreszenzsignal auf. Wenn aber das Fluoreszenzsystem dahingehend verändert wird, dass die zwischen Reporter und Quencher gelegene Nukleotidsequenz mit einer Zielsequenz hybridisieren kann, werden Reporter und Quencher in eine räumliche Entfernung verbracht, die bewirkt, dass der Quencher so weit von dem Reportermolekül entfernt ist, dass das Reportermolekül dann eine Fluoreszenzstrahlung emittiert, wenn die Reaktionsmischung mit einer Anregungslichtquelle bestrahlt wird. Der Abstand zwischen Reporter und Quencher hängt von den jeweils verwendeten Molekülen ab. Üblicherweise wird die Emission von Licht nach Bestrahlung des Fluoreszenzreporters dann reduziert oder nahezu vollständig vermindert, wenn Quencher und Reporter in einer Entfernung von weniger als 25 Nukleotiden voneinander entfernt sind. Diese Entfernung kann entweder durch die Nukleotidsequenz bewirkt werden oder durch spezielle Moleküle, die den Abstand bewirken, wie Linker oder Spacer.

In einer weiteren Ausführungsform kann der Fluoreszenzreporter ein spezifischer Reporter-Donor-Paar (FRET-Paar) mit einem Donor-Fluorophor bilden, welches in der Lage ist, die absorbierte Energie auf Fluoreszenzreporter durch Fluoreszenz-Resonanz-Energie-Transfer (FRET) zu übertragen.

Ein Reporter-Donor-Paar welches einen Fluoreszenzreporter und ein passendes Donor-Fluorophor umfasst, und ein Fluoreszenz-Resonanz-Energie-Transfer-Paar bildet, kann so beschaffen sein, dass nur einer der Partner eines solchen FRET-Paares an der Oligonukleotid-Sonde gekoppelt ist und der andere Partner am Aktivator-Oligonukleotid gekoppelt ist. Sowohl Fluoreszenzreporter als auch Donor-Fluorophor sind am jeweiligen Oligonukleotid dermassen gekoppelt, dass im nicht-hybridisieren Zustand der Oligonukleotid-Sonde das Donor-Fluorophor nicht in der Lage ist, die absorbierte Energie auf den Fluoreszenzreporters zu übertragen. Durch eine gleichzeitige Hybridisierung des Aktivator-Oligonukleotides an das synthetisierte erste Primer-Verlängerungsprodukt und der Oligonukleotid-Sonde an das 3'-Ende desselben ersten Primer-Verlängerungsproduktes erfolgt eine Bindung an benachbarten Sequenzpositionen des ersten Primer-Verlängerungsproduktes, was in räumlicher Annäherung des Donor-Fluorophores und des Fuoreszenzreporters resultiert, und der Abstand zwischen dem Fluoreszenreporter und Donor-Fluorophor dermaßen verringert wird, dass FRET von Donor-Fluorophor auf Fluoreszenzreporter erfolgen kann. Dies führt zur Generierung eines Fluoreszenzsignals des Fluoreszenzreporters und resultiert in einer erfassbaren Zunahme des Fluoreszenzsignals des Fluoreszenzreporters.

Der Abstand zwischen Donor-Fluorophor und dem Fluoreszenzreporter soll nach Hybridisierung weniger als 25, bevorzugt weniger als 15 und besonders bevorzugt weniger als 5 Nukleotide darstellen. Die Wellenlänge des Lichts bei Anregung wird vom Donor-Fluorophor absorbiert und auf den Reporter übertragen, wodurch Licht emittiert wird, das detektiert werden kann.

Was die Anordnung des Detektionssystems im Amplifikationsprodukt angeht, sind folgende Ausführungsformen bevorzugt, wobei entweder der Fluoreszenzreporter oder der Fluoreszenzquencher oder das Donor-Fluorophor entweder am Aktivator-Oligonukleotid, insbesondere im dritten Bereich des Aktivator-Oligonukleotids gekoppelt sind:
- Die Kopplung am Aktivator-Oligonukleotid erfolgt bevorzugt im dritten Bereich des Aktivator-Oligonukleotides,
- Die Kopplung am Aktivator-Oligonukleotid erfolgt bevorzugt am 5'-Ende oder des dritten Bereichs oder in seiner Nähe, z.B. 2 bis etwa 10 Nukleotide vom 5'-Ende des dritten Bereichs
- Kopplung an der Oligonukleotid-Sonde erfolgt bevorzugt im mittleren Bereich der Oligonukleotid-Sonde,
- Kopplung an der Oligonukleotid-Sonde erfolgt bevorzugt im 3'-Segment der Oligonukleotid-Sonde.

Im Rahmen der vorliegenden Erfindung sind folgende Aspekte besonders bevorzugt:
- Der Abstand zwischen beiden Elementen eines FRET-Paares im hybridisierten Zustand von Oligonukleotiden, welche an das erste Primer-Verlängerungsprodukt hybridisiert sind, beträgt weniger als etwa 30 Nukleotide.
- Die Oligonukleotid-Sonde, die ein komplementäres 3'-Ende zum ersten Primer-Verlängerungprodukt umfasst, ist dermaßen modifiziert, dass die Polymerase nicht in der Lage ist, dieses 3'-Ende zu verlängern.
- Die Oligonukleotid-Sonde umfasst ein komplementäres 3'-Ende zum ersten Primer-Verlängerungprodukt, wobei die Polymerase dieses 3'-Ende unter Verwendung des ersten Primer-Verlängerungsproduktes als Matrize verlängern kann unter Ausbildung eines komplementäres Stranges.
- Die Oligonukleotid-Sonde umfasst ein komplementäres 3'-Ende zum ersten Primer-Verlängerungprodukt, wobei die Polymerase dieses 3'-Ende unter Verwendung des ersten Primer-Verlängerungsproduktes als Matrize verlängern kann unter Ausbildung eines komplementäres Stranges, wobei die Oligonukleotid-Sonde und der zweite Amplifikations-Primer unterschiedlich sind.
- Verfahren zur Detektion von Amplifikation, wobei zwei oder mehr Nukleinsäureketten amplifiziert werden, bei welchem für jede zu amplifizierende Nukleinsäurekette ein spezifisches Detektions-System verwendet wird.
- Verfahren zur Detektion von Amplifikation, wobei zwei oder mehr zu amplifizierende Nukleinsäureketten amplifiziert werden, bei welchem die Detektion der Amplifikation von mindestens zwei zu amplifizierenden Nukleinsäureketten durch ein einheitliches Detektions-System erfolgt.
- Die Amplifikation umfasst eine asymetrische Vermehrung des ersten und des zweiten Primer-Verlängerungsproduktes.
- Die asymetrische Amplifikation resultiert in einem relativen Überschuss der ersten Primer-Verlängerungsprodukte, so dass mehr erste Primer-Verlängerungsprodukte gebildet werden als zweite Primer-Verlängerungsprodukte.
- Die Anregung des Fluoreszenzreporters und die Messung des Fluoreszenzsignals des Fluoreszenzreporters erfolgt während der Amplifikation.
- Die Anregung des Donor-Fluorophores und die Messung des Fluoreszenzsignals des Fluoreszenzreporters erfolgt während der Amplifikation.
- Die Anregung des Fluoreszenzreporters und die Messung des Fluoreszenzsignals des Fluoreszenzreporters erfolgt nach der Amplifikation.
- Die Anregung des Donor-Fluorophores und die Messung des Fluoreszenzsignals des Fluoreszenzreporters erfolgt nach der Amplifikation.
- Die Oligonukleotid-Sonde ist ein DNA-Oligonukleotid.
- Die Oligonukleotid-Sonde umfasst ein komplementäres Sequenz-Segment des ersten Primer-Verlängerungsprodukt, wobei dieses Sequenz-Segment eine Länge von 8 bis etwa 60 Nukleotide umfasst.
- Eine Oligonukleotid-Sonde umfasst ein komplementäres Sequenz-Segment zum 3'-Segment des ersten Primer-Verlängerungsprodukt, welches nicht vom Aktivator-Oligonukleotid gebunden wird, wobei dieses Segment eine Länge von 8 bis etwa 40 Nukleotide umfasst.
- Eine Oligonukleotid-Sonde umfasst selbstkomplementäre Sequenzsegmente (Stem-Segment 1 und 2), wobei diese selbstkomplementären Sequenzsegmente durch ein nicht-selbstkomplementeres Sequenzsegment von einander getrennt sind (Loop-Segment).
- Eine Oligonukleotid-Sonde umfasst selbstkomplementäre Sequenzsegmente (Stem-Segment 1 und 2), wobei diese selbstkomplementären Sequenzsegmente eine Länge von 3 bis etwa 20 Nukleotiden umfassen.
- Eine Oligonukleotid-Sonde kann ein Loop-Segment zwischen beiden selbstkomplementeren Sequenzsegmenten in einer Länge von etwa 2 bis etwa 40 Nukleotide umfassen.
- Eine Oligonukleotid-Sonde umfasst selbstkomplementäre Sequenzsegmente (Stem-Segment 1 und 2), wobei diese selbstkomplementären Sequenzsegmente durch ein nicht-selbstkomplementeres Sequenzsegment von einander getrennt sind (Loop-Segment), wobei eines der beiden selbstkomplementären Sequenzsegmente an das 3'-Segment des ersten Primer-Verlängerungsproduktes komplementär binden kann.
- Eine Oligonukleotid-Sonde umfasst selbstkomplementäre Sequenzsegmente (Stem-Segment 1 und 2), wobei diese selbstkomplementären Sequenzsegmente durch ein nicht-selbstkomplementeres Sequenzsegment von einander getrennt sind (Loop-Segment), wobei das Loop-Segment an das 3'-Segment des ersten Primer-Verlängerungsproduktes komplementär binden kann.
- Eine Oligonukleotid-Sonde umfasst selbstkomplementäre Sequenzsegmente (Stem-Segment 1 und 2), wobei diese selbstkomplementären Sequenzsegmente durch ein nicht-selbstkomplementeres Sequenzsegment von einander getrennt sind (Loop-Segment) und Fluoreszenzreporter und Fluoreszenzquencher an jeweils unterschiedliche selbstkomplementären Sequenzsegmente gebunden sind.
- Eine Oligonukleotid-Sonde umfasst selbstkomplementäre Sequenzsegmente (Stem-Segment 1 und 2), wobei diese selbstkomplementären Sequenzsegmente durch ein nicht-selbstkomplementeres Sequenzsegment von einander getrennt sind (Loop-Segment) und weiterhin ein viertes Sequenzsegment umfasst, welches an das erste Primer-Verlängerungsprodukt komplementär binden kann und nicht Stem-Segment 1,2 oder Loop-Segment ist.
- Eine Oligonukleotid-Sonde umfasst mindestens eine weitere Modifkation, ausgewählt aus der folgenden Gruppe: Linker (z.B. HEG, C3, C6), Phosphat-Zucker-Rückgrad Modifikationen (z.B. PTO, 2'-O-Me, RNA, PNA, LNA Modifikationen).

In einer Ausführungsform wird das Verfahren unter Bedingungen ausgeführt, welche keine Trennung von komplementären Strängen der zu amplifizierenden Nukleinsäure in Abwesenheit von Aktivator-Oligonukleotid zulassen.

In einer Ausführungsform wird das Kopieren des Polynukleotid-Schwanzes im zweiten Primer-Bereich durch einen Stopp Bereich für die Polymerase bewirkt, der zwischen dem ersten und dem zweiten Bereich angeordnet ist.

In einer Ausführungsform ist der dritte einzelsträngige Bereich des Aktivator-Oligonukleotid zum Segment des von Polymerase synthetisierten Verlängerungsprodukts des ersten Primer-Verlängerungsprodukts, welches unmittelbar an den ersten Primer-Bereich anschließt, im Wesentlichen komplementär ist, wobei:
In einer Ausführungsform ist der dritte einzelsträngige Bereich des Aktivator-Oligonukleotides zum genannten 5'-Segment des Verlängerungsprodukts des ersten Primer-Verlängerungsprodutkes vollständig komplementär, wobei die Länge dieses komplemetären Sequenzabschnittes folgende Bereiche umfasst: von mindestens 3 bis 70 Nukleotide, besser von mindestens 5 bis 50 Nukleotide, bevorzugt von 5 bis 40 Nukleotide, weiter bevorzugt von 5 bis 30 Nukleotide, besonders bevorzugt von 5 bis 20 Nukleotide.

In einer weiteren Ausführungsform umfassen die Sequenzen des dritten einzelsträngigen Bereich des Aktivator-Oligonukleotides und die entsprechenden Sequenz des genannten 5'-Segments des Verlängerungsprodukts des ersten Primer-Verlängerungsproduktes komplementäre Sequenzen bis auf eine Sequenzposition (ein Nukleotid-Paar / ein Basenpaar) mit nicht komplementärer Basenpaarung (im Sinne von Watson-Crick Basenpaarung) über eine Länge von mindestens 3 bis 70 Nukleotiden, besser von mindestens 5 bis 60 Nukleotiden, bevorzugt von 10 bis 40 Nukleotiden, besonders bevorzugt von 10 bis 20 Nukleotiden. Die Bindung des Aktivator-Oligonukleotides erfolgt somit im Wesentlichen komplementär.

In einer weiteren Ausführungsform umfassen die Sequenzen des dritten einzelsträngigen Bereich des Aktivator-Oligonukleotides und die entsprechenden Sequenzen des genannten 5'-Segments des Verlängerungsprodukts des ersten Primer-Verlängerungsproduktes komplementäre Sequenzen bis auf zwei Sequenzpositionen (ein Nukleotid-Paar / ein Basenpaar) mit nicht komplementärer Basenpaarung (im Sinne von Watson-Crick Basenpaarung) über eine Länge von mindestens 3 bis 70 Nukleotiden, besser von mindestens 5 bis 60 Nukleotiden, bevorzugt von 10 bis 40 Nukleotiden, besonders bevorzugt von 10 bis 20 Nukleotiden. Die Bindung des Aktivator-Oligonukleotides erfolgt somit im Wesentlichen komplementär.

In einer weiteren Ausführungsform umfassen die Sequenzen des dritten einzelsträngigen Bereichs des Aktivator-Oligonukleotides und die Sequenz des genannten 5'-Segments des Verlängerungsprodukts des ersten Primer-Verlängerungsproduktes komplementäre Sequenzen (im Sinne von Watson-Crick Basenpaarung) über eine Länge von mindestens 3 bis 70 Nukleotiden, besser von mindestens 5 bis 60 Nukleotiden, bevorzugt von 10 bis 40 Nukleotiden, besonders bevorzugt von 10 bis 20 Nukleotiden, weiterhin umfassen genannte Segmente nicht komplementäre Bereiche an mindestens drei Sequenzpositionen, wobei diese Positionen im 5'-Segment des dritten Abschnitt des Aktivator-Oligonukleotides liegen. Die Bindung des Aktivator-Oligonukleotides erfolgt somit im Wesentlichen komplementär.

In einer weiteren Ausführungsform umfassen die Sequenzen des dritten einzelsträngigen Bereichs des Aktivator-Oligonukleotides und die Sequenz des genannten 5'-Segments des Verlängerungsprodukts des ersten Primer-Verlängerungsprodutkes komplementäre Sequenzen bis auf mindestens eine und maximal zehn Sequenzpositionen mit nicht komplementärer Basenpaarung (im Sinne von Watson-Crick Basenpaarung) über eine Länge von mindestens 3 bis 70 Nukleotiden, besser von mindestens 5 bis 60 Nukleotiden, bevorzugt von 10 bis 40 Nukleotiden, besonders bevorzugt von 10 bis 20 Nukleotiden, wobei an Sequenzpositionen mit nicht komplementärer Basenpaarung (im Sinne von Watson-Crick Basenpaarung) mindestens ein modifiziertes Nukleotid mit modifizierten Nukleobasen beteiligt sind. Solche modifizierte Nukleobasen umfassen beispielsweise Nukleobasen mit verstärkter Bindung von natürlichen Nukleobasen (z.B. 2-Amino-Adenine), oder mit abgeschwächter Bindung, wie beispielsweise sogenannte universale Basen, wie Inosine oder 5-Nitroindol. Die modifizierten Nukleobasen sind vorzugsweise im dritten Sequenzbereich des Aktivator-Oligonukleotides lokalisiert. Die Bindung des Aktivator-Oligonukleotides erfolgt somit im Wesentlichen komplementär.

In einer weiteren Ausführungsform des Verfahrens wird Schritt (e) des Verfahrens weiterhin modifiziert und umfasst:
Bindung des Aktivator-Oligonukleotides an das komplementäre Segment des Verlängerungsproduktes des ersten verlängerten Primer-Oligonukleotids, unter Verdrängung des zu diesem Verlängerungsprodukt komplementären Stranges der zur amplifizierenden Nukleinsäurekette, bis dieser komplementärer Strang der zu amplifizierenden Nukleinsäure vom ersten Primer-Verlängerungsprodukt abgelöst wird, wobei das 3'-Segment des ersten Primer-Verlängerungsproduktes einzelsträngig wird.

In einer weiteren Ausführungsform des Verfahrens wird Schritt (f) des Verfahrens weiterhin modifiziert und umfasst:
Hybridisierung eines zweiten Oligonukleotid-Primers an das erste Primer-Verlängerungsprodukt, wobei gleichzeitig zumindest partielle Verdrängung des Aktivator-Oligonukleotides aus der Bindung mit dem ersten Verlängerungsprodukt durch Strangverdrängung erfolgt.

In einer weiteren Ausführungsform des Verfahrens wird Schritt (g) des Verfahrens weiterhin modifiziert und umfasst eine Verdrängung des Aktivator-Oligonukleotides aus der Bindung mit dem ersten Primer-Verlängerungsprodukt unter Mitwirkung der Polymerase.

In einer weiteren Ausführungsform des Verfahrens wird Schritt (h) des Verfahrens weiterhin modifiziert und umfasst: ggf. Bindung des Aktivator-Oligonukleotides an den unkopierten Polynukleotid-Schwanz des ersten verlängerten Primer-Oligonukleotid und Verdrängung des zweiten Primer-Verlängerungsprodukts aus der Bindung an das erste Primer-Verlängerungsprodukt unter gleichzeitiger Ausbildung eines komplementären Doppelstranges mit einem Segment des ersten spezifischen Verlängerungsproduktes des ersten Primer-Oligonukleotides.

In einer weiteren Ausführungsform des Verfahrens wird Verfahren weiterhin modifiziert und umfasst: h) Fortführung der Reaktion unter Bedingungen, welche Wiederholung von Schritten (a) bis (g) zulassen.

In einer weiteren Ausführungsform des Verfahrens wird des Verfahrens weiterhin modifiziert und umfasst: gleichzeitige Amplifizierung des ersten und zweiten Primer-Verlängerungsproduktes in einer exponentiellen Reaktion unter Verwendung des ersten und zweiten Primer-Oligonukleotid und des Aktivator-Oligonukleotides, wobei die gebildeten Primer-Verlängerungsprodukte als Matrizen für gegenseitige Synthese auftreten.

### Reaktionsbedingungen

Reaktionsbedingungen umfassen unter anderem Puffer-Bedingungen, TemperaturBedingungen, Zeitdauer der Reaktion und Konzentrationen von jeweiligen Reaktionskomponenten.

Im Verlauf der Reaktion akkumuliert die Menge der spezifischen produzierten zu amplifizierenden Nukleinsäure in einer exponentiellen Weise. Die Reaktion umfassend die Synthese der Verlängerungsprodukte kann zur Produktion der gewünschten Menge der spezifischen Nukleinsäuresequenz so lange wie nötig durchgeführt werden. Das erfindungsgemäße Verfahren wird bevorzugt kontinuierlich durchgeführt. In bevorzugter Ausführungsform läuft die Amplifikations-Reaktion bei gleicher Reaktionstemperatur ab, wobei die Temperatur bevorzugt zwischen 50°C und 70°C liegt. In einer anderen Ausführungsform kann die Reaktionstemperatur auch variabel gesteuert werden, so dass einzelne Schritte der Amplifikation bei jeweils unterschiedlichen Temperaturen verlaufen.

Die für die exponentielle Amplifikation benötigten Reagenzien sind vorzugsweise bereits zu Beginn einer Reaktion im gleichen Ansatz vorhanden. In einer anderen Ausführungsform können Reagenzien auch in späteren Stadien des Verfahrens zugegeben werden. Bevorzugt werden keine Helicasen oder Recombinasen in der Reaktionsmischung zur Trennung der neu synthetisieren Doppelstränge der zu amplifizierenden Nukleinsäure verwendet.

In einer bevorzugten Ausführungsform beinhaltet die Reaktionsmischung keine biochemischen Energie-spendenden Verbindungen wie ATP.

Die Menge der zu Beginn der Reaktion vorliegenden zu amplifizierenden Nukleinsäure kann zwischen wenigen Kopien und mehreren Milliarden Kopien in einem Ansatz vorliegen. Bei diagnostischen Einsätzen kann die Menge der zu amplifizierenden Nukleinsäurekette unbekannt sein.

In der Reaktion können auch weitere, nicht zu amplifizierende Nukleinsäuren vorliegen. Diese Nukleinsäuren können von natürlichen DNA oder RNA oder ihren Äquivalenten abstammen. In einer Ausführungsform liegen Kontroll-Sequenzen im gleichen Ansatz vor, welche parallel zu der zu amplifizierenden Nukleinsäure ebenfalls amplifiziert werden sollen.

Bevorzugt wird ein molarer Überschuss von ungefähr 10³:1 bis ungefähr 10¹⁵:1 (Verhältnis Primer:Matrize) der eingesetzter Primer und des Aktivator-Oligonukleotides zu der Reaktionsmischung gegeben, welche Matrizen-Stränge für die Synthese der zu amplifizierenden Nukleinsäurekette umfasst.

Es kann die Menge der Zielnukleinsäuren nicht bekannt sein, wenn das erfindungsgemäße Verfahren in diagnostischen Anwendungen benutzt wird, so dass die relative Menge des Primers und des Aktivator-Oligonukleotides bezüglich des komplementären Stranges nicht mit Sicherheit bestimmt werden kann. Die Menge des zugefügten Primers wird im Allgemeinen im molaren Überschuss bezüglich der Menge des komplementären Stranges (Matrize) vorhanden sein, wenn die zu amplifizierende Sequenz in einem Gemisch von komplizierten langkettigen Nukleinsäuresträngen enthalten ist. Ein großer molarer Überschuss wird bevorzugt, um die Effizienz des Verfahrens zu verbessern.

Die verwendeten Konzentrationen von Primer-1, Primer-2 und Aktivator-Oligonukleotide liegen beispielsweise in Bereichen zwischen 0,01 µmol/l und 100 µmol/l, bevorzugt zwischen 0,1 µmol/l und 100 µmol/l, bevorzugt zwischen 0,1 µmol/l und 50 µmol/l, besser zwischen 0,1 µmol/l und 20 µmol/l. Die hohe Konzentration von Komponenten kann die Geschwindigkeit der Amplifikation erhöhen. Die jeweiligen Konzentrationen einzelner Komponenten können unabhängig von einander variiert werden, um das gewünschte Reaktionsergebnis zu erzielen.

Die Konzentration von Polymerase liegt in Bereichen zwischen 0,001 µmol/l und 50 µmol/l, vorzugsweise zwischen 0,01 µmol/l und 20 µmol/l, besser zwischen 0,1µmol/l und 10 µmol/l.

Die Konzentration von einzelnen dNTP-Substraten liegt in Bereichen zwischen 10 µmol/l und 10 mmol/l, vorzugsweise zwischen 50 µmol/l und 2 mmol/l, besser zwischen 100 µmol/l und 1 mmol/l. Die Konzentration von dNTP kann die Konzentration von divalenten Metal-Kationen beeinflußen. Gegebenenfalls wird diese entsprechend angepasst.

Als divalente Metal-Kationen werden beispielsweise Mg2+ verwendet. Als entsprechendes Anion können beispielsweise Cl, Acetat, Sulfat, Glutamat etc. verwendet werden.

Die Konzentration von divalente Metal-Kationen wird beispielsweise an den für jeweilige Polymerase optimalen Bereich angepasst und umfasst Bereiche zwischen 0,1 mmol/l und 50 mmol/l, besser zwischen 0,5 mmol/l und 20 mmol/l, bevorzugter zwischen 1 mmol/l und 15 mmol/l.

Die enzymatische Synthese erfolgt im Allgemeinen in einer gepufferten wässrigen Lösung. Als Puffer-Lösungen können gelöste konventionelle Puffer-Substanzen, wie Tris-HCI, Tris-Acetat, Kalium-Glutamat, HEPES-Puffer, Natrium-Glutamat in gängigen Konzentrationen verwendet werden. Der pH-Wert dieser Lösungen liegt üblicherweise zwischen 7 und 9,5, bevorzugt etwa bei 8 bis 8,5. Die Puffer-Bedingungen können beispielsweise nach Empfehlung des Herstellers der verwendeten Polymerase angepasst werden.

Weitere Substanzen, wie sogenannte Tm-Depressoren (z.B. DMSO, Betaine, TPAC) etc. können zum Puffer zugegeben werden. Solche Substanzen verringern die Schmelztemperatur ("Tm-Depressoren") von Doppelsträngen und können somit einen positiven Einfluss auf die Öffnung von Doppelsträngen haben. Auch Polymerase-stabilisierende Komponenten, wie Tween 20 oder Triton 100 können in üblichen Mengen zum Puffer zugegeben werden. EDTA oder EGTA kann zu Komplexierung von Schwermetallen in konventionellen Mengen zugegeben werden. Polymerase-stabilisierende Substanzen, wie Trehalose oder PEG 6000 können ebenfalls zum Reaktionsgemisch zugegeben werden.

Vorzugsweise enthält die Reaktionsmischung keine Inhibitoren der Strangverdrängungsreaktion und keine Inhibitoren einer Polymerasenabhängigen Primer-Verlängerung.

In einer Ausführungsform enthält die Reaktionsmischung DNA-bindende Farbstoffe, bevorzugt interkalierenden Farbstoffe, wie z.B. EvaGreen oder SybrGreen. Solche Farbstoffe können ggf. bei Detektion der Neubildung von Nukleinsäureketten ermöglichen.

Die Reaktionsmischung kann weiterhin Proteine bzw. andere Substanzen enthalten, welche beispielsweise aus einem Original-Material stammen und welche die Amplifikation vorzugsweise nicht beeinflussen.

### Bevorzugte Ausführungsformen von Reaktionsbedingungen

Die Temperatur hat einen wesentlichen Einfluss auf die Stabilität der Doppelstränge.

In einer bevorzugten Ausführungsform werden während der Amplifikationsreaktion keine Temperaturbedingungen verwendet, welche im Wesentlichen zur Trennung von Doppelsträngen der zu amplifizierenden Nukleinsäure in Abwesenheit von Aktivator-Oligonukleotid führen. Damit soll sichergestellt werden, dass die Doppelstrang-Trennung von zu amplifizierenden Nukleinsäureketten von Anwesenheit des Aktivator-Oligonukleotides im gesamten Verlauf der Amplifikation abhängig ist.

Bei Temperatur etwa in Höhe von der gemessenen Schmelztemperatur (Tm) der zu amplifizierenden Nukleinsäure kommt es zu einer spontanen Trennung von beiden Strängen der zu amplifizierenden Nukleinsäure, so dass Einfluss des Aktivator-Oligonukleotids auf Trennung von synthetisierten Strängen und somit auf die Sequenzspezifität der Amplifikation minimal bis begrenzt ist.

Bei einer exponentiellen Amplifikation, welche weniger sequenzspezifisch (d.h. wenig Aktivator-Oligonukleotid-abhängig) ablaufen muss, kann die Reaktionstemperatur beispielsweise um die Schmelztemperatur (d.h. Tm plus / minus 3° bis 5°C) der zu amplifizierenden Nukleinsäure liegen. Bei einer solchen Temperatur können Sequenzunterschiede zwischen Aktivator-Oligonukleotid und dem synthetisierten Primer-Verlängerungsprodukt im Rahmen einer Strangverdrängungsreaktion im Allgemeinen gut toleriert werden.

Auch im Temperatur-Bereich von ca. (Tm minus 3° C) bis ca. (Tm minus 10°C) kann es immer noch zu einer spontanen Strangtrennung von synthetisierten Primer-Verlängerungsprodukten kommen, obwohl mit geringerer Effizienz. Der Einfluss von Aktivator-Oligonukleotid auf die Sequenzspezifität der zu amplifizierenden Nukleinsäure ist höher als bei Temperaturbedingungen um die Schmelztemperatur (Tm) der zu amplifizierenden Nukleinsäurekette.

Zwar findet die Strangtrennung mit sinkender Reaktionstemperatur im Wesentlichen dank Interaktion des neusynthetisierten Doppelstranges mit Aktivator-Oligonukleotid statt, Allerdings können Duplexe aus Primer bei Verlängerungstemperatur unter den genannten Bedingungen auch spontan zerfallen, d.h. ohne sequenzabhängige Strangverdrängung durch Aktivator-Oligonukleotid. Beispielsweise liegt die Reaktionstemperatur bei einer vermindert sequenzspezifischen Amplifikation in Bereichen zwischen ca. (Tm minus 3°C) und ca. (Tm minus 10°C), bevorzugt zwischen ca. (Tm minus 5°C) und ca. (Tm minus 10°C). Bei einer solchen Temperatur werden Sequenzunterschiede zwischen Aktivator-Oligonukleotid und dem synthetisierten Primer-Verlängerungsprodukt im Rahmen einer Strangverdrängungsreaktion schlechter toleriert.

Eine hohe Sequenzspezifität der Amplifikation des Verfahrens wird vor allem dann erreicht, wenn die neu synthetisierten Stränge der zu amplifizierenden Nukleinsäure unter Reaktionsbedingungen nicht spontan in Einzelstränge dissoziieren können. In einem solchen Fall spielt die sequenzspezifische Strangverdrängung durch Aktivator-Oligonukleoid für eine sequenzspezifische Strangtrennung eine entscheidende Rolle und ist für die Sequenzspezifität der Amplifikatonsreaktion maßgeblich verantwortlich. Dies kann im allgemeinen erreicht werden, wenn die Reaktionstemperatur deutlich unter der Schmelztemperatur von beiden Strängen der zu amplifizierenden Nukleinsäure liegt und keine weiteren Komponenten für eine Strangtrennung verwendet werden, beispielsweise keine Helicasen oder Recombinasen. Beispielsweise liegt die Reaktionstemperatur bei einer sequenzspezifischen Amplifikation in Bereichen zwischen ca. (Tm minus 10°C) und ca. (Tm minus 50°C), bevorzugt zwischen ca. (Tm minus 15°C) und ca. (Tm minus 40°C), besser zwischen ca. (Tm minus 15°C) und ca. (Tm minus 30°C).

In einer bevorzugten Ausführungsform der Amplifikation wird die maximale Reaktions-Temperatur im Verlauf der gesamten Amplifikationsreaktion nicht über die Schmelztemperatur der zu amplifizierenden Nukleinsäurekette erhöht.

In einer weiteren Ausführungsform der Amplifikation kann die Reaktionstemperatur über die Schmelztemperatur der zu amplifizierenden Nukleinsäureketten mindestens einmal erhöht werden. Die Erhöhung der Temperatur kann beispielsweise zu Beginn der Amplifikationsreaktion erfolgen und zu Denaturierung von Doppelsträngen einer genomischen DNA führen. Dabei muss beachet werden, dass während eines solchen Schrittes die Abhängigkeit der Doppelstrang-Trennung von der Wirkung des Aktivator-Oligonukleotides aufgehoben ist oder zumindest signifikant reduziert ist.

Die Reaktionstemperaturen der einzelnen Schritte der Amplifikationsreaktion können im Bereich von ca. 15°C bis ca. 85°C, besser im Bereich von ca. 15°C bis ca. 75°C, bevorzugt im Bereich von ca. 25°C bis ca. 70°C liegen.

In den nachfolgend beschriebenen Beispielen 2 und 3 wurde eine Reaktionstemperatur der Amplifikationsreaktion von 65°C verwendet, wobei die Tm der zu amplifizierenden Nukleinsäuren zwischen ca. 75°C und ca. 80°C lag. Somit war der Doppelstrang der zu amplifizierenden Nukleinsäure unter Reaktionsbedingungen stabil und die Amplifikationsreaktion sequenzspezifisch (siehe Beispiel 3).

Im Allgemeinen kann für die Reaktionstemperatur für jeden einzelnen Reaktionsschritt optimal eingestellt werden, so dass für jeden Reaktionsschritt eine solche Temperatur herbeigeführt wird. Die Amplifikationsreaktion umfasst somit eine sich wiederholende Änderung von Temperaturen, welche zyklisch wiederholt werden. In einer vorteilhaften Ausführungsform des Verfahrens werden Reaktionsbedingungen für mehrere Reaktionsschritte vereinheitlicht, so dass die Anzahl von Temperatur-Schritten geringer ist als die Anzahl von Reaktionsschritten. In einer solchen bevorzugten Ausführungsform der Erfindung erfolgt mindestens einer der Schritte der Amplifikation bei einer Reaktionstemperatur, welche sich von der Reaktionstemperatur anderer Schritte der Amplifikation unterscheidet. Die Reaktion verläuft somit nicht isothermal, sondern die Reaktionstemperatur wird zyklisch geändert.

Es werden beispielsweise während der Amplifikation mindestens zwei Temperatur-Bereiche verwendet, welche abwechselnd herbeigeführt werden (zyklische Änderung von Temperaturen zwischen einzelnen Temperatur-Bereichen). In einer Ausführungsform umfasst der untere Temperatur-Bereich beispielsweise Temperaturen zwischen 25°C und 60°C, besser zwischen 35°C und 60°C, bevorzugt zwischen 50°C und 60°C und der obere Temperatur-Bereich umfasst beispielsweise Temperaturen zwischen 60°C und 75°C, besser zwischen 60°C und 70°C.

In einer weiteren Ausführungsform umfasst der untere Temperatur-Bereich beispielsweise Temperaturen zwischen 15°C und 50°C, besser zwischen 25°C und 50°C, bevorzugt zwischen 30°C und 50°C und der obere Temperatur-Bereich umfasst beispielsweise Temperaturen zwischen 50°C und 75°C, besser zwischen 50°C und 65°C.

In einer weiteren Ausführungsform umfasst der untere Temperatur-Bereich beispielsweise Temperaturen zwischen 15°C und 40°C, besser zwischen 25°C und 40°C, bevorzugt zwischen 30°C und 40°C und der obere Temperatur-Bereich umfasst beispielsweise Temperaturen zwischen 40°C und 75°C, besser zwischen 40°C und 65°C.

Die Temperatur kann im jeweiligen Bereich konstant gehalten werden oder als TemperaturGradient (abfallend oder aufsteigend) geändert werden.

Weitere Erläuterungen zu Temperatur-Einstellungen werden in folgenden Abschnitten bei Ausführungsformen im Einzelnen angeführt.

Jede herbeigeführte Temperatur kann eine gewisse Zeit aufrechterhalten werden, so dass dabei ein Inkubations-Schritt resultiert. Das Reaktionsgemisch kann somit während einer Amplifikation bei einer ausgewählten Temperatur eine gewisse Zeit inkubiert werden. Diese Zeit kann unterschiedlich lange für jeweiligen Inkubations-Schritt sein und kann sich an den Fortschritt der jeweiligen Reaktion bei gegebener Temperatur richten (z.B. Primer-Verlängerung oder Strangverdrängung usw.). Die Zeit eines Inkubationsschrittes kann folgende Bereiche umfassen: zwischen 0,1 sec und 10.000 sec, besser zwischen 0,1 sec und 1000 sec, bevorzugt zwischen 1 sec und 300 sec, noch bevorzugter zwischen 1 sec und 100 sec.

Durch eine solche Temperatur-Änderung können einzelne Reaktionsschritte bevorzugt bei einer ausgewählten Temperatur ausgeführt werden. Dadurch können Ausbeuten von einem jeweiligen Reaktionsschritt verbessert werden. Innerhalb eines Synthese-Zyklus kann Temperatur-Änderung bzw. Temperatur-Wechsel zwischen einzelnen Temperatur-Bereichen ggf. mehrmals herbeigeführt werden. Synthese-Zyklus kann somit mindestens ein Temperatur-Wechsel umfassen. Ein solcher Temperatur-Wechsel kann beispielsweise in einem PCR-Gerät/Thermocycler routinemäßig als zeitliches Programm ausgeführt werden.

In einer Ausführungsform wird ein Amplifikationsverfahren bevorzugt, bei welchem mindestens eines der Schritte, welche Strangverdrängung umfassen, und mindestens eines der Schritte, welche Primer-Verlängerungsreaktionen umfassen, gleichzeitig bzw. parallel stattfinden und unter gleichen Reaktionsbedingungen erfolgen. In einer solchen Ausführungsform kann beispielsweise eine Primer-Verlängerungsreaktion von mindestens einem Primer-Oligonukleotid (z.B. vom ersten Primer-Oligonukleotid) bevorzugt bei Temperaturbedingungen im unteren Temperatur-Bereich erfolgen. Hingegen erfolgt die Strangverdrängung unter Mitwirkung von Aktivator-Oligonukletid und die eine weitere Primer-Verlängerungsreaktion (z.B. vom zweiten Primer-Oligonukleotid) bevorzugt im Reaktionsschritt im oberen Temperatur-Bereich.

In einer weiteren Ausführungsform wird ein Amplifikationsverfahren bevorzugt, bei welchem mindestes eines der Schritte, welche Strangverdrängung durch das Aktivator-Oligonukleotid umfassen und mindestens eines der Schritte, welche Primer-Verlängerungsreaktion umfassen, bei unterschiedlichen Temperaturen durchgeführt. In einer solchen Ausführungsform können beispielsweise Primer-Verlängerungsreaktionen von mindestens einem Primer-Oligonukleotid (z.B. vom ersten Primer-Oligonukleotid und / oder von zweiten Primer-Oligonukleotid) bevorzugt bei Temperaturbedingungen im unteren Temperatur-Bereich erfolgen. Hingegen erfolgt die Strangverdrängung unter Mitwirkung von Aktivator-Oligonukletid bevorzugt im Reaktionsschritt im oberen Temperatur-Bereich.

In einer weiteren bevorzugten Ausführungsform verlaufen sämltliche Schritte einer Amplifikatons-Reaktion unter gleichen Reaktionsbedingungen.

In einer solchen Ausführungsform kann das Amplifikationsverfahren unter isothermalen Bedingungen durchgeführt werden, d.h. dass keine Temperaturänderungen für die Ausführung des Verfahrens erforderlich sind. In einer solchen bevorzugten Ausführungsform der Erfindung erfolgt die gesamte Amplifikationsreaktion unter konstanter Temperatur, d.h. die Reaktion ist isothermal. Die Zeit einer solchen Reaktion umfasst beispielsweise folgende Bereiche: zwischen 100 sec und 30.000 sec, besser zwischen 100 sec und 10.000 sec, noch besser zwischen 100 sec und 1000 sec.

Im Abschnitt "Beispiele" wird gezeigt, dass es möglich ist, Strukturen einzelner Reaktionskomponenten und entsprechende Reaktionsschritte dermassen aneinander abzustimmen, dass eine isothermale Reaktion möglich ist.

Die Summe aller Verfahrensschritte, welche zu einer Verdoppelung der Menge einer zu amplifizierenden Nukleinsäurekette führt, kann als Synthese-Zyklus bezeichnet werden. Ein solcher Zyklus kann entsprechend isothermal ablaufen oder auch durch Änderungen der Temperatur in seinem Verlauf gekennzeichnet sein. Die Temperatur-Änderungen können sich von Zyklus zu Zyklus wiederholen und identisch gestaltet werden.

Besonders vorteilhaft sind Amplifikationsverfahren, bei welchen die maximal erreichbare Temperatur eine Strang-Trennung unter Mitwirkung von Aktivator-Oligonukleotid nur dann im Wesentlichen zulässt, wenn mehr als 5 Nukleotide des dritten Bereichs des Aktivator-Oligonukleotides eine komplementäre Bindung mit dem ersten Primer-Verlängerungsprodukt eingehen können, vorteilhafter ist es, wenn mehr als 10, noch vorteilhafter, wenn mehr als 20 Nukleotide des Aktivator-Oligonukleotides mit dem ersten Primer-Verlängerungsprodukt eine Bindung eingehen. Im Allgemeinen gilt, je länger die erforderliche Bindung zwischen Aktivator-Oligonukleotid und dem komplementären Strang des ersten Primer-Verlängerungsproduktes, bevor die synthetisierten Stränge unter Reaktionsbedingungen dissoziieren, desto spezifischer ist die Amplifikations-Reaktion. Im Einzelnen, kann durch Verlängerung bzw. Verkürzung des dritten Abschnittes des Aktivator-Oligonukleotides das gewünschte Maß an Spezifität ermittelt werden.

Ein Verfahrensschritt kann bei seiner Wiederholung bei konstanter Temperatur erfolgen über die Gesamt-Dauer des Verfahrens oder auch bei unterschiedlichen Temperaturen. Einzelne Verfahrensschritte können jeweils hintereinander durch Zugabe von einzelnen Komponenten ausgeführt werden. Bei einer vorteilhaften Ausführungsform liegen sämtliche für die Ausführung einer Amplifikation notwendige Reaktions-Komponenten zu Beginn einer Amplifikation in einem Reaktionsgemisch vor.

Der Start einer Amplifikations-Reaktion kann durch Zugabe einer Komponente erfolgen, z.B. durch Zugabe einer Nukleinsäurekette umfassend eine Zielsequenz (z.B. eine Start-Nukleinsäurekette), oder einer Polymerase oder divalenten Metal-Ionen, oder auch durch Herbeiführung von Reaktions-Bedingungen, welche für Amplifikation notwendig sind, z.B. Einstellung einer erforderlichen Reaktionstemperatur für einen oder mehrere Verfahrenschritte.

Die Amplifikation kann so lange ausgeführt werden, bis die gewünschte Menge an zu amplifizierenden Nukleinsäure erreicht ist. In einer anderen Ausführungsform wird die Amplifikationsreaktion über eine Zeit ausgeführt, welche bei Vorliegen einer zu amplifizierenden Nukleinsäure ausgereicht hätte, um eine ausreichende Menge zu bekommen. In einer anderen Ausführungsform wird die Amplifikationsreaktion über eine hinreichede Anzahl von Synthese-Zyklen (Verdoppelungszeiten) ausgeführt, welche bei Vorliegen einer zu amplifizierenden Nukleinsäure ausgereicht hätte, um eine ausreichende Menge zu bekommen.

Die Reaktion kann durch verschiedene Eingriffe gestoppt werden. Beispielsweise durch Änderung der Temperatur (z.B. Abkühlen oder Erhitzen, wobei beispielsweise Polymerase in ihrer Funktion gestört wird) oder durch Zugabe einer Substanz, welche eine Polymerase-Reaktion stoppt, z.B. EDTA oder Formamid.

Im Anschluss an die Amplifikation kann die amplifizierte Nukleinsäurekette für weitere Analysen verwendet werden. Dabei können synthetisierte Nukleinsäureketten durch verschiedene Detektionsverfahren analysiert werden. Beispielsweise können fluoreszenzmarkierte Oligonukleotid-Sonden verwendet werden oder Sequenzierungsverfahren (Sanger-Sequenzierung oder Next-Generation Sequenzierung), fest-Phasen-Analysen wie Microarray oder Bead-Array-Analysen usw. Die synthetisierte Nukleinsäurekette kann als Substrat / Matrize in weiteren Primer-Verlängerungsreaktionen verwendet werden.

In einer vorteilhaften Ausführungsform wird der Fortschritt der Synthese-Reaktion während der Reaktion überwacht. Das kann beispielsweise durch Einsatz von interkalierenden Farbstoffen erfolgen, z.B. Sybrgreen oder Evagreen, oder unter Einsatz von markierten Primern (z.B. Lux-Primer oder Scorpion-Primer) oder unter Einsatz von fluoreszenzmarkierten Oligonukleotid-Sonden.

Die Detektion der Veränderung der Fluoreszenz während der Amplifikation wird in einem Detektions-Schritt des Verfahrens umgesetzt. Dabei kann die Temperatur und die Dauer dieses Schrittes an die jeweiligen Erfordernisse der Oligonukleotid-Sonde angepasst werden. Die Temperaturen des Detektioins-Schrittes umfassen beispielsweise Bereiche zwischen 20°C und 75°C, besser zwischen 40 und 70°C, bevorzugt zwischen 55 und 70°C.

Während des Detektionschrittes erfolgt Beleuchtung der Reaktion mit Licht einer Wellenlänge, welche in der Lage in ein verwendetes Fluorophor des Detektions-Systems (ein Donor oder ein Fluoreszenzreporter) anzuregen. Die Signal-Erfassung erfolgt in der Regel parallel zu Anregung, wobei das spezifische Fluoreszenzsignal detektiert wird und seine Intensität quantifiziert wird.

Das Amplifikationsverfahren kann zur Überprüfung der Anwesenheit einer Zielnukleinsäurekette in einem biologischen Material oder einem diagnostischen Material im Rahmen eines diagnostischen Verfahrens eingesetzt werden.

### Bevorzugte Ausführungsformen einer Start-Nukleinsäurekette:

Die zu Beginn der Amplifikations-Reaktion eingesetzte bzw. einzusetzende Nukleinsäurekette kann als Start-Nukleinsäurekette bezeichnet werden (Fig. 1).

Ihre Funktion kann dahingehend gesehen werden, dass sie die anfängliche Matrize darstellt, welche eine korrekte Positionierung von Primern, der Synthese-Abschnitte zwischen beiden Primern, sowie die Initiierung von Bindungs- und Verlängerungsvorgängen ermöglicht. In einer bevorzugten Ausführungsform umfasst eine Start-Nukleinsäurekette eine Zielsequenz.

Durch Bindung von Primern an ihre entsprechenden Primer-Bindungsstellen (PBS 1 und PBS 2) und Initiierung von entsprechenden Primer-Verlängerungsreaktionen erfolgt durch Generierung von ersten Primer-Verlängerungsprodukten. Diese werden als spezifische Kopien der zu Begin der Reaktion vorliegenden Nukleinsäurekette synthetisiert.

In einer Ausführungsform kann diese in das Reaktionsgemisch vor Beginn der Amplifikatons-Reaktion einzusetzende Nukleinsäurekette (Start-Nukleinsäurekette) mit der zu amplifizierenden Nukleinsäurekette identisch sein. Durch die Amplifikations-Reaktion wird lediglich die Menge einer solchen Nukleinsäurekette vermehrt.

In einer weiteren Ausführungsform unterscheiden sich die zu amplifizierende Nukleinsäure und die Start-Nukleinsäurekette dahingehend, dass die Start-Nukleinsäurekette zwar die Anordnung einzelner Sequenzelemente der zu amplifizierenden Nukleinsäurekette vorgibt, doch kann die Sequenzzusammensetzung der Start-Nukleinsäurekette von der Sequenz der zu amplifizierenden Nukleinsäurekette abweichen. Beispielsweise können im Rahmen der Primer-Bindung und Verlängerung während einer Amplifikation neue Sequenzinhalte (bezogen auf Start-Nukleinsäurekette) in die zu amplifizierende Nukleinsäurekette integriert werden. Weiterhin können sich Sequenzelemente einer zu amplifizierenden Nukleinsäurekette von solchen Sequenzelementen einer Start-Nukleinsäurekette in ihrer Sequenzzusammensetzung unterscheiden (z.B. Primer-Bindungsstellen oder Primer-Sequenzen). Die Start-Nukleinsäure dient nur als initiale Matrize für die spezifische Synthese der zu amplifizierenen Nukleinsäurekette. Diese initiale Matrize kann im Reaktionsgemisch bis zum Ende der Amplifikation verbleiben. Durch den exponentiellen Charakter der Amplifikation überwiegt allerdings die Menge der zu amplifizierenden Nukleinsäurekette am Ende einer Amplifikationsreaktion die Menge einer in die Reaktion zugegebenen Start-Nukleinsäurekette.

### Bevorzugte Ausführungsformen des ersten Primer-Oligonukleotids (Primer-1)

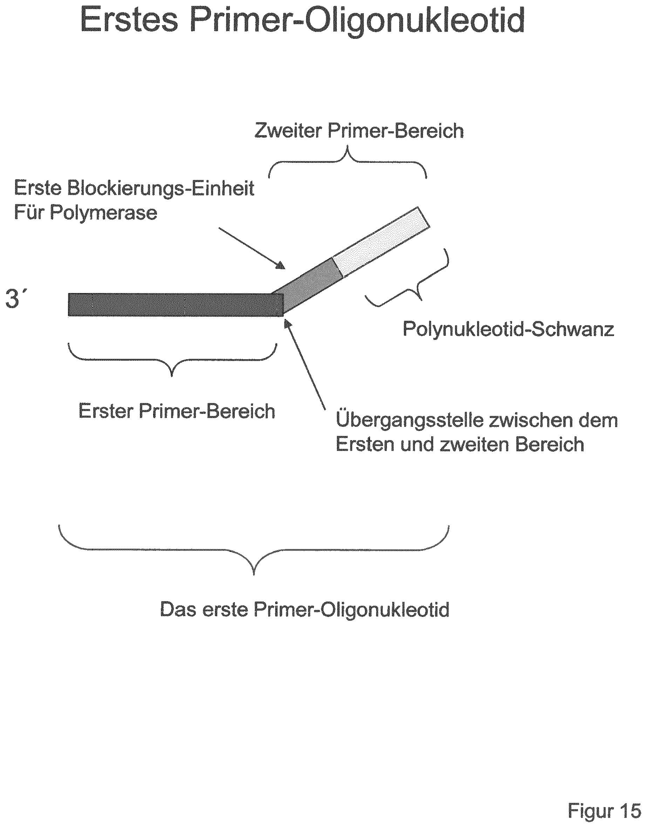

**Das erste Primer-Oligonukleotid** (Primer-1) ist eine Nukleinsäurekette welche mindestens folgende Bereiche einschließt (Fig. 14 und 15):
- Einen ersten Primer-Bereich im 3'-Segment des ersten Primer-Oligonukleotides, der an einen Strang einer zu amplifizierenden Nukleinsäurekette im Wesentlichen sequenzspezifisch binden kann
- Einen zweiten Bereich, gekoppelt direkt oder via einen Linker an das 5'-Ende des ersten Primer-Bereichs des ersten Primer-Oligonukleotides, welcher einen Polynukleotid-Schwanz umfasst, welcher zur Bindung eines Aktivator-Oligonukleotids und Unterstützung der Strangverdrängung (Schritt c) durch Aktivator-Oligonukleotids geeignet ist, wobei der Polynukleotid-Schwanz unter Reaktionsbedingungen im wesentlichen einzelsträngig bleibt, d.h. keine stabile Hairpin-Struktur oder ds-Strukturen ausbildet, und vorzugsweise nicht von Polymerase kopiert wird.

Die Gesamtlänge des ersten Primer-Oligonukleotides liegt zwischen 10 und 80, vorzugsweise zwischen 15 und 50, besser zwischen 20 und 30 Nukleotiden oder ihren Äquivalenten (z.B. Nukleotid-Modifikationen). Die Struktur des ersten Primer-Oligonukleotids ist dermaßen angepasst, dass es unter gewählten Reaktionsbedingungen eine reversible Bindung an Aktivator-Oligonukleotid eingehen kann. Weiterhin ist die Struktur des ersten Primer-Oligonukletides an seine Primer-Funktion angepasst. Weiterhin ist die Struktur so angepasst, dass eine Strangverdrängung mittels Aktivator-Oligonukleotid ausgeführt werden kann. Insgesamt sind Strukturen des ersten und des zweiten Bereichs aneinander angepasst, so dass eine exponentielle Amplifikation ausgeführt werden kann.

In einer vorteilhaften Ausführungsform der Erfindung sind der erste und der zweite Bereich des Primers in einer konventionellen 5'-3' Anordnung gekoppelt. In einer weiteren Ausführungsform der Erfindung erfolgt die Kopplung von beiden Abschnitten über eine 5'-5'-Bindung, so dass der zweite Bereich eine umgekehrte Richtung hat als der erste Bereich.

Die Kopplung von Bereichen zwischeneinander/untereinander erfolgt vorzugsweise kovalent. In einer Ausführungsform ist die Kopplung zwischen dem ersten und zweiten Bereich eine für DNA konventionelle 5'-3'-Phosphodiester-Kopplung. In einer weiteren Ausführungsform ist es eine 5'-5'-Phosphodiester-Kopplung. In einer weiteren Ausführungsform ist es eine 5'-3'-Phosphodiester-Kopplung, wobei zwischen benachbarten endständigen Nukleotiden bzw. Nukleotid-Modifikationen der beiden Bereiche mindestens ein Linker (z.B. ein C3, C6, C12 oder ein HEG-Linker oder eine abasische Modifikation) positioniert ist.

Einzelne Bereiche können unterschiedliche Nukleotid-Modifikationen einschließen. Dabei können individuelle Elemente von Nukleotiden modifiziert sein: Nukleobase und Rückgrad (Zucker-Anteil und / oder Phosphat-Anteil). Weiterhin können Modifikationen verwendet werden, welchen mindestens eine Komponente der Standard-Nukleotid-Bausteine fehlt oder modifiziert ist, z.B. PNA.

In einer weiteren Ausführungsform umfasst ein zweiter Bereich des ersten Primer-Oligonukleotides weitere Sequenzen, die nicht an das Aktivator-Oligonukleotid binden. Diese Sequenzen können zu anderen Zwecken verwendet werden, z.B. zu Bindung an die feste Phase. Diese Sequenzen sind vorzugsweise am 5'-Ende des Polynukleotid-Schwanzes lokalisiert.

In einer weiteren Ausführungsform kann ein erstes Primer-Oligonukleotid charakteristische Markierung umfassen. Beispiele für eine solche Markierung stellen Farbstoffe dar (z.B. FAM, TAMRA, Cy3, Alexa 488 etc.) oder Biotin bzw. andere Gruppen, die spezifisch gebunden werden können, z.B. Digoxigenin.

### Der erste Primer-Bereich des ersten Primer-Oligonukleotides

Die Sequenz-Länge liegt zwischen ca. 3-30 Nukleotiden, vorzugsweise zwischen 5 und 20 Nukleotiden, wobei die Sequenz vorwiegend komplementär zum 3'-Segment eines Stranges der zu amplifizierenden Nukleinsäurekette ist. Im Einzelnen, muss dieser Primer-Bereich in der Lage sein, an das komplementäre 3'-Segment eines zweiten Primer-Verlängerungsproduktes spezifisch zu binden. Dieser erste Bereich soll bei Rücksynthese kopiert werden können und dient auch als Matrize für 2. Strang. Die Nukleotid-Bausteine sind vorzugsweise untereinander via übliche 5'-3' Phosphodieser-Bindung oder Phosphothioester-Bindung verknüpft.

Der erste Primer-Bereich schließt bevorzugt Nukleotid-Monomere ein, welche Funktion der Polymerase nicht oder nur unwesentlich beeinflussen, dazu gehöhren beispielsweise:
- natürliche Nukleotide (dA, dT, dC, dG etc.) oder deren Modifikationen ohne veränderte Basen-Paarung
- modifizierte Nukleotide, 2-Amino-dA, 2-thio-dT oder andere Nukleotid-Modifikationen mit abweichender Basen-Paarung.

In einer bevorzugten Ausführungsform ist das 3'-OH-Ende dieses Bereichs vorzugsweise frei von Modifikationen und hat eine funktionsfähige 3'-OH Gruppe, welche von Polymerase erkannt werden kann. Der erste Primer-Bereich dient als Initiator der Synthese des ersten Primer-Verlängerungsproduktes in der Amplifikation. In einer weiteren bevorzugten Ausführungsform umfasst der erste Bereich mindestens eine Phosphorothioat-Verbindung, so dass kein Abbau von 3'-Ende des Primern durch 3'-Exonuklease Aktivität von Polymerasen erfolgen kann.

Die Sequenz des ersten Bereichs des ersten Primer-Oligonukleotids und die Sequenz des zweiten Bereichs des Aktivator-Oligonukleotides sind vorzugsweise einander komplementär.

In einer Ausführungsform kann der erste Primer-Bereich oder sein 3'-Segment an Sequenzsegmente einer Zielsequenz binden.

### Der zweite Bereich des ersten Primer-Oligonukleotides

Der zweite Bereich des ersten Primer-Oligonukleotides ist vorzugsweise eine Nukleinsäuresequenz, welche mindestens einen Polynukleotid-Schwanz umfasst, welcher vorzugsweise während der Synthesereaktion von Polymerase unkopiert bleibt und welcher zur Bindung mit dem ersten Bereich des Aktivator-Oligonukleotides fähig ist. Das Segment des zweiten Bereichs, welches überwiegend diese Bindung mit dem Aktivator-Oligonukleotid eingeht, kann als Polynukleotid-Schwanz bezeichnet werden.

Weiterhin muss der zweite Bereich des ersten Primer-Oligonukleotids nicht nur das Aktivator-Oligonukleotides unter Reaktionsbedingungen spezifisch binden, sondern auch beim Vorgang der Strangverdrängung mittels Aktivator-Oligonukleotid mitwirken. Die Struktur des zweiten Bereichs muß folglich für die Herbeiführung einer räumlichen Nähe zwischen dem Aktivator-Oligonukleotid und dem entsprechenden Doppelstrang-Ende (im Einzelnen, dem 3'-Ende des zweiten Primer-Verlängerungsproduktes) geeignet sein.

Die Gestaltung der Struktur des zweiten Bereichs des ersten Primer-Oligonukleotids wird in mehreren Ausführungsformen genauer dargestellt. Dabei werden Anordnung der Oligonukleotid-Segmente und verwendete Modifikationen berücksichtigt, welche zu einem Stopp in der Polymerase-katalysierten Synthese führen.

Die Länge des zweiten Bereichs liegt zwischen 3 und 60, vorzugsweise zwischen 5 und 40, bevorzugt zwischen 6 und 15 Nukleotiden oder ihren Äquivalenten.

Die Sequenz des zweiten Bereichs kann willkürlich gewählt sein. Vorzugsweise ist sie nicht komplementär mit der zu amplifizierenden Nukleinsäure und/oder mit dem zweiten Primer-Oligonukleotid und/oder mit dem ersten Bereich des ersten Primer-Oligonukleotid. Weiterhin enthält sie vorzugsweise keine selbstkomplementären Segmente, wie Hairpins oder Stemmloops.

Die Sequenz des zweiten Bereichs ist vorzugsweise auf die Sequenz des ersten Bereichs des Aktivator-Oligonukleotids abgestimmt, so dass beide Sequenzen unter Reaktionsbedingungen eine Bindung eingehen können. In einer bevorzugten Ausführungsform ist diese Bindung unter Reaktionsbedingungen reversibel: es besteht somit ein Gleichgewicht zwischen aneinander gebundenen Komponenten und nichtgebundenen Komponenten.

Die Sequenz des zweiten Bereichs des ersten Primer-Oligonukleotides ist vorzugsweise dermaßen gewählt, dass die Anzahl von komplementären Basen, welche mit dem ersten Bereich des Aktivator-Oligonukleotid binden können, zwischen 1 und 40, besser zwischen 3 und 20, bevorzugt zwischen 6 und 15 liegt.

Die Funktion des zweiten Bereichs besteht unter anderem in Bindung des Aktivator-Oligonukleotides. In einer Ausführungsform ist diese Bindung vorzugsweise spezifisch, so dass ein zweiter Bereich eines ersten Primer-Oligonukleotides ein spezifisches Aktivator-Oligonukleotid binden kann. In einer anderen Ausführungsform kann ein zweiter Bereich mehr als nur ein Aktivator-Oligonukleotid unter Reaktionsbedingungen binden.

Es besteht im Allgemeinen keine Notwendigkeit in einer perfekten Übereinstimmung in der Sequenz zwischen dem zweiten Bereich des ersten Primer-Oligonukleotides und dem ersten Bereich des Aktivator-Oligonukleotides. Das Maß der Komplementarität zwischen dem zweiten Bereich des ersten Primer-Oligonukleotides und dem ersten Bereich des Aktivator-Oligonukleotides kann zwischen 20 % und 100 %, besser zwischen 50 % und 100 %, bevorzugt zwischen 80 % und 100 % liegen. Die jeweils komplementären Bereiche können unmittelbar aneinander anschließend positioniert sein oder auch nichtkomplementäre Sequenz-Segmente dazwischen umfassen.

Der zweite Bereich des ersten Primer-Oligonukleotides kann in einer Ausführungsform mindestens eine Tm-modifizierende Modifikation einschließen. Durch Einführung solcher Modifikationen kann die Stabilität der Bindung zwischen dem zweiten Bereich des ersten Primer-Oligonukleotides und dem ersten Bereich des Aktivator-Oligonukleotides modifiziert werden. Beispielsweise können Tm-erhöhende Modifikationen (Nukleotid-Modifikationen oder nicht-Nukleotid-Modifikationen) verwendet werden, wie LNA-Nukleotide, 2-Amino-Adenosine oder MGB-Modifikationen. Andererseits können auch Tm-Senkende Modifikationen verwendet werden, wie beispielsweise Inosine-Nukleotid. In der Struktur des zweiten Bereichs können auch Linker (z.B. C3, C6, HEG-Linker) intergriert werden.

Für die Strangverdrängung muss das Aktivator-Oligonukleotid in räumliche Nähe des Doppelstrang-Endes der zu amplifizierenden Nukleinsäure gebracht werden. Dieses Doppelstrang-Ende besteht aus Segmenten des ersten Primer-Bereichs des ersten Primer-Verlängerungsproduktes und einem entsprechend komplementären dazu 3'-Segment des zweiten Primer-Verlängerungsproduktes.

Der Polynukleotid-Schwanz bindet vorwiegend komplementär das Aktivator-Oligonukleotid unter Reaktionsbedingungen und bewirkt damit eine vorübergehende Annäherung des zweiten Bereichs des Aktivator-Oligonukleotides und des ersten Bereichs eines verlängerten Primer-Verlängerungsproduktes, so dass eine komplementäre Bindung zwischen diesen Elementen im Rahmen eines Strangverdrängungsvorgangs initiiert werden kann.

In einer Ausführungsform führt die Bindung des Aktivator-Oligonukleotides an den Polynukleotid-Schwanz des ersten Primer-Oligonukleotides unmittelbar zu einem solchen Kontakt. Das bedeutet, dass der Polynukleotid-Schwanz und der erste Primer-Bereich des ersten Primer-Oligonukleotides unmittelbar aneinander gekoppelt sein müssen. Dank einer solchen Anordnung kann nach einer Bindung eines Aktivator-Oligonukleotids im seinem ersten Bereich unmittelbar zu einem Kontakt zwischen komplementären Basen des zweiten Bereichs des Aktivator-Oligonukleotides und zu entsprechenden Basen des ersten Primer-Bereichs kommen, so dass eine Strangverdrängung initiiert werden kann.

In einer weiteren Ausführungsform liegen zwischen Strukturen des Polynukleotid-Schwanzes und dem ersten Primer-Bereich andere Strukturen des zweiten Bereichs des ersten Primer-Oligonukleotides. Nach einer Bindung eines Aktivator-Oligonukleotids an den Polynukleotid-Schwanz ist dieser somit nicht unmittelbar an den ersten Primer-Bereich positioniert, sondern in einer gewissen Distanz dazu. Die Strukturen zwischen dem unkopierbarem Polynukleotid-Schwanz und dem kopierbaren ersten Primer-Bereich des Primer-Oligonukleotides können einen solchen Abstand generieren. Dieser Abstand hat einen Wert, welcher zwischen 0,1 und 20 nm, vorzugsweise zwischen 0,1 und 5 nm, besser zwischen 0,1 und 1 nm liegt.

Solche Strukturen stellen beispielsweise Linker (z.B. C3 oder C6 oder HEG-Linker) oder nicht zu Aktivator-Oligonukleotid komplementäre Segmente dar (z.B. in Form von nichtkomplementären, nicht-kopierbaren Nukleotid-Modifikationen). Die Länge dieser Strukturen kann im Allgemeinen in Ketten-Atomen ausgemessen werden. Diese Länge beträgt zwischen 1 und 200 Atomen, vorzugsweise zwischen 1 und 50 Kettenatomen, bevorzugt zwischen 1 und 10 Kettenatomen.

Damit der Polynukleotid-Schwanz von Polymerase unter Amplifikationsbedingungen unkopierbar bleibt, umfasst der zweite Bereich des ersten Primer-Oligonukleotides im allgemeinen Sequenz-Anordnungen bzw. Strukturen, welche zu einem Stopp der Polymerase in der Synthese des zweiten Primer-Verlängerungsproduktes bewirken, nachdem die Polymerase den ersten Primer-Bereich erfolgreich kopiert hat. Diese Strukturen sollen das Kopieren des Polynukleotid-Schwanzes des zweiten Bereichs verhindern. Der Polynukleotid-Schwanz bleibt somit vorzugsweise von der Polymerase unkopiert.

In einer Ausführungsform liegen solche Strukturen zwischen dem ersten Primer-Bereich und dem Polynukleotid-Schwanz.

In einer weiteren Ausführungsform kann die Sequenz des Polynukleotid-Schwanzes Nukleotid-Modifikationen einschließen, welche zum Stopp der Polymerase führen. Dadurch kann ein Sequenzsegment des zweiten Bereichs des ersten Primer-Oligonukleotids beide Funktionen umfassen: es ist sowohl ein Polynukleotid-Schwanz als auch eine zum Stopp der Polymerase führende Sequenz aus Nukleotid-Modifikationen.

Modifikationen im zweiten Bereich des ersten Primer-Oligonukleotid, welche zu einem Synthese-Stopp führen und somit den Polynukleotid-Schwanz unkopierbar lassen, werden in dieser Anmeldung unter dem Begriff "erste Blockierungs-Einheit oder einen Stopp-Bereich" zusammengefasst.

Zusammenfassend kann die Termination der Synthese von Polymerase im zweiten Bereich auf verschiedene Art und Weise erreicht werden. Diese Blockade erfolgt bevorzugt allerdings erst, wenn Polymerase den ersten Bereich des ersten Primer-Oligonukleotides kopiert hat. Damit wird sichergestellt, dass ein zweites Primer-Verlängerungsprodukt eine passende Primer-Bindungsstelle in seinem 3'-Segment besitzt. Diese Primer-Bindungsstelle wird im Rahmen der Strangverdrängung freigelegt und steht somit für eine erneute Bindung eines weiteren ersten Primer-Oligonukleotids zur Verfügung.

Bei der Synthese des komplementären Stranges zum ersten Primer-Verlängerungsprodukt bleibt die Primer-Verlängerungsreaktion vor dem Polynukleotid-Schwanz stehen. Da dieser Polynukleotid-Schwanz für die Interaktion mit dem Aktivator-Oligonukleotid dadurch einzelsträngig bleibt und somit für Bindung zur Verfügung steht, unterstützt dieser die Initiierung der Strangverdrängungs-Reaktion durch das Aktivator-Oligonukleotid, indem er den entsprechenden komplementäre Segmente des Aktivator-Oligonukleotids in unmittelbare Nähe des passenden Duplex-Endes bringt. Der Abstand zwischen dem komplementären Teil des Aktivator-Oligonukleotides (zweiter Bereich) und dem komplementären Teil des verlängerten Primer-Oligonukleotid (erster Bereich) wird dadurch auf ein Minimum reduziert. Solche räumliche Nähe erleichtert die Initiierung der Strangverdrängung.

Im Rahmen einer schematischen Darstellung einer nukleinsäurevermittelten Strangverdrängungs-Reaktion liegt nun eine komplementäre Sequenz von Aktivator-Oligonukleotid unmittelbar in der Nähe des passenden Duplex-Endes. Dabei kommt es zu Konkurrenz um die Bindung an den ersten Bereich des ersten Primer-Oligonukleotids zwischen dem Strang des Aktivator-Oligonukleotides und dem zum Primer komplementären Matrizenstrang. Durch repetetive Schließung und Formierung von Basenpaarung zwischen dem Primer-Bereich und dem komplementären Segment des Aktivator-Oligonukleotids (zweiter Bereich des Aktivator-Nukleotides) bzw. dem komplementären Segment des Matrizenstranges kommt es zur Initiierung des nukleinsäurevermittelten Strangverdrängungs-Vorgangs.

Im Allgemeinen ist die Ausbeute der Initiierung der Stang-Verdrängung umso höher, je näher der entsprechende komplementäre Sequenzabschnitt des Aktivator-Oligonukleotides zum komplementären Segment des Primer-Bereichs liegt. Bei Vergrößerung dieses Abstandes sinkt dagegen die Ausbeute der Initiierung der Strangverdrängung.

Im Rahmen der vorliegenden Erfindung ist es nicht zwingend notwendig, dass die Initiierung der Strangverdrängung mit maximaler Ausbeute funktioniert. Daher können Abstände zwischen dem 5'-Segment des ersten Primer-Bereichs des ersten Primer-Oligonukleotid, welcher eine Bindung mit einem komplementären Strang der Matrize eingeht und eine komplementäre Duplex bildet, und einem entsprechend komplementären Sequenzabschnitt im Aktivator-Oligonukleotid, wenn gebunden an Polynukleotid-Schwanz des zweiten Bereichs des ersten Primer-Oligonukleotides, in folgenden Bereichen liegen: zwischen 0,1 und 20 nm, besser zwischen 0,1 und 5 nm, noch besser zwischen 0,1 und 1 nm. Im bevorzugten Fall beträgt diese Distanz weniger als 1 nm. In anderen Einheiten ausgedrückt entspricht dieser Abstand einer Strecke von weniger als 200 Atomen, noch besser weniger als 50 Atomen, noch besser weniger als 10 Atomen. Im bevorzugten Fall ist diese Distanz ein Atom. Die Angaben zur Distanz sollen lediglich zur Orientierung dienen und veranschaulichen, dass kürzere Distanzen zwischen diesen Strukturen bevorzugt werden. Die Messung dieser Distanz ist in vielen Fällen nur durch Analyse der genauen Strukturen von Oligonukleotiden und Ausmessung der Messung von Sequenzabständen bzw. von Linker-Längen möglich.

Der erste Primer kann auch noch weitere Sequenzabschnitte umfassen, welche nicht zu Interaktion mit Aktivator-Oligonukleotid oder Matrizen-Strang benötigt werden. Solche Sequenzabschnitte können beispielsweise weitere Oligonukleotide binden, welche als Detektionssonden oder Immobilisierungspartner bei einer Bindung an die feste Phase verwendet werden.

### Primer-Funktion des ersten Primer-Oligonukleotides

Das erste Primer-Oligonukleotid kann in mehreren Teilschritten verwendet werden. In erster Linie übernimmt es eine Primer-Funktion in der Amplifikation. Dabei wird Primer-Verlängerungsreaktion unter Verwendung des zweiten Primer-Verlängerungsproduktes als Matrize ausgeführt. In einer weiteren Ausführungsform kann das erste Primer-Oligonukleotid zu Beginn der Amplifikationsreaktion die Start-Nukleinsäurekette als Matrize verwenden. In einer weiteren Ausführungsform kann das erste Primer-Oligonukleotid bei der Erstellung / Bereitstellung einer Start-Nukleinsäurekette verwendet werden.

Im Rahmen der Amplifikation dient der erste Primer als Initiator der Synthese des ersten Primer-Verlängerungsproduktes unter Verwendung des zweiten Primer-Verlängerungsproduktes als Matrize. Das 3'-Segment des ersten Primers umfasst eine Sequenz, welche vorwiegend komplementär an das zweite Primer-Verlängerungsprodukt binden kann. Die enzymatische Verlängerung des ersten Primer-Oligonukleotid unter Verwendung des zweiten Primer-Verlängerungsproduktes als Matrize führt zu Ausbildung des ersten Primer-Verlängerungsproduktes. Ein solches erstes Primer-Verlängerungsprodukt umfasst die Zielsequenz bzw. ihre Sequenz-Anteile. Im Verlauf der Synthese des zweiten Primer-Verlängerungsproduktes wird die Sequenz des kopierbaren Anteils des ersten Primer-Oligonukleotid von Polymerase als Matrize erkannt und eine entsprechende komplementäre Sequenz synthetisiert wird, so dass eine entsprechende Primer-Bindungsstelle für das erste Primer-Oligonukleotid resultiert. Die Synthese des ersten Primer-Verlängerungsproduktes erfolgt bis einschließlich 5'-Segment des zweiten Primer-Oligonukleotids. Unmittelbar nach der Synthese des ersten Primer-Verlängerungsproduktes ist dieses Produkt an das zweite Primer-Verlängerungsprodukt gebunden und bildet einen doppelsträngigen Komplex. Das zweite Primer-Verlängerungsprodukt wird aus diesem Komplex sequenzspezifisch durch das Aktivator-Oligonukleotid verdrängt. Dabei bindet das Aktivator-Oligonukleotid an das erste Primer-Verlängerungsprodukt. Nach einer erfolgreichen Strangverdrängung durch Aktivator-Oligonukleotid kann das zweite Primer-Verlängerungsprodukt wiederum selbst als Matrize für die Synthese des ersten Primer-Verlängerungsproduktes dienen. Das nun frei gewordene 3'-Segment des ersten Primer-Verlängerungsproduktes kann ein weiteres zweites Primer-Oligonukleotid binden, so dass eine neue Synthese des zweiten Primer-Verlängerungsproduktes initiiert werden kann.

Weiterhin kann das erste Primer-Oligonukleotid als Initiator der Synthese des ersten Primer-Verlängerungsproduktes ausgehend von Start-Nukleinsäurekette zu Begin der Amplifikation dienen. In einer Ausführungsform ist die Sequenz des ersten Primers vollständig komplementär zum entsprechenden Sequenzsegment einer Start-Nukleinsäurekette. In einer weiteren Ausführungsform ist die Sequenz des ersten Primer-Oligonukleotides nur teilweise komplementäre zum entsprechenden Sequenzsegment einer Start-Nukleinsäurekette. Diese abweichende Komplementarität soll das erste Primer-Oligonukleotid allerdings nicht daran hindern, eine vorwiegend sequenzspezifische Primer-Verlängerungsreaktion zu starten. Die jeweiligen Unterschiede in Komplementarität des ersten Primer-Oligonukleotides zur jeweiligen Position in der Start-Nukleinsäurekette liegen vorzugsweise im 5'-Segment des ersten Bereichs des ersten Primer-Oligonukleotides, so dass im 3'-Segment vorwiegend komplementäre Basenpaarung und Initiierung der Synthese möglich ist. Für die Initiierung der Synthese sollen beispielsweise besonders die ersten 4-10 Positionen im 3'-Segment vollkomplementär zur Matrize (Start-Nukleinsäurekette) sein. Die restlichen Nukleotidpositionen können von der perfekten Komplementarität abweichen. Das Ausmaß einer perfekten Komplementarität im restlichen 5'-Segment des ersten Bereichs des ersten Primer-Oligonukleotides kann somit Bereiche umfassen zwischen 50 % bis 100 %, besser zwischen 80 % und 100 % der Basenzusammensetzung. Je nach Länge des ersten Bereichs des ersten Primer-Oligonukleotides, umfassend die Sequenz-Abweichungen von 1 bis maximal 15 Positionen, besser 1 bis maximal 5 Positionen. Das erste Primer-Oligonukleotid kann somit eine Synthese von einer Start-Nukleinsäurekette initiieren. Bei einer darauffolgenden Synthese des zweiten Primer-Verlängerungsproduktes werden kopierbare Sequenzabschnitte des ersten Primer-Oligonukleotides von Polymerase kopiert, so dass wiederum in darauf folgenden Synthese-Zyklen eine vollkomplementäre Primer-Bindungsstelle innerhalb des zweiten Primer-Verlängerungsprodukts für die Bindung des ersten Primer-Oligonukleotides ausgebildet wird und in darauf folgenden Synthese-Zyklen zur Verfügung steht.

In einer weiteren Ausführungsform kann das erste Primer-Oligonukleotid im Rahmen der Vorbereitung einer Start-Nukleinsäurekette verwendet werden. Dabei kann ein solches erste Primer-Oligonukleotid an eine Nukleinsäure (z.B. eine einzelsträngige genomische DNA oder RNA oder ihre Äquivalente umfassend eine Zielsequenz) vorwiegend/bevorzugt sequenzspezifisch binden und in Anwesenheit einer Polymerase eine matrizenabhängige Primer-Verlängerungsreaktion initiieren. Die Bindungsposition ist dermassen gewählt, dass das Primer-Verlängerungsprodukt eine gewünschte Zielsequenz umfasst. Durch die Verlängerung des ersten Primer-Oligonukleotides resultiert ein Nukleinsäure-Strang, welcher eine zur Matrize komplementäre Sequenz aufweist. Ein solcher Strang kann von der Matrize abgelöst werden (z.B. durch Hitze oder Alkalie) und damit in eine einzelsträngige Form überführt werden. Eine solche einzelsträngige Nukleinsäurekette kann als Start-Nukleinsäurekette zu Beginn der Amplifikation dienen. Eine solche Start-Nukleinsäurekette umfasst in ihrem 5'-Segment die Sequenzanteile des ersten Primer-Oligonukleotides, weiterhin umfasst sie eine Zielsequenz oder ihre Äquivalente und eine Primer-Bindungsstelle für das zweite Primer-Oligonukleotid. Weitere Schritte sind im Abschnitt "Start-Nukleinsäurekette" erläutert.

Die Synthese des ersten Primer-Verlängerungsproduktes ist eine Primer-Verlängerungsreaktion und bildet einen Teilschritt in der Amplifikation. Die Reaktionsbedingungen während dieses Schrittes sind entsprechend angepasst. Die Reaktions-Temperatur und die Reaktions-Zeit sind dermaßen gewählt, dass die Reaktion erfolgreich stattfinden kann. Die jeweils bevorzugte Temperatur in diesem Schritt hängt von der verwendeten Polymerase ab und der Bindungsstärke des jeweiligen ersten Primer-Oligonukleotides an seine Primer-Bindungsstelle und umfasst beispielsweise Bereiche von 15°C bis 75°C, besser von 20°C bis 65°C, bevorzugt von 25°C bis 65°C. Die Konzentration des ersten Primer-Oligonukleotides umfasst Bereiche von 0,01 µmol/l bis 50 µmol/l, besser von 0,1 µmol/l bis 20 µmol/l, bevorzugt von 0,1 µmol/l bis 10 µmol/l.

In einer Ausführungsform verlaufen alle Schritte der Amplifikation unter stringenten Bedingungen, welche Ausbildung von unspezifischen Produkten / Nebenprodukten verhindern bzw. verlangsamen. Zu solchen Bedingungen zählen beispielsweise höhere Temperaturen, beispielsweise über 50°C.

Falls mehr als eine spezifische Nukleinsäurekette in einem Ansatz amplifiziert werden müssen, werden in einer Ausführungsform bevorzugt jeweils sequenzspezifische Primer-Oligonukleotide für Amplifikation von entsprechenden jeweiligen Zielsequenzen verwendet.

Vorzugsweise sind Sequenzen des ersten, des zweiten Primer-Oligonukleotides und des Aktivator-Oligonukleotides dermaßen aneinander angepasst, dass Nebenreaktionen, z.B. Primer-Dimer-Ausbildung, minimiert sind. Zu diesem Zweck sind beispielsweise die Sequenz des ersten und des zweiten Primer-Oligonukleotides aneinander dermaßen angepasst, daß beide Primer-Oligonukleotide nicht in der Lage sind, eine Amplifikations-Reaktion in Abwesenheit einer passenden Matrize und/oder einer Zielsequenz und/oder einer Start-Nukleinsäurekette zu starten bzw. zu unterstützen. Das kann beispielsweise dadurch erreicht werden, dass das zweite Primer-Oligonukleotid keine Primer-Bindungsstelle für das erste Primer-Oligonukleotid umfasst und das erste Primer-Oligonukleotid keine Primer-Bindungsstelle für das zweite Primer-Oligonukleotid umfasst. Weiterhin soll vermieden werden, dass die Primer-Sequenzen ausgedehnte eigen-komplementäre Strukturen (Self-complement) umfassen.

In einer Ausführungsform verläuft die Synthese des ersten und des zweiten Primer-Verlängerungsproduktes bei gleicher Temperatur. In einer weiteren Ausführungsform verläuft die Synthese des ersten und des zweiten Primer-Verlängerungsproduktes bei unterschiedlichen Temperaturen. In einer weiteren Ausführungsform verläuft die Synthese des ersten Primer-Verlängerungsproduktes und Strangverdrängung durch Aktivator-Oligonukleotid bei gleicher Temperatur. In einer weiteren Ausführungsform verläuft die Synthese des ersten Primer-Verlängerungsproduktes und Strangverdrängung durch Aktivator-Oligonukleotid bei unterschiedlichen Temperaturen.

### Bevorzugte Ausführungsformen des Detektions-Systems

Das Detektions-System umfasst mindestens einen Fluoreszenzreporter (Reporter) und mindestens eine Oligonukleotid-Sonde. Weiterhin kann ein Detektionssystem einen zum Fluoreszenzreporter passenden Fluoreszenzquencher (Quencher genannt) umfassen, so dass dieser Quencher in der Lage ist, die Fluoreszenzsignale des Fluoreszenzreporters unter bestimmten Umständen zu verringern bzw. die Signal-Intensität zu reduzieren. Weiterhin kann ein Detektionssystem eine zum Fluoreszenzreporter passenden Donor-Fluorophor umfassen, so dass diese Donor-Fluorophor in der Lage ist, die Fluoreszenzsignale des Fluoreszenzreporters unter bestimmten Umständen durch Energie-Übertragung zu ermöglichen. Weiterhin kann das Detektions-System das Aktivator-Oligonukleotid umfassen, wobei ein solches Aktivator-Oligonukleotid entweder einen Fluoreszenzreporter oder eine Donor-Fluorophor oder ein Fluoreszenzquencher umfasst.

Die Anordnung einzelner Elemente (Fluoreszenzreporter, Fluoreszenzquencher, Donor-Fluorophor) an der Oligonukleotid-Sonde und / oder an dem Aktivator-Oligonukleotid sollen in Anwesenheit eines ersten Primer-Verlängerungsproduktes unter Ausbildung jeweils komplementären Komplexe zu Änderung des Fluoreszenzsignals vom Fluoreszenzreporter führen. Diese Änderung kann je nach gewählter Konstellation zwischen einem Fluoreszenzreporter und/oder einer Donor-Fluorophor und/oder eines Fluoreszenzquenchers zu einer Zunahme oder Abnahme der Signal-Intensität des Flupreszenzrepoters führen. Diese Änderung kann mit bekannten geeigneten Mitteln (z.B. in einem Real-Time PCR-Gerät, wie StepOne-PCR oder Lightcycler oder Rotorgene, siehe Angaben von Herstellern) während oder nach einer abgelaufenen Reaktion detektiert werden. Die Erfassung der Änderungen des Signals kann dabei Rückschlüsse auf Verlauf der Reaktion ermöglichen: z.B. Amplitude des Signals, Kinetik, Zeit- bzw. Konzentrations-Abhängigkeit der Signal-Erscheinung. Bei Verwendung mehrere Target-Sequenzen können Multiplexe Analysen entsprechend durch unterschiedliche spektrale Eigenschaften kodiert werden, so dass auch mehrere Reaktionen parallel zu einander beobachet werden können.

In der Literatur sind viele verschiedene Anordnungen von Sonden und FluoreszenzReportern, Fluoreszenz-Quenchern und Donor-Fluorophoren bekannt. Ebenfalls sind viele Fluoreszenzreporter-Quencher sowie Fluoreszenzreporter-Donor-Fluorophoren (auch als Donor-Aceptor-Paare oder auch als FRET-Paare genannt) bekannt ("Fluorescent Energy Transfer Nucleic Acid Probes" Ed. Didenko, 2006, beispielsweise Chapter 1 and 2; Product Description "Flurescent Molecular Probes", published by Gene Link Inc.). Die meisten Sonden wurden für PCR-basierte Verfahren entwickelt, wobei sowohl spezifische Anordnung von Primern, Sonden als auch von verwendeten Polymerasen, z.B. mit 3'-Exonuklease (FEN) verwendet wurden.

Die vorliegende Erfindung beschreibt einige Ausführungsformen von Oligonukleotid-Sonden, welche zur Erfassung des Reaktionsfortschrittes besonders vorteilhaft sind.

Die Oligonukleotid-Sonde ist ein Oligonukleotid, welches vorzugsweise aus DNA-Nukleotiden aufgebaut ist. In einer anderen Ausführungsform kann dieses Oligonukleotid aus anderen Nukleotid-Monomeren aufgebaut sein, z.B. aus RNA oder aus Nukleotid-Modifikationen z.B. mit Zucker-Phosphat-Rückgrad-Modifikationen wie PTO oder LNA oder 2'-O-Me. In einer anderen Ausführungsform ist diese Oligonukleotid-Probe ein Mischpolymer, welches sowohl DNA als auch nicht DNA-Elemente (z.B. RNA, PTO LNA) umfasst. Diese Oligonukleotid-Sonde kann weitere Oligonukleotid-Modifikationen umfassen, z.B. Linker oder Spacer (z.B. C3, HEG, Abasische Monomere, z.B. THF-Modifikation).

Die Basen-Zusammensetzung der Oligonukleotid-Sonde umfasst vorzugsweise Basen, welche mit natürlichen Nukleobasen (A,C,T,G) komplementäre Bindungen unter Hybridisierungbedingungen eingehen kann. In einer weiteren Ausführungsform umfasst die Sonde auch Modifikationen, welche beispielsweise Universal-Basen umfasst (z.B. Inosine, 5-Nitro-lndol). Die Sonde kann weitere Modifikationen umfassen, welche Bindungsverhalten von Oligonukleotiden beeinflussen, z.B. MGB-Modifikationen.

Die Länge der Oligonukleotid-Sonde liegt vorzugsweise zwischen 8 und 80 Nukleotiden, besser zwischen 12 und 80 Nukleotiden, noch besser zwischen 12 und 50, bevorzugt zwischen 12 und 35 Nukleotiden.

In einer Ausführungsform ist das 3'-Ende der Oligonukleotid-Sonde durch eine Modifikation geblockt, z.B. durch einen Fluorophor oder Quencher oder Donor oder durch eine andere Modifikation, welche Polymerase daran hindert, das Oligonukleotid als Primer zu verwenden (z.B. Phosphat-Rest oder C3-Linker oder dideoxy-Nukleotid-Modifikation).

In einer weiteren Ausführungsform ist das 3'-Ende der Oligonukleotid-Sonde frei und kann mit dem ersten Primer-Verlängerungsprodukt komplementär binden und die Synthese durch die Polymerase starten. Dadurch kann die Oligonukleotid-Sonde wie ein Primer verlängert werden, was zu Bildung eines Sonden-Verlängerungsproduktes führt.

In einer Ausführungsform entspricht die Sequenzzusammensetzung der Sonde der Sequenzzusammensetzung des Primers.

Position von Fluoreszenzreporter, Fluoreszenzquencher oder Donor-Fluorophor können je nach Ausführungsform der Oligonukleotid-Sonde unterschiedlich sein. Diese Elemente können sowohl an einem der jeweiligen Enden der Oligonukleotid-Sonde kovalent gebunden werden oder im mittleren Bereich. Viele solche Modifikationen sind einem Fachmann bekannt, beispielsweise Kopplung von FAM-Reporter an das 3'-Ende oder an das 5'-Ende eines Nukleotides, oder Verwendung von dT-BHQ1 oder dT-FAM oder dT-TMR Modifikationen für Kopplung von Sonden im Mittleren bzw. inneren Sequenzsegment der Sonde. Solche modifizierte Oligonukleotid-Sonden können bei kommerziellen Anbietern bezogen werden (z.B. Sigma-Aldrich, Eurofins, IDT, Eurogentec, Thermofisher Scientific).

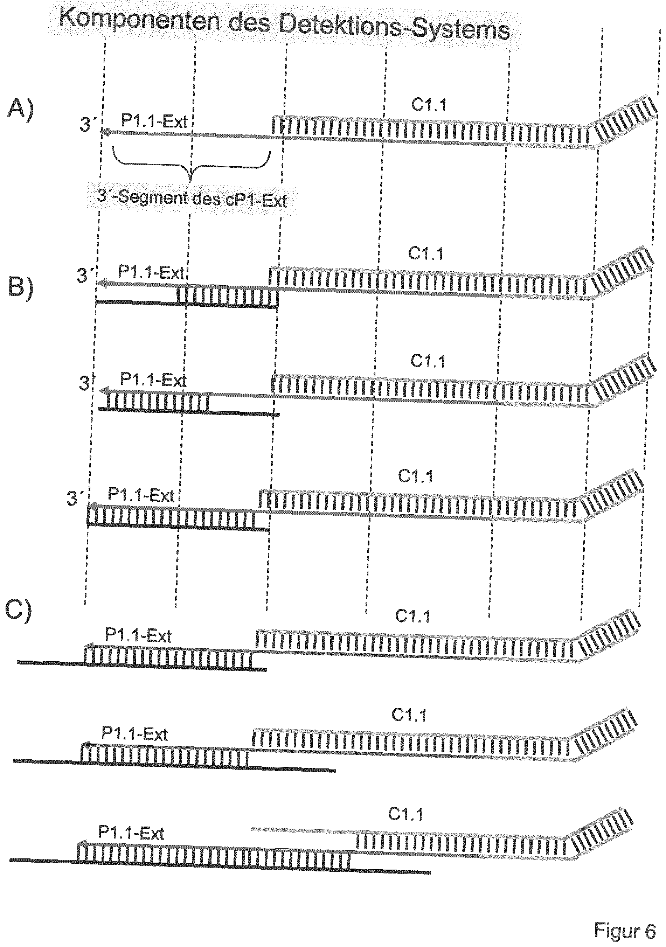

Die Oligonukleotid-Sonde umfasst zumindest ein Sequenzsegment, welches in der Lage ist, mit dem während der Amplifikation gebildeten ersten Primer-Verlängerungsprodukt eine im Wesentlihen komplementäre Bindung unter geeigneten Reaktionsbedingungen eines Detektions-Schrittes einzugehen (Fig. 6A). Dabei wird die Oligonukleotid-Sonde zumindest teilweise an das einzelsträngige 3'-Segment des synthetisierten Primer-Verlängerungsproduktes komplementär gebunden (Fig. 6B), welches nicht vom Aktivator-Oligonukleotid gebunden wird. Dadurch wird ein Komplex generiert, welches ein erstes Primer-Verlängerungsprodukt, Oligonukleotid-Sonde und Aktivator-Oligonukleotid umfasst. Die Bindung der Oligonukleotid-Sonde und des Aktivator-Oligonukleotides an das synthetisierte erste Primer-Verlängerungsprodukt erfolgt bevorzugt sequenzspezifisch. Die Länge dieses zum 3'-Segment des ersten Primer-Verlängerungsproduktes komplementären Sequenz-Segments liegt beispielsweise in Bereichen von 8 bis 80 Nukleotiden, besser zwischen 12 und 80 Nukleotiden, noch besser zwischen 12 und 50, bevorzugt zwischen 12 und 35 Nukleotiden, besonders bevorzugt zwischen 15 und 25 Nukleotiden.

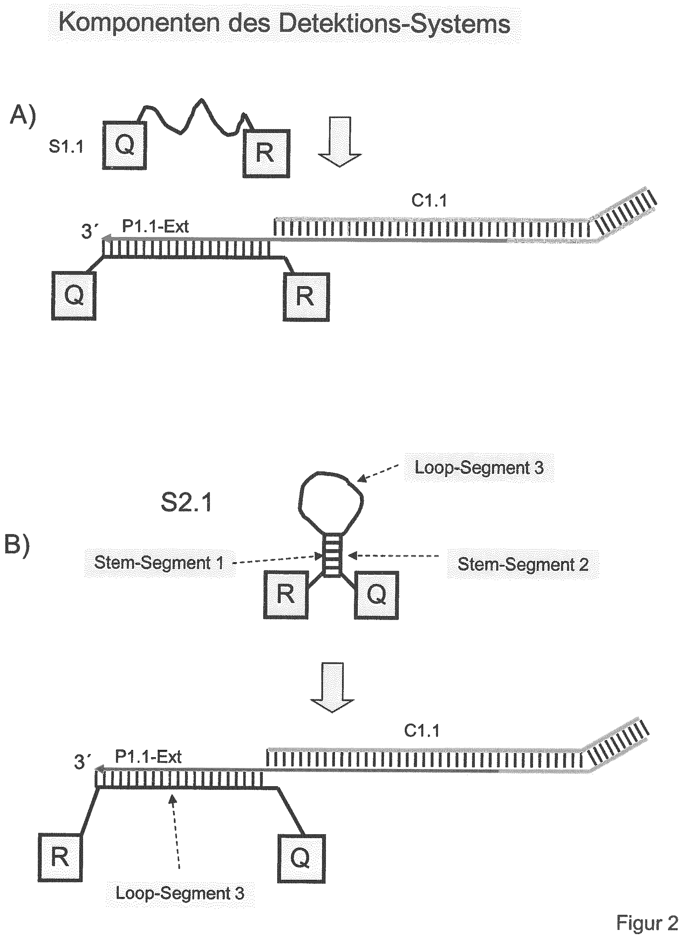

In einer Ausführungsform umfasst die Oligonukleotid-Sonde weiterhin zusätzliche Sequenzsegmente, welche mit dem ersten Primer-Verlängerungsprodukt keine komplementären Bindungen eingehen. Solche Sequenzsegmente können beispielsweise zur räumlichen Trennung von einzelnen Komponenten des Detektions-Systems von Vorteil sein (z.B, Loop-Segment 3 und Stem-Segment 2 in Fig. 2 und 3). Diese Segmente können mit Aktivator-Oligonukleotid um die Bindung an das erste Primer-Verlängerungsprodukt konkurrieren (Fig. 6C)

Das Detektions-System, umfassend zumindestens einen Fluoreszenz-Reporter gebunden entweder an der Oligonukleotid-Sonde oder am Aktivator-Oligonukleotid, ist in der Lage die Signal-Generierung bzw. Signal-Intenstität des Fluoreszenz-Reporters in Abhängigkeit von der Bindung der Oligonukleotid-Sonde an komplementäre Sequenzen zu verändern. Je nach Ausführungsform des Detektions-Systems kann diese Änderung in einer Generierung und / oder einer Zunahme oder einer Abnahme des Signals resultieren.

Während einer Amplifikation erfolgt eine Trennung des ersten und des zweiten Primer-Verlängerungsproduktes, so dass 3'-Segment des ersten Primer-Verlängerungsproduktes vorübergehend oder dauerhaft in einzelsträngier Form vorliegt. An dieses 3'-Segment kann eine Oligonukleotid-Sonde während der Reaktion oder erst nach ihrem Abschluss binden.

Die Bindung erfolgt im Wesentlichen sequenzspezifisch, kann aber auch Abweichungen von vollständiger Komplementarität tolerieren.

Die Bindung der Oligonukleotid-Sonde verhindert vorzugsweise die Amplifikation nicht. Die Konzentration der Sonde und ihre Länge werden dermassen angepasst, dass hinreichende Menge an zweiten Primern während der Reaktion auch an das 3'-Segment des ersten Primer-Verlängerungsprodutkes binden kann und somit die Synthese des zweiten Primer-Verlängerungsprodutektes initiieren kann. In der Regel liegt ein Überschuss des zweiten Primers im Reaktionsansatz vor, so dass Konzentration der Sonde geringer ist als die Konzentration des zweiten Primers. Beispielsweise kann die Konzentration der Sonde an die des zweiten Primers angepasst werden. Das resultierende Verhältnis zwischen dem zweiten Primer und Oligonukleotid-Sonde liegt vorzugsweise in folgenden Bereichen (Konzentration der Sonde zu Konzentraiton des zweiten Primers): von 1:100 bis 10:1. Bei gleichen thermodynamischen Bindungs-Eigenschaften (z.B. bei gleicher Tm) erfolgt somit keine Absättigung des 3'-Segmentes des ersten Primer-Verlängerungsproduktes durch die Oligonukleotid-Sonde.

Mit fortschreitender Reaktion wird eine hinreichende Menge an ersten Primer-Verlängerungsprodukten gebildet, so dass die Oligonukleotid-Sonde ebenfalls hinreichend binden kann, um eine erfassbare Signal-Veränderung zu bedingen.

Ein geeignetes Detektions-System umfassend mindestens einen Fluoreszenz-Reporter, auch Reporter genannt. Bei einer Anregung des Fluoreszenz-Reporters mit einer geeigneten Licht-Wellenlänge erfolgt eine Emission des Fluoreszenzsignals, welches mit geeigneten technischen Mitteln erfasst werden kann.

Ein geeignetes Detektions-System umfassend mindestens einen Fluoreszenz-Reporter umfasst weiterhin in einer Ausführungsform mindestens einen zum Fluoreszenz-Reporter passenden Fluoreszenz-Quencher. Der Fluoreszenz-Quencher (auch Quencher genannt) kann dabei als Kontakt-Quencher oder als FRET-Quencher seine Funktion entfalten. Passende Beispiele in der Literatur sind bekannt ("Fluorescent Energy Transfer Nucleic Acid Probes" Ed. Didenko, 2006, beispielsweise Chapter 1 and 2; Product Description "Flurescent Molecular Probes", published by Gene Link Inc.). Beispielsweise kann als Fluoreszenz-Reporter Fluoresein (FAM) dienen, als ein geeigneter Fluoreszenz-Quencher kann dabei BHQ-1 oder BHQ-2 oder TAMRA auftreten. Bei einer Ausführungsform können Guanosine-Nukleobasen als Quencher auftreten (z.B. in Kombination mit einem FAM als Reporter). Bei einer Anregung des Fluoreszenz-Reporters mit einer geeigneten Licht-Wellenlänge erfolgt in Abwesenheit des Quenchers eine Emission des Fluoreszenzsignals. Bei einer räumlichen Nähe zwischen dem Fluoreszenz-Reporter und einem geeigneten Quencher erfolgt alerdings Minderung/Verringerung der Intensität eines Fluoreszenz-Reporters. Mit zunehmender Entfernung/Separierung des Fluoreszenz-Reporters und des Quenchers steigt die Signalintensität an.

Ein weiteres geeignetes Detektions-System umfassend mindestens einen Fluoreszenz-Reporter umfasst weiterhin in einer Ausführungsform mindestens einen zum Fluoreszenz-Reporter passenden Donor-Fluorophor (auch Donor genannt), so dass ein FRET-Paar gebildet wird. Passende Beispiele in der Literatur sind bekannt ("Fluorescent Energy Transfer Nucleic Acid Probes" Ed. Didenko, 2006, beispielsweise Chapter 1 and 2; Product Description "Fluorescent Molecular Probes", published by Gene Link Inc.). Ein FRET-Paar umfasst in der Regel einen Donor und einen Akzeptor (in der Regel tritt ein Reporter als Akzeptor auf). Beispielsweise kann als Fluoreszenz-Reporter Tetramethylrodamin (TAMRA) dienen, als ein geeigneter Partner eines FRET-Paares kann dabei FAM (Donor) auftreten, ein anderes Beispiel ist FAM (Donor) und Cy3 (als Akzeptor oder Reporter). Bei einer räumlichen Nähe erfolgt dabei bei Anregung des Donors (z.B. FAM) die Übertragung der Energie auf den Reporter (TAMRA oder Cy3), so dass der Reporter selbst dadurch in die Lage versetzt wird, Energie als elektromagnetische Strahlung (erfassbares Lichtsignal bzw. Fluoreszenzsignal) abzugeben. Dieses Fluoreszenzsignal des Reporters kann mit geeigneten Mitteln erfasst werden. Mit zunehmender räumlicher Separierung des Reporters und des Donor-Fluorophor sinkt das Signal zunehmend und ist in der Regel ab einer Distanz von über 50 Nukleotiden (gemessen als 50 Nukleotide eines Doppelstranges) nicht mehr messbar detektierbar.

Durch geeignete Positionierung von einzelnen Elementen des Detektionssystems an Oligonukleotid-Sonde und/oder Aktivator-Oligonukleotid ist es möglich, Bindungsereignisse dieser Komponenten an das erste Primer-Verlängerungsprodukt durch Signal-Zunahme oder Signal-Abnahme zu erfassen.

Eine solche Erfassung kann entweder während der Amplifikation stattfinden (z.B. als On-Line-Erfassung) oder in geeigneten zeitlichen Abständen oder auch erst am Ende einer Reaktion erfolgen.

Nachfolgend sind einige Ausführungsbeispiele für Oligonukleotid-Sonden bzw. Kombinationen aus Oligonukleotid-Sonden und Aktivator-Oligonukleotiden aufgeführt.

In einer Ausführungsform umfasst ein geeignetes Detektionssystems folgende Komponenten (Fig. 2A):
Eine Oligonukleotid-Sonde (S1.1) ist ein Oligonukleotid, welches einen Fluoreszenz-Reporter (R) und einen Quencher (Q) umfasst. Im nicht an die komplementäre Sequenz gebundenen, vorwiegend einzelsträngigen Zustand der Sonde sind Reporter und Quencher räumlich nicht hinreichend getrennt, so dass Fluoreszenzsignal vom Reporter durch den Quencher gelöscht wird. Bei einer vorwiegend komplementären Bindung an das 3'-Segment eines P1-Ext erfolgt räumliche Trennung von Quencher und Reporter, so dass Fluoreszenzsignal zunimmt. Diese Zunahme des Signals kann während der Amplifikation und / oder erst danach detektiert werden.

In einer Ausführungsform umfasst ein geeignetes Detektionssystems folgende Komponenten (Fig. 2B):
Eine Oligonukleotid-Sonde (S2.1) ist ein Oligonukleotid, welches einen Fluoreszenz-Reporter (R) und einen Quencher (Q) umfasst. Die Sonde umfasst selbstkomplementäre Sequenzelemente (Stem-Segment 1 und Stem-Segment 2), welche durch ein Loop-Segment 3 voneinder getrennt sind. Die Sonde ist somit als "Molekular Beacon" konstruiert, d.h. die Sonde ist dermassen konstruiert, dass beide Stem-Segmente aneinander unter Ausbildung eines Doppelstranges binden können. Quencher und Reporter sind auf separaten Stem-Segment platziert. Im nicht an die komplementäre Sequenz gebundenen Zustand liegt das "Loop-Segment" der Sonde vorwiegend im einzelsträngigen Zustand (nicht gebunden an komplementäre Sequenz) vor. Dabei sind beide komplementäre Stem-Segmente (1 und 2) in der Lage einen Doppelstrang miteinander auszubilden, so dass Reporter und Quencher unter Reaktionsbedingungen des Detektionsschrittes im Wesentlichen räumlich nicht hinreichend getrennt sind und das Fluoreszenzsignal vom Reporter durch den Quencher im Wesentlichen verringert wird.

Bei einer vorwiegend komplementären Bindung des "Loop-Segment" 3 an ein Sequenz-Anteils des 3'-Segments eines P1-Ext erfolgt unter verwendeten Reaktionsbedigungen des Detektions-Schrittes Ausbildung eines Doppelstranges zwischen dem Loop-Segment 3 und dem komplementären Anteil des 3'-Segmentes des ersten Primer-Verlängerungsprodukt., was in einer räumlichen Trennung von beiden Stem-Segmenten resultiert und zu Vergrößerung der Distanz zwischen dem Quencher und dem Reporter führt. Daraus resultiert eine Verringerung des Einflusses des Quencher auf den Reporter, was zu einer Zunahme des Fluoreszenzsignals führt. Diese Zunahme des Signals kann während der Amplifikation und / oder erst danach detektiert werden.

In einer Ausführungsform umfasst ein geeignetes Detektionssystems folgende Komponenten (Fig. 3A):
Eine Oligonukleotid-Sonde (S3.1) ist ein Oligonukleotid, welches einen Reporter und einen Quencher umfasst. Die Sonde umfasst selbstkomplementäre Sequenzelemente (Stem-Segment 1 und Stem-Segment 2), welche durch ein Loop-Segment-3 voneinander getrennt sind. Die Sonde ist im Wesentlichen als "Molekular Beacon" konstruiert, d.h. die Sonde ist dermaßen konstruiert, dass beide Stem-Segmente aneinander unter Ausbildung eines Doppelstranges binden können. Quencher und Reporter sind dabei auf separaten Stem-Segmenten platziert. Im Zustand, bei welchem ein "Stem-Segment" der Sonde vorwiegend frei an ein komplementäres Sequenzsegment des anderen "Stem-Segments" binden kann, sind Reporter und Quencher unter Reaktionsbedingungen des Detektions-Schrittes im Wesentlichen räumlich nicht hinreichend getrennt und das Fluoreszenzsignal vom Reporter wird durch den Quencher verringert.

Ein "Stem-Segment 1" der Sonde kann mit einem Anteil des 3'-Segments des ersten Primer-Verlängerungsproduktes (P1-Ext) komplementäre Bindung eingehen. Weiterhin umfasst die Sonde ein weiteres Sequenz-Segment 4, welches mit dem 3'-Segment des P1-Ext einen komplementären Strang bilden kann. Dieses weitere Sequenz-Segment 4 ist vorzugsweise mit dem Stem-Segment-1 dermaßen verbunden, dass beide Sequenz-Segmente komplementär an benachbarten Sequenzabschnitten des 3'-Segments eines P1-Ext unter Reaktionsbedingungen des Detektionsschrittes binden können. Bei einer gleichzeitigen vorwiegend komplementären Bindung des Stem-Segmentes 1 und Sequenz-Segmentes 4 an das 3'-Segment eines P1-Ext erfolgt unter Reaktionsbedingungen des Detektionsschrittes eine Doppelstrang-Bildung zwischen der Sonde und dem 3'-Segment des ersten Primerverlängerungsproduktes. Dabei beteiligt sich Stem-Segment 1 an dieser Doppelstrangbildung und steht somit nicht mehr zur Verfügung als Bindungspartner für das Stem-Segment 2. Es resultiert eine räumliche Trennung von Quencher und Reporter, so dass das Fluoreszenzsignal des Reporters zunimmt. Diese Zunahme des Signals kann während der Amplifikation und/oder erst danach detektiert werden.

In einer Ausführungsform umfasst ein geeignetes Detektionssystems folgende Komponenten (Fig. 3B):
Eine Oligonukleotid-Sonde (S4.1) ist ein Oligonukleotid, welches einen Reporter und einen Quencher umfasst. Die Sonde umfasst selbstkomplementäre Sequenzelemente (Stem-Segment 1 und Stem-Segment 2), welche durch ein Loop-Segment-3 voneinder getrennt sind. Die Sonde ist im Wesentlichen als "Molekular Beacon" konstruiert, d.h. Sonde ist dermassen konstruiert, dass Quencher und Reporter auf separaten Stem-Segmenten platziert sind. Im Zustand, bei welchem ein "Stem-Segment" der Sonde vorwiegend frei an ein komplementäres Sequenzsegment des anderen "Stem-Segments" binden kann, sind Reporter und Quencher unter Reaktionsbedingungen des Detektions-Schrittes im Wesentlichen räumlich nicht hinreichend getrennt und das Fluoreszenzsignal vom Reporter wird durch den Quencher im Wesentlichen gelöscht.

Ein "Stem-Segment 12 der Sonde kann mit einem Anteil des 3'-Segments des P1-Ext komplementäre Bindung eingehen. Weiterhin umfasst die Sonde ein weiteres Sequenz-Segment 4, welches mit dem 3'-Segment des P1-Ext einen komplementären Strang bilden kann und weiterhin kann dieses Segment-4 als Primer von Polymerase erkannt und verlängert werden. Die Sequenz des Sequenz-Segmentes 4 ist dermassen angepasst, dass dieses Segment unter Verwendung des P1-Ext als Matrize die Synthese eines komplementären Stranges initiieren kann. Dieser Vorgang erfolgt unter Verdrängung des Aktivator-Oligonukleotides aus der Bindung mit dem P1-Ext. Dabei kommt es zu Ausbildung eines Primer-Verlängerungsproduktes S4.1-Ext.

In einer Ausführungsform umfasst das Stem-Segment 1 und / oder Sequenzsegment 4 zumindest eine für Polymerase nicht-kopierbare Nukleinsäurebausteine (z.B. PNA, 2'-O-Me RNA-Modifikation oder 2'-MOE RNA-Modifikationen oder Linker, wie C3 oder HEG), so dass Polymerase bei einer möglichen Rücksynthese diese Segmente nicht als Matrize verwenden kann.

Bei einer anderen Ausführungsform umfasst das Stem-Segment 1 und Sequenzsegment 4 für Polymerase kopierbare Nukleinsäurebausteine (z.B. DNA), so dass Polymerase bei einer Rücksynthese diese Segmente zumindest teilweise als Matrize verwenden kann. Es kann vorteilhaft sein, Polymerase an der Fortführung der Synthese über diese Segmente hinaus zu hindern.

Dazu können beispielsweise eine oder mehrere Modifikationen verwendet werden, welche im mittleren Bereich der Sonde (z.B. zwischen dem Stem-Segment 1 und dem Loop-Segment 3) angeordnet sind. Solche Modifikationen umfassen beispielsweise C3-Linker oder HEG-Linker oder andere Modifikationen, welche zum Polymerase-Stopp führen. Weitere Beispiele für solche Modifikationen sind im Abschnitt "Ausführungsformen des ersten Primer-Oligonukleotids" beschrieben.

In einer Ausführungsform umfasst das Loop-Segment-3 zumindest eine Modifikation, welche für eine Polymerase nicht als Matrize dient und die Fortführung der Synthese hindert (z.B. PNA oder 2'-O-Me oder 2'-MOE) oder die Synthese unterbricht (z.B. C3-Linker oder HEG-Linker).

Das Sequenz-Segment 4 ist vorzugsweise an seinem 5'-Ende mit dem Stem-Segment-1 dermassen verbunden, dass beide Sequenz-Segmente komplementär an das 3'-Segment eines P1-Ext unter Reaktionsbedigungen des Detektionsschrittes binden können. Bei einer gleichzeitigen vorwiegend komplementären Bindung des Stem-Segmentes 1 und Sequenz-Segmentes 4 an das 3'-Segment eines P1-Ext erfolgt unter Reaktionsbedigungen des Detektionsschrittes räumliche Trennung von Quencher und Reporter, so dass Fluoreszenzsignal des Reporters zunimmt. Diese Zunahme des Signals kann während der Amplifikation und / oder erst danach detektiert werden. Diese Ausführungsform wird in ***Beispiel* 2** genauer dargestellt.

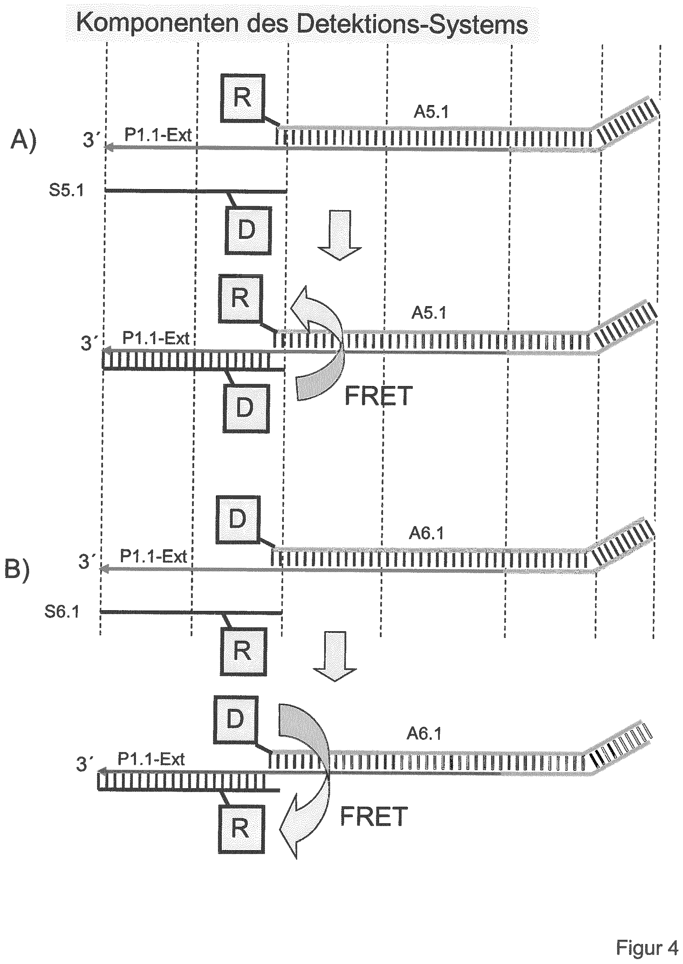

In einer Ausführungsform umfasst ein geeignetes Detektionssystems folgende Komponenten (Fig. 4A und 4B): mindestens eine Oligonukleotid-Sonde (S5.1) und mindestens ein Aktivator-Oligonukleotid (A5.1) und weiterhin zwei Fluorophore, welche ein FRET-Paar bilden. Ein Fluorophor dient als Donor, ein anderes als Akzeptor (Fluoreszenzreporter). Viele FRET-Paare / bzw. Donor-Akzeptor-Paare sind bekannt, z.B. z.B. Fluoreszein/Cy3. Dabei sind Donor und Reporter jeweils getrennt auf einem der beiden Oligonukleotide eines spezifischen Detektions-System platziert (meistens durch kovalente Bindung an das jeweilige Oligonukleotid) und dermassen angeordnet, dass es bei einer komplementären Bindung von beiden Oligonukleotiden an ein und dasselbe erste Primer-Verlängerungsprodukt zu einer räumlichen Nähe zwischen dem Donor und dem Akzeptor (Reporter) kommt, so dass eine Energie-Übertragung vom Donor auf Reporter stattfinden kann und es zu einem Fluoreszensignal des Fluorszenzreporters kommt, welches detektiert werden kann. Üblicherweise beträgt der Abstand zwischen beiden Komponenten eines FRET Paares für eine erfolgreiche Energie-Übertragung zwischen 1 bis etwa 20 Nukleotide. Ein Partner des jeweiligen FRET-Paares kann dabei im 5'-Segment des Aktivator-Oligonukleotides lokalisiert. Eine bevorzugte Position ist das 5'-Ende des Aktivator-Oligonukleotides. Ein anderer Partner des FRET-Paares ist auf dem Oligonukleotid (S5.1) bevorzugt in seinem mittleren Segment oder bevorzugt im 3'-Segment lokalisiert.

Unter Reaktionsbedingungen des Detektionsschrittes kommt es zu einer gleichzeitigen komplementären Bindung des dritten Bereichs des Aktivator-Oligonukleotides und des Oligonukleotides (S5.1) an das Primer-Verlängerungsprodukt (P1-Ext). Dabei wird das 5'-ständige Segment des Aktivator-Oligonukleotides und das 3'-ständige Segment des Oligonukleotides (S5.1) in räumliche Nähe gebracht. Die Anregung des Donors führt dabei zu Energie-Übertragung auf den Reporter, wobei ein detektierbares Fluoreszenzsignal des Fluoreszenzreporters entsteht. Diese Zunahme des Signals kann während der Amplifikation und/oder erst danach detektiert werden. Aufgrund einer hohen Spezifität der Bindung des Aktivator-Oligonukleotides an das P1-Ext unter Verdrängung des komplementären P2-Ext erfolgt die Ausbildung des FRET-Paares mit hoher Sequenzspezifität.

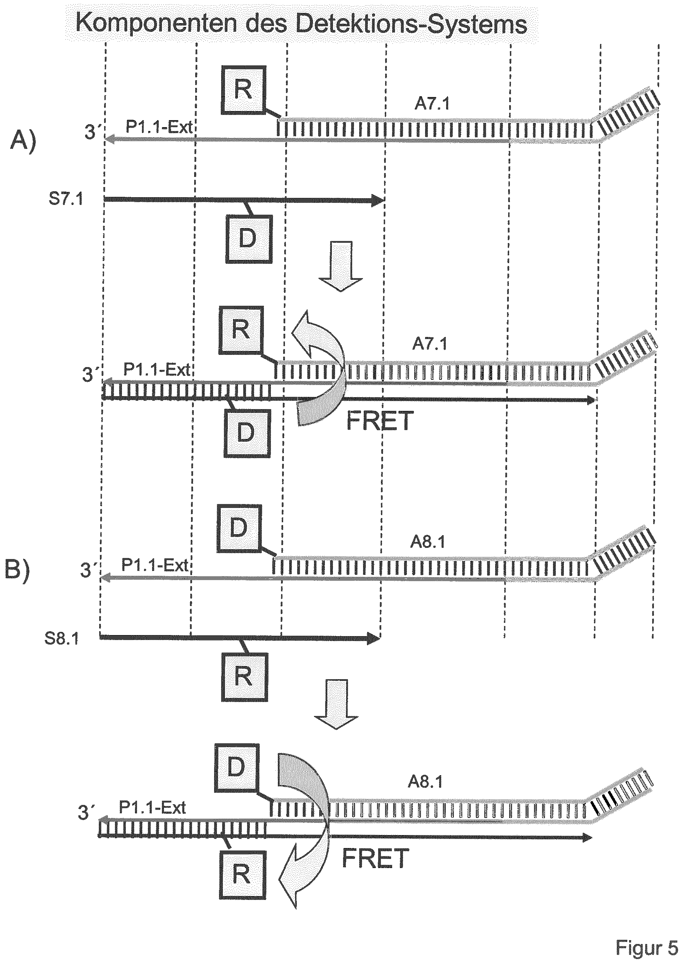

In einer Ausführungsform umfasst ein geeignetes Detektionssystems folgende Komponenten (Fig. 5A und 5B): mindestens eine Oligonukleotid-Sonde (S7.1 bzw. S8.1) und mindestens ein Aktivator-Oligonukleotid (A7.1 bzw. A8.1) und weiterhin zwei Fluorophore, welche ein FRET-Paar bilden, ähnlich wie in der Abbildung 4A und 4B. Im Unterschied dazu kann allerdings das verwendete Oligonukleotid S7.1 oder S8.1 bei einer komplementären Bindung an das P1-Ext von einer Polymerase unter Verdrängung des Aktivator-Oligonukleotides verlängert werden, so dass dabei ein S7.1-Verlängerungsprodukt (S7.1-Ext) oder S8.1-Verlängerungsprodukt (S8.1-Ext) generiert werden.

Vorzugsweise werden keine Oligonukleotid-Sonden verwendet, welche eine "Scorpion"-Struktur haben.

Erfassung des Fluoreszenzsignals erfolgt in einem Detektions-Schritt. Im Detektions-Schritt des Verfahrens soll überprüft werden, ob die Oligonukleotid-Sonde an das 3'-Segment des ersten Primer-Verlängerungsproduktes (P1-Ext) komplementär gebunden hat oder nicht. Im Detektionsschritt wird die Reaktoinstemperatur daher dermassen angepasst, dass die Sonde in der Lage wäre, an das 3'-Segment des ersten Primer-Verlängerungsproduktes im Wesentlichen komplementär zu binden. Diese Temperatur kann entweder einer der Temperaturen während der Amplifikation entsprechen oder einen von Amplifikations-Temperatur-Schritten separaten Schritt darstellen.

Während dieses Schrittes kann die Reaktion von einer Lichtquelle mit dem Licht einer geeigneten Wellenlänge angeregt werden. Je nach Ausgestaltung des Detektions-Systems wird die Wellenlänge an das Absorbtionsspektrum des Fluoreszenzreporters bzw. des Donor-Fluorophores angepasst. Falls die Oligonukleotid-Sonde an das 3'-Ende eines synthetisierten ersten Primer-Verlängerungsproduktes binden kann, ist ein Signal vom Fluoreszenzreporter zu erwarten. Dieses Signal hat ein charakteristisches Spektrum der Wellenlängen und kann durch ein entsprechendes Erfassungs-System detektiert und quantifiziert werden. Gegenwärtige Real-Time-PCR Geräte umfassen in der Regel sowohl Lichtquelle für die Anregung als auch ein Erfassungs-System zu Detektion der Fluoreszenz von Reportern, sowie temperierbare Behälter für Reaktionsgefäße. Ein Beispiel dafür stellen Real-Time PCR-Geräte wie StepOne oder LightCycler oder RotorGene.

Die Erfassung kann zur Quantifizierung von einer oder mehreren in der Reaktion vorliegenden Start-Nukleinsäureketten verwendet werden. Weiterhin kann die Erfassung zum Nachweis einer Verfügbarkeit einer Start-Nukleinsäurekette zu Beginn einer Reaktion verwendet werden. Weiterhin kann ein Detektions-System in Verbindung mit einer Internen-Amplifikations-Kontrolle verwendet werden.

In einer weiteren Ausführungsform können zwei oder mehr Nukleinsäureketten in einem Amplifikations-Ansatz spezifisch amplifiziert werden. Dabei können beispielsweise Kombinationen von spezifischen ersten Primern und/oder spezifischen Aktivator-Oligonukleotiden und/oder spezifischen zweiten Primern verwendet werden. Beispielsweise werden Zielsequenzen und eine Interne Amplifikatons-Kontrolle in einem Ansatz amplifiziert. Es ist daher vorteilhaft, die Erfassung einzelner Nukleinsäureketten während ihrer Amplifikation getrennt und unabhängig von einander durchzuführen.

In einer Ausführungsform werden jeweils spezifische Detektions-Systeme verwendet, so dass Monitoring der Amplifikation einer Nukleinsäurekette durch ein jeweils spezifisches Detektions-System erfolgt. Die jeweils spezifischen Signale von Fluoreszenzreportern können gleichzeitig detektiert werden. Vorzugsweise unterscheiden sich die spektralen Eigenschaften von Fluoreszenzreportern dermaßen, dass sie durch Erfassung von jeweiligen Fluoreszenzsignalen bei charakteristischen Wellenlängen erfolgen kann. Beispielsweise können zwei oder drei oder vier Fluoreszenzreporter verwendet werden, Die jeweiligen spezifischen Wellenlängen von Fluoreszenzsignalen liegen vorzugsweise um mehr als 10 nm, besser um mehr als 20 nm, bevorzugt um mehr als 30 nm gemessen bei maximaler Intensität (Fluoresenz-Peak) eines Fluoreszenzspektrums). Solche Kombinationen sind bekannt. Beispielsweise eignen sich Kombinationen umfassend FAM und/oder Cy3 und/oder Cy5 oder FAM und/oder HEX und/oder ROX. Die jeweiligen Quencher werden bevorzugt spezifisch für jeden Fluoreszenzreporter gewählt, so dass Signal-Minderung durch ein Quencher eine hohe Effizienz aufweist. Beispielsweise werden Kombinationen von FAM/BHQ-1 und HEX/ BHQ-2 oder FAM/ BHQ-1 und Cy5/BHQ-2 verwendet.

In einer weiteren Ausführungsform kann ein Detektions-System verwendet werden, welches Monitoring der Amplifikation einer Gruppe von unterschiedlichen Nukleinsäureketten ermöglicht. Dabei kann diese Gruppe zwei oder mehr zu amplifizierenden Nukleinsäureketten umfassen. Die Komponenten eines Detektions-System werden entsprechend angepasst. In einer Ausführungsform umfasst eine solche Gruppe von unterschiedlichen zu amplifizierenden Nukleinsäureketten bespielsweise mindestens ein einheitliches, für die verwendete Oligonukleotid-Sonde spezifisches Sequenzsegment für eine komplementäre Bindung einer Oligonukleotid-Sonde unter Reaktionsbedingungen eines Detektions-Schrittes. In einer weiteren Ausführungsform umfasst eine solche Gruppe von unterschiedlichen zu amplifizierenden Nukleinsäurketen bespielsweise mindestens ein Sequenzsegment für eine vorwiegend komplementäre Bindung einer Oligonukleotid-Sonde, wobei die Sequenzzusammensetzung dieses Sequenzsegmentes innerhalb dieser Gruppe unterschiedlich ist. Diese Unterschiede können von 1 bis 10 Nukleotide umfassen, vorzugsweise von 1 bis 3 Nukleotide.

### Bevorzugte Ausführungsformen des Aktivator-Oligonukleotids

Ein Aktivator-Oligonukleotid (Fig. 19) umfasst:
- Einen ersten einzelsträngigen Bereich, welcher an den Polynukleotid-Schwanz des zweiten Bereichs des ersten Primer-Oligonukleotides binden kann,
- einen zweiten einzelsträngigen Bereich, welcher an den ersten Bereich des ersten Primer-Oligonukleotides im wesentlich komplementär binden kann,
- einen dritten einzelsträngigen Bereich, welcher zumindest zu einem Segment des Verlängerungsprodukts des ersten Primer-Verlängerungsprodukts im Wesentlichen komplementär ist,
und das Aktivator-Oligonukleotid dient nicht als Matrize für Primerverlängerung des ersten oder des zweiten Primer-Oligonukleotids.

Im Allgemeinen wird die Sequenz des dritten Bereichs des Aktivator-Oligonukleotides an die Sequenz der zu amplifizierenden Nukleinsäure angepasst, da diese als Matrize für die Reihenfolge der Nukleotide im Verlängerungsprodukt eines ersten Primers maßgeblich ist. Die Sequenz des zweiten Bereichs von Aktivator-Oligonukleotid wird an die Sequenz des ersten Primer-Bereiches angepasst. Die Struktur des ersten Bereichs des Aktivator-Oligonukleotides wird an die Sequenz des zweiten Bereichs des ersten Primer-Oligonukleotides angepasst, vor allem an die Beschaffenheit des Polynukleotid-Schwanzes.

Ein Aktivator-Oligonukleotid kann auch weitere Sequenz-Segmente einschließen, welche nicht zum ersten, zweiten oder dritten Bereich gehören. Diese Sequenzen können beispielsweise als flankierende Sequenzen am 3'- und am 5'-Ende angebracht werden. Vorzugsweise stören diese Sequenzsegmente die Funktion des Aktivator-Oligonukleotids nicht.

Die Struktur des Aktivator-Oligonukleotids weist bevorzugt folgende Eigenschaften auf:
Die einzelnen Bereiche sind untereinander kovalent gebunden. Die Bindung kann beispielsweise via konventionelle 5'-3'-Bindung erfolgen. Es kann beispielsweise eine Phospho-Diesterbindung oder eine Nuklease-resistente Phospho-Thioesterbindung verwendet werden.

Ein Aktivator-Oligonukleotid kann mittels seines ersten Bereichs an den Polynukleotid-Schwanz des ersten Primer-Oligonukleotides binden, wobei die Bindung vorwiegend durch Hybridisierung komplementärer Basen vermittelt wird. Die Länge dieses ersten Bereichs beträgt 3-80 Nukleotide, vorzugsweise 4-40 Nukleotide, besonders bevorzugt 6-20 Nukleotide. Das Ausmaß an Sequenzübereinstimmung zwischen der Sequenz des ersten Bereichs des Aktivator-Oligonukleotids und der Sequenz des zweiten Bereichs des ersten Primer-Oligonukleotide kann zwischen 20 % und 100 %, vorzugsweise zwischen 50 % und 100 %, besonders bevorzugt zwischen 80 % und 100 % liegen. Die Bindung des ersten Bereichs des Aktivator-Oligonukleotids sollte vorzugsweise spezifisch an den zweiten Bereich des ersten Primer-Oligonukleotid unter Reaktionsbedingungen erfolgen.

Die Sequenz des ersten Bereichs des Aktivator-Oligonukleotids ist vorzugsweise dermaßen gewählt, dass die Anzahl von komplementären Basen, welche mit dem zweiten Bereich des ersten Primer-Oligonukleotids komplementär binden können, zwischen 1 und 40, besser zwischen 3 und 20, bevorzugt zwischen 6 und 15 liegt.

Da das Aktivator-Oligonukleotid keine Matrize für Polymerase darstellt, kann es Nukleotid-Modifikationen einschließen, welche die Polymerase-Funktion nicht unterstützen, welche sowohl Basenmodifikationen und/oder Zucker-Phosphat-Rückgrad-Modifikationen sein können. Das Aktivator-Oligonukleotid kann beispielsweise in seinem ersten Bereich Nukleotide und/oder Nukleotid-Modifikationen einschließen, welche aus der folgenden Liste ausgewählt sind: DNA, RNA, LNA ("locked nucleic acids" Analoga mit 2'-4'-Brückenbindung im Zuckerrest), UNA ("unlocked nucleid acids" ohne Bindung zwischen 2'-3'-Atomen des Zuckerrestes), PNA ("peptide nucleic acids" Analoga), PTO (phosphorothioate), Morpholino-Analoga, 2'-O-Alkyl-RNA Modifikationen (wie 2'-OMe, 2'-O Propargyl, 2'-O-(2-Methoxyethyl), 2'-O-Propyl-Amin), 2'-Halogen-RNA, 2'-Amino-RNA etc. Diese Nukleotide oder Nukleotid-Modifikationen sind untereinander beispielsweise durch konventionelle 5'-3'-Bindung oder 5'-2'-Bindung verknüpft. Es kann beispielsweise eine Phospho-Diesterbindung oder eine nuklease resistente Phospho-Thioesterbindung verwendet werden.

Das Aktivator-Oligonukleotid kann in seinem ersten Bereich Nukleotide und/oder Nukleotid-Modifikationen einschließen, wobei die Nukleobasen aus der folgenden Liste ausgewählt sind: Adenine und seine Analoga, Guanine und seine Analoga, Cytosine und seine Analoga, Uracil und seine Analoga, Thymine und seine Analoga, Inosine oder andere Universalbasen (z.B. Nitroindol), 2-Amino-Adenin und seine Analoga, Iso-Cytosine und seine Analoga, Iso-Guanine und seine Analoga.

Das Aktivator-Oligonukleotid kann in seinem ersten Bereich Nicht-Nukleotid-Verbindungen einschließen, welche aus der folgenden Liste ausgewählt sind: interkallierende Stoffe, welche Bindungsstärke zwischen dem Aktivator-Oligonukleotid und dem ersten Primer-Oligonukleotid beeinflussen können, z.B. MGB, Naphthalene etc. Gleiche Elemente können auch im zweiten Bereich des ersten Primers verwendet werden.

Das Aktivator-Oligonukleotid kann in seinem ersten Bereich Nicht-Nukleotid-Verbindungen einschließen, z.B. Linker wie C3, C6, HEG Linker, welche einzelne Segmente des ersten Bereichs untereinander verknüpfen.

Das Aktivator-Oligonukleotid kann mittels seines zweiten Bereichs an den ersten Primer-Bereich des ersten Primer-Oligonukleotids binden, wobei die Bindung im Wesentlichen durch Hybridisierung komplementärer Basen vermittelt wird.

Die Länge des zweiten Bereichs des Aktivator-Oligonukleotids ist an die Länge des ersten Bereichs des ersten Primer-Oligonukleotid angepasst und stimmt mit dieser vorzugsweise überein. Sie liegt zwischen ca. 3-30 Nukleotiden, vorzugsweise zwischen 5 und 20 Nukleotiden. Die Sequenz des zweiten Bereichs von Aktivator-Oligonukleotid ist vorzugsweise komplementär zum ersten Bereich des ersten Primer-Oligonukleotid. Das Maß der Übereinstimmung in Komplementarität liegt zwischen 80 % und 100 %, vorzugsweise zwischen 95 % und 100 %, bevorzugt bei 100 %. Der zweite Bereich von Aktivator-Oligonukleotid schließt vorzugsweise Nukleotid-Modifikationen ein, welche Polymerase bei Verlängerung des ersten Primer-Oligonukleotides verhindern allerdings die Ausbildung von komplementären Doppelsträngen nicht blockieren oder nicht wesentlich verhindern, beispielsweise 2'-O-Alkyl RNA-Analoga (z.B. 2'-O-Me, 2'-O-(2-Methoxyethyl), 2'-O-Propyl, 2'-O-Propargyl Nukleotid-Modifikationen), LNA, PNA oder Morpholino Nukleotid-Modifikationen. Einzelne Nukleotid-Monomere sind vorzugsweise via 5'-3'-Bindung verknüpft, aber auch alternative 5'-2'-Bindung zwischen Nukleotid-Monomeren kann verwendet werden.

Die Sequenz-Länge und ihre Beschaffenheit des ersten und des zweiten Bereichs des Aktivator-Oligonukleotides sind vorzugsweise dermassen gewählt, dass die Bindung dieser Bereiche an das erste Primer-Oligonukleotid unter Reaktionsbedingungen zumindestens in einem Reaktions-Schritt des Verfahrens reversibel ist. Das bedeutet, dass Aktivator-Oligonukleotid und das erste Primer-Oligonukleotid zwar spezifisch an einander binden können, diese Bindung soll allerdings nicht zu Ausbildung eines unter Reaktionsbedingungen dauerhaft stabilen Komplexes aus beiden Elementen führen. Vielmehr soll ein Gleichgewicht zwischen einer gebunden Komplex-Form aus Aktivator-Oligonukleotid und dem ersten Primer-Oligonukleotid und einer freien Form von einzelnen Komponenten unter Reaktionsbedingungen zumindest in einem Reaktions-Schritt angestrebt bzw. ermöglicht werden. Damit wird sichergestellt, dass zumindest ein Anteil der ersten Primer-Oligonukleotide unter Reaktionsbedingungen in freier Form vorliegt und mit der Matrize interagieren kann, um eine Primer-Verlängerungsreaktion zu initiieren. Andererseits, wird damit sichergestellt werden, dass entsprechende Sequenzbereiche des Aktivator-Oligonukleotides zu Bindung mit einem verlängerten Primer-Oligonukleotid zur Verfügung stehen.

Durch Temperatur-Wahl während der Reaktion kann der Anteil von freien, einzelsträngigen und somit reaktionsfähigen Komponenten beeinflusst werden: durch Absenkung der Temperatur binden erste Primer-Oligonukleotide an die Aktivator-Oligonukleotide, so dass beide Teilnehmer einen komplementären doppelsträngigen Komplex binden. Damit kann die Konzentration einzelsträngiger Formen einzelner Komponenten abgesenkt werden. Eine Erhöhung der Temperatur kann zu Dissoziation beider Komponenten in einzelsträngige Form führen. Im Bereich der Schmelz-Temperatur des Komplexes (Aktivator-Oligonukleotid / erstes Primer-Oligonukleotid) liegen ca. 50 % der Komponenten in einzelsträngiger Form und ca. 50 % als doppelsträngiger Komplex vor. Durch Verwendung entsprechender Temperaturen kann somit die Konzentration einzelsträngiger Formen im Reaktionsgemisch beeinflusst werden.

Bei Ausführungsformen des Amplifikationsverfahrens, welche Temperatur-Wechsel zwischen einzelnen Reaktions-Schritten umfassen, können gewünschte Reaktionsbedingungen während entsprechender Reaktions-Schritte herbeigeführt werden. Beispielsweise durch Verwendung von Temperatur-Bereichen etwa in Höhe von Schmelztemperatur von Komplexen aus Aktivator-Oligonukleotid / erstes Primer-Oligonukleotid können Anteile von jeweils freien Formen einzelner Komponenten beeinflusst werden. Dabei führt die verwendete Temperatur zu einer Destabilisierung von Komplexen umfassend Aktivator-Oligonukleotid / erstes Primer-Oligonukleotid, so dass während dieses Reaktionsschrittes einzelne Komplex-Komponente zumindest vorübergehend einzelsträngig werden und damit in die Lage versetzt werden, mit anderen Reaktionspartnern zu interagieren. Beispielsweise kann der erste Sequenzbereich des Aktivator-Oligonukleotides aus dem doppelsträngigen Komplex mit einem nichtverlängerten ersten Primer freigesetzt werden und kann somit mit dem zweiten Sequenzbereich eines verlängerten ersten Primer-Oligonukleotides interagieren und damit eine Strangverdrängung initiieren. Andererseits, führt Freisetzung eines ersten, nicht verlängerten Primer-Oligonukleotides aus einem Komplex umfassend Aktivator-Oligonukleotid / erster Primer-Oligonukleotid dazu, dass der erste Primer-Bereich einzelsträngig wird und somit mit der Matrize interagieren kann, so dass eine PrimerVerlängerung durch eine Polymerase initiiert werden kann.

Die verwendete Temperatur muss dabei nicht exakt der Schmelztemperatur des Komplexes aus Aktivator-Oligonukleotid / erstes Primer-Oligonukleotid entsprechen. Es genügt, wenn Temperatur in einem Reaktionsschritt etwa im Bereich der Schmelztemperatur verwendet wird. Beispielsweise umfasst die Temperatur in einem der Reaktionsschritte Bereiche von Tm +/- 10°C, besser Tm +/- 5°C, bevorzugt Tm +/- 3°C des Komplexes aus Aktivator-Oligonukleotid / erstes Primer-Oligonukleotid.

Eine solche Temperatur kann beispielsweise im Rahmen des Reaktions-Schrittes eingestellt werden, welcher eine sequenzspezifische Strangverdrängung durch das Aktivator-Oligonukleotid umfasst.

Bei Ausführungsformen des Amplifikationsverfahrens, welche keinen Temperatur-Wechsel zwischen einzelnen Reaktions-Schritten umfassen und Amplifikation unter isothermalen Bedingungen verläuft, werden über die Gesamtdauer der Amplifikationsreaktion Reaktionsbedingungen aufrechterhalten, bei welchen ein Gleichgewicht zwischen einer Komplex-Form aus Aktivator-Oligonukleotid und dem ersten Primer-Oligonukleotid und einer freien Form von einzelnen Komponenten möglich ist.

Das Verhältnis zwischen einer Komplex-Form aus Aktivator-Oligonukleotid und dem ersten Primer-Oligonukleotid und freien Formen einzelner Komponenten kann sowohl durch Reaktionsbedingungen (z.B. Temperatur und Mg2+ Konzentration) als auch durch Strukturen und Konzentrationen einzelner Komponenten beeinflusst werden.

Die Sequenz-Länge und ihre Beschaffenheit des ersten und des zweiten Bereichs des Aktivator-Oligonukleotides sind in einer Ausführungsform so gewählt, dass unter gegebenen Reaktionsbedingungen (z.B. im Reaktionsschritt einer Strangverdängung durch das Aktivator-Oligonukleotid) das Verhältnis zwischen einem Anteil eines freien Aktivator-Oligonukleotids und einem Anteil eines Aktivator-Oligonukleotid im Komplex mit einem ersten Primer-Oligonukleotid folgende Bereiche umfasst: von 1:100 bis 100:1, vorzugsweise von 1:30 bis 30:1, besonders bevorzugt von 1:10 bis 10:1. Das Verhältnis zwischen einem Anteil eines freien ersten Primer-Oligonukleotid und einem Anteil eines ersten Primer-Oligonukleotids im Komplex mit einem Aktivator-Oligonukleotid umfasst Bereiche von 1:100 bis 100:1, vorzugsweise von 1:30 bis 30:1, besonders bevorzugt von 1:10 bis 10:1.

In einer Ausführungsform ist die Konzentration des ersten Primer-Oligonukleotides höher als die Konzentration des Aktivator-Oligonukleotides. Dadurch liegt ein Überschuss des ersten Primers in der Reaktion vor und Aktivator-Oligonukleotid muss für seine Wirkung aus der Bindung mit dem ersten Primer durch Wahl entsprechender Reaktionstemperatur freigesetzt werden. Im Allgemeinen erfolgt das durch eine Temperaturerhöhung, bis hinreichende Konzentrationen von freien Formen des Aktivator-Oligonukleotids vorliegen.

In einer weiteren Ausführungsform ist die Konzentration des ersten Primer-Oligonukleotides niedriger als die Konzentration des Aktivator-Oligonukleotides. Dadurch liegt ein Überschuss des Aktivator-Oligonukleotids vor und das erste Primer-Oligonukleotid muss für seine Wirkung aus der Bindung mit dem Aktivator-Oligonukleotid durch Wahl entsprechender Reaktionstemperatur freigesetzt werden. Im Allgemeinen erfolgt das durch eine Temperaturerhöhung, bis hinreichende Konzentrationen von freien Formen des ersten Primer-Oligonukleotids vorliegen.

Bei isothermalen Bedingungen liegt ein Gleichgewicht vor: gewisse Anteile des ersten Primer-Oligonukleotides und Aktivator-Oligonukleotides sind aneinander gebunden, andere dagegen sind als einzelsträngige Form in der Reaktion vorhanden.

Das Aktivator-Oligonukleotid kann mittels seines dritten Bereichs an mindestens ein Segment des spezifisch synthetisierten Verlängerungsprodukts des ersten Primer-Oligonukleotids binden. Die Bindung erfolgt bevorzugt durch Hybridisierung komplementärer Basen zwischen dem Aktivator-Oligonukleotid und dem von Polymerase synthetisierten Verlängerungsprodukt.

Zu Unterstützung der Strangverdrängungsreaktion soll die Sequenz des dritten Bereichs vorzugsweise eine hohe Komplementarität zum Verlängerungsprodukt aufweisen. In einer Ausführungsform ist die Sequenz des dritten Bereichs zu 100 % dem Verlängerungsprodukt komplementär.

Die Bindung des dritten Bereichs erfolgt vorzugsweise an das Segment des Verlängerungsproduktes, welches unmittelbar an den ersten Bereich des ersten Primer-Oligonukleotides anschließt. Somit liegt das Segment des Verlängerungsprodukts vorzugsweise im 5'-Segment des gesamten Verlängerungsprodukts des ersten Primer-Oligonukleotids.

Die Bindung des dritten Bereichs des Aktivator-Oligonukleotids erfolgt vorzugsweise nicht über die gesamte Länge des Verlängerungsprodukts des ersten Primer-Oligonukleotides. Vorzugsweise bleibt ein Segment am 3'-Ende des Verlängerungsproduktes ungebunden. Dieses 3'-ständige Segment ist für die Bindung des zweiten Primer-Oligonukleotides notwendig.

Die Länge des dritten Bereichs ist entsprechend dermaßen angepasst, dass der dritte Bereich zum einen an das 5'-ständige Segment des Verlängerungsproduktes bindet, andererseits das 3'-ständige Segment des Verlängerungsproduktes nicht bindet.

Die Gesamtlänge des dritten Bereichs des Aktivator-Oligonukleotids beträgt von 2 bis 100, vorzugsweise von 6 bis 60, besonders bevorzugt von 10 bis 40 Nukleotide oder ihrer Äquivalente. Das Aktivator-Oligonukleotid kann mit dem Segment des Verlängerungsproduktes über diese Länge komplementäre Bindung eingehen und damit dieses 5'-ständige Segment des Verlängerungsprodukts aus der Bindung mit seinem komplementären Matrizen-Strang verdrängen.

Die Länge des 3'-ständigen Segments des Verlängerungsproduktes, welches nicht vom Aktivator-Oligonukleotid gebunden wird, umfasst beispielsweise Bereiche zwischen 5 und 200, besser zwischen 5 und 100, noch besser 5 und 60 Nukleotide, vorzugsweise zwischen 10 und 40, bevorzugt zwischen 15 und 30 Nukleotide.

Die Oligonukleotid Sonde kann an dieses 3'-Segment des ersten Primer-Verlängerungsproduktes komplementär binden. Die Bindung kann dabei entweder über die gesamte Länge dieses Fragmentes erfolgen oder auch nur an einen Teil des Fragmentes für die Bindung beanspruchen.

Dieses 3'-ständige Segment des Verlängerungsprodukts wird nicht vom Aktivator-Oligonukletid aus der Bindung mit dem Matrizenstrang verdrängt. Auch bei vollständig gebundenem dritten Bereich des Aktivator-Oligonukleotids an sein komplementäres Segment des Verlängerungsproduktes kann das erste Primer-Verlängerungsprodukt via sein 3'-ständiges Segment mit dem Matrizenstrang gebunden bleiben. Die Bindungsstärke dieses Komplexes ist vorzugsweise so gewählt, dass er unter Reaktionsbedingungen (Schritt e)) beispielsweise spontan dissoziieren kann. Dies kann bespielsweise dadurch erreicht werden, dass die Schmelztemperatur dieses Komplexes aus dem 3'-ständigen Segment des Verlängerungsprodukts des ersten Primer-Oligonukleotid und seinem Matrizenstrang etwa im Bereich der Reaktionstemperatur liegt oder unterhalb der Reaktionstemperatur bei einem entsprechenden Reaktionsschritt (Reaktionsschritt e). Bei geringer Stabilität dieses Komplexes im 3'-Segment des Verlängerungsprodutkes führt eine vollständige Bindung des dritten Bereichs des Aktivator-Oligonukleotid an das 5'-ständige Segment des Verlängerungsproduktes zu einer schnellen Dissoziation der ersten Primer-Verlängerungsprodukts von seinem Matrizenstrang.

Das Aktivator-Oligonukleotid hat insgesamt eine passende Struktur, um seine Funktion auszuführen: unter entsprechenden Reaktionsbedingungen ist es in der Lage, das verlängerte erste Primer-Oligonukleotid aus der Bindung mit dem Matrizenstrang sequenzspezifisch zu verdrängen, wodurch der Matrizenstrang in einzelsträngige Form überführt wird und somit für weitere Bindungen mit einem neuen ersten Primer-Oligonukleotid und deren zielsequenzspezifische Verlängerung durch Polymerase zur Verfügung steht.

Um die Funktion der Strangverdrängung zu erfüllen, sollten Bereiche eins, zwei und drei des Aktivator-Oligonukleotids vorwiegend in einzelsträngiger Form unter Reaktionsbedingungen vorliegen. Daher sollten doppelsträngige selbstkomplementäre Strukturen (z.B. Hairpins) in diesen Bereichen nach Möglichkeit vermieden werden, da sie Funktionalität des Aktivator-Oligonukleotides herabsetzen können.

Das Aktivator-Oligonukleotid soll im erfindungsgemäßen Verfahren nicht als Matrize auftreten, daher soll das erste Primer-Oligonukleotid, wenn angelagert an das Aktivator-Oligonukleotid unter Reaktionsbedingungen, nicht von Polymerase verlängert werden.

Dies wird vorzugsweise durch Verwendung von Nukleotid-Modifikationen erreicht, welche die Polymerase am Kopieren des Stranges hindern. Vorzugsweise bleibt das 3'-Ende des ersten Primer-Oligonukleotid nicht verlängert, wenn das erste Primer-Oligonukleotid an das Aktivator-Oligonukleotid unter Reaktionsbedingungen bindet.

Das Ausmaß der Blockade/Hinderung/Verlangsamung/Erschwerung der Reaktion kann zwischen vollständiger Ausprägung dieser Eigenschaft (z.B. 100 % Blockade unter gegebenen Reaktionsbedingungen) und einer partiellen Ausprägung dieser Eigenschaft liegen (z.B. 30-90 % Blockade unter gegebenen Reaktionsbedingungen). Bevorzugt werden Nukleotid-Modifikationen, welche einzeln oder in einer Reihe aneinander gekoppelt (z.B. als Sequenzfragment bestehend aus modifizierten Nukleotiden) mehr als 70 %, bevorzugt mehr als 90 %, bevorzugter mehr als 95 %, und besonders bevorzugt 100 % die Verlängerung eines ersten Primers verhindern können.

Die Nukleotid-Modifikationen können Basen-Modifikationen und/oder Zucker-Phosphat-Rest Modifikationen umfassen. Die Zucker-Phosphat-Modifikationen sind bevorzugt, da durch Kombination mit konventionellen Nukleobasen eine beliebige komplementäre Sequenz eines Aktivator-Oligonukleotides zusammengestellt werden kann. Die Nukleotide mit Modifikationen im Zucker-Phosphat-Rest, welche zu Hinderung bzw. Blockade der Synthese der Polymerase führen können, schließen beispielsweise ein: 2'-O-Alkyl-Modifikationen (z.B. 2'-O-Methyl, 2'-O-(2-Methoxyethyl), 2'-O-Propyl, 2'-O-Propargyl Nukleotid-Modifikationen), 2'-Amino-2'-Deoxy-Nukleotid-Modifikationen, 2'-Amino-Alkyl-2'-Deoxy-Nukleotid-Modifikationen, PNA, Morpholino-Modifikationen usw.

Die Blockade kann sowohl durch eine einzelne Nukleotid-Modifikation erfolgen oder erst durch Kopplung von mehreren Nukleotid-Modifikationen in Reihe erfolgen (z.B. als Sequenzfragment bestehend aus modifizierten Nukleotiden). Beispielsweise können mindestens 2, vorzugsweise mindestens 5, besonders bevorzugt mindestens 10 solcher Nukleotid-Modifikationen nebeneinander im Aktivator-Oligonukleotid gekoppelt sein.

Ein Aktivator-Oligonukleotid kann eine einheitliche Art von Nukleotid-Modifikationen aufweisen oder auch mindestens zwei verschiedene Arten der Nukleotid-Modifizierung umfassen.

Die Position solcher Nukleotid-Modifikationen im Aktivator-Oligonukleotid soll vorzugsweise die Polymerase daran hindern, das 3'-Ende eines am Aktivator-Oligonukleotid gebundenen ersten Primer-Oligonukleotids zu verlängern.

In einer Ausführungsform sind solche Nukleotid-Modifikationen im zweiten Bereich des Aktivator-Oligonukleotids lokalisiert. In einer weiteren Ausführungsform sind solche Nukleotid-Modifikationen im dritten Bereich des Aktivator-Oligonukleotids lokalisiert. In einer weiteren Ausführungsform sind solche Nukleotid-Modifikationen im zweiten und im dritten Bereich des Aktivator-Oligonukleotids lokalisiert.

Beispielsweise besteht der zweite Bereich des Aktivator-Oligonukleotids zu mindestens 20 % seiner Positionen aus solchen Nukleotid-Modifikationen, vorzugsweise zu mindestens 50 %.

Beispielsweise besteht der dritte Bereich des Aktivator-Oligonukleotids zu mindestens 20 % seiner Positionen aus solchen Nukleotid-Modifikationen, vorzugsweise zu mindestens 50 %, besonders bevorzugt zu mindestens 90%. In einer Ausführungsform umfasst der gesamte dritte Bereich Nukleotid-Modifikationen, welche eine Polymerase daran hindern, einen an einen solchen Bereichen gebundenen Primer unter Verwendung des Aktivator-Oligonukleotides als Matrize zu verlängern. In einer weiteren Ausführungsform umfasst der gesamte dritte und zweite Bereich solche Nukleotid-Modifikationen. In einer weiteren Ausführungsform umfasst der gesamte erste, zweite und dritte Bereich solche Nukleotid-Modifikationen. Somit kann das Aktivator-Oligonukleotid vollständig aus solchen Nukleotid-Modifikationen bestehen. Solch modifizierten Aktivator-Oligonukleotide können beispielsweise bei Multiplex-Analysen verwendet werden, bei welchen weitere Primer verwendet werden. Dadurch soll verhindert werden, dass es zu unbeabsichtigten Primer-Verlängerungs-Reaktionen an einem oder mehreren Aktivator-Oligonukleotiden kommt.

Die Sequenz der Nukleobasen von diesen Nukleotid-Modifikationen ist den Anforderungen an die Sequenz im jeweiligen Bereich angepasst.

Den restlichen Anteil können beispielsweise natürliche Nukleotide ausmachen, oder Nukleotid-Modifikationen, welche die Polymerasen-Funktion gar nicht oder nur unwesentlich hindern z.B. DNA-Nukleotide, PTO-Nukleotide, LNA-Nukleotide, RNA-Nukleotide. Dabei können weitere Modifikationen, beispielsweise Basenmodifikationen wie 2-Amino-Adenosin, 2-Aminopurine, 5-Methyl-Cytosine, Inosine, 5-Nitroindole, 7-Deaza-Adenosin, 7-Deaza-Guanosin, 5-Propyl-Cytosin, 5-Propyl-Uridin oder Nicht-Nukleotid-Modifikationen wie Farbstoffe, oder MGB-Modifikationen etc. z.B. zur Anpassung von Bindungsstärken von einzelnen Bereichen der Aktivator-Oligonukleotide verwendet werden. Die einzelnen Nukleotid-Monomere können via konventionelle 5'-3'-Bindung untereinander gekoppelt werden oder auch abweichend via 5'-2'-Bindung.

Ein Segment des Aktivator-Oligonikleotids mit Nukleotid-Modifikationen, welche Verlängerung von 3'-Ende eines an Aktivator-Oligonukleotid gebundenen ersten Primer-Oligonukleotids durch Polymerase verhindern, wird als "zweite Blockierungs-Einheit" bezeichnet. Die Länge dieses Segments kann zwischen 1 bis 50 Nukletid-Modifikationen einschließen, vorzugsweise zwischen 4 und 30. Dieses Segment kann beispielsweise dermaßen im Aktivator-Oligonukleotid lokalisiert sein, dass das 3'-Ende des gebundenen ersten Primer-Oligonukleotids in diesem Segment liegt. Somit kann dieses Segment die Bereiche zwei und drei überspannen.

In einer Ausführungsform werden vorzugsweise keine Linker-Strukturen oder Spacer-Strukturen, wie C3, C6, HEG-Linker zur Verhinderung der Verlängerung des 3'-Ende eines an das Aktivator-Oligonukleotid gebundenen ersten Primer-Oligonukleotids verwendet.

In einer Ausführungsform umfasst ein Aktivator-Oligonukleotid in seinem dritten Bereich mindestens eine Komponte des Detektions-Systems (z.B. Fluoreszenzreporter oder Fluoreszenzquencher oder einen Donor-Fluorophor. Die Position dieser Komponente liegt in einer Ausführungsform am 5'-Ende des Aktivator-Oligonukleotides. In einer anderen Ausführungsform liegt diese Komponente im inneren Sequenzsegment des dritten Bereichs. Dabei kann die Distanz bis zum 5'-Ende des Aktivator-Oligonukleotides zwischen 2 bis 50 Nukleotide betragen, besser zwischen 2 und 20, bevorzugt zwischen 2 und 10 Nukleotiden. Bei einer gleichzeitigen Bindung der Oligonukleotid-Sonde und des Aktivator-Oligonukleotides am selben ersten Primer-Verlängerungsprodukt kommt es dabei zu einer räumlichen Nähe der beiden Komponenten des Detektions-System (z.B. zwischen einem Fluoreszenzreporter an der Oligonukleotid-Sonde und einem Donor-Fluorophor am Aktivator-Oligonukleotid).

Das Aktivator-Oligonukleotid kann außer Bereichen eins, zwei und drei noch weitere Sequenzsegmente umfassen, welche beispielsweise im 5'-Segment oder 3'-Segment des Aktivator-Oligonukleotid die oben genannten Bereiche flankieren. Solche Sequenzelemente können beispielsweise für weitere Funktionen verwendet werden, wie beispielsweise Interaktion mit Sonden, Bindung an feste Phase etc. Solche Bereiche stören vorzugsweise die Funktion der Bereiche eins bis drei nicht. Die Länge dieser flankierenden Sequenzen kann beispielsweise zwischen 1 bis 50 Nukleotide betragen. Weiterhin kann ein Aktivator-Oligonukleotid mindestes ein Element zu Immobilisierung an einer festen Phase umfassen, z.B. ein Biotin-Rest. Weiterhin kann ein Aktivator-Oligonukleotid mindestes ein Element zu Detektion umfassen, z.B. ein Fluoreszenz-Farbstoff.

In Anwesenheit von einem Aktivator-Oligonukleotid werden neusynthetisierte Sequenzen auf ihre Sequenz-Inhalte durch Interaktion mit dem Aktivator-Oligonukleotid überprüft.
- Bei korrekter Übereinstimmung mit vorgegebenen Sequenzinhalten des Aktivator-Oligonukleotides erfolgt eine Strangverdrängung, wobei die Matrizen-Stränge von neusynthetisierten Strängen abgelöst werden. Dabei werden Primer-Bindungsstellen in einzelsträngigen Zustand überführt und stehen für neue Interaktion mit Primern und somit für eine weitere Synthese zur Verfügung. Somit wird das System aus beiden Primer-Verlängerungsprodukten in einen aktiven Zustand versetzt. Das Aktivator-Oligonukleotid hat somit eine aktivierende Wirkung auf das System.
- Bei fehlender Übereinstimmung mit vorgegebenen Sequenzinhalten des Aktivator-Oligonukleotides wird Strangverdrängung der Matrizen-Stränge von neusynthetisierten Strängen beeinflusst. Die Strangverdrängung und /oder Ablösung wird entweder quantitativ verlangsamt oder komplett aufgehoben. Es kommt somit gar nicht oder seltener zu Überführung von Primer-Bindungsstellen in einzelsträngigen Zustand. Somit stehen für neue Interaktion mit Primern gar keine oder quantitativ gesehen wenige Primer-Bindungsstellen zur Verfügung. Somit wird das System aus beiden Primer-Verlängerungsprodukten seltener in einen aktiven Zustand versetzt bzw. aktiver Zustand wird gar nicht erreicht.

Die Effizienz der Doppelstrang-Öffnung der neusynthetisierten Primer-Verlängerungsprodukte nach jedem einzelnen Synthese-Schritt wirkt sich auf die potenziell erreichbare Ausbeuten in darauf folgenden Zyklen aus: je weniger freie/einzelsträngige Primer-Bindungsstellen einer zu amplifizierenden Nukleinsäurekette zu Beginn eines Synthese-Schrittes zu Verfügung gestellt werden, um so geringer ist die Anzahl von neusynthetisierten Stränge der zu amplifizierenden Nukleinsäurekette in diesem Schritt. Anders ausgedrückt: Die Ausbeute eines Synthese-Zyklus ist proportional der Menge von Primer-Bindungsstellen, welche für Interaktion mit entsprechenden komplementären Primern zur Verfügung stehen. Insgesamt kann dadurch ein Regelungs-Kreis realisiert werden.

Dieser Regelungs-Kreis entspricht einer Echtzeit-Kontrolle (real-time / on-line control) von synthetisierten Fragmenten: Sequenz-Kontrolle erfolgt im Reaktionsansatz während Amplifikation stattfindet. Diese Sequenzkontrolle erfolgt nach einem vorgegebenen Muster und das Oligonukleotid-System (durch Strang-öffnende Wirkung von Aktivator-Oligonukleotid) kann ohne äußere Einmischung zwischen "korrekten" und "nicht-korrekten" Zuständen entscheiden. Im korrekten Zustand wird die Synthese von Sequenzen fortgesetzt, im nicht-korrekten Zustand wird die Synthese entweder verlangsamt oder vollständig verhindert. Die resultierenden Unterschiede in Ausbeuten an "korrekten" und "nicht-korrekten" Sequenzen nach jedem Schritt wirken sich auf die gesamte Amplifikation aus, welche eine Vielzahl solcher Schritte umfasst.

Bei einer exponentiellen Amplifikation ist diese Abhängigkeit exponentiell, so dass sogar geringfügige Unterschiede in der Effizienz im Rahmen eines einzelnen Synthese-Zyklus aufgrund Sequenzunterschiede eine signifikante zeitliche Verzögerung der Gesamtamplifikation bedeuten können, bzw. das vollständige Ausbleiben einer detektierbaren Amplifikation in einem vorgegebenen Zeitrahmen bewirken.

Dieser Effekt der Echtzeit-Kontrolle der neusynthetisierten Nukleinsäureketten ist verbunden mit dem Einsatz des Aktivator-Oligonukleotides und der Einfluss von Aktivator-Oligonukleotid im Rahmen einer Amplifikation geht somit über die Länge von Primer-Oligonukleotide deutlich hinaus.

### Reaktionsbedingungen bei Strangverdrängungsreaktion

Die Verdrängung des zweiten Primer-Verlängerungsproduktes aus der Bindung mit dem ersten Primer-Verlängerungsprodukt mittels einer sequenzabhänigen Strangverdrängung durch das Aktivator-Oligonukleotid bildet einen Teilschritt in der Amplifikation. Die Reaktionsbedingungen während dieses Schrittes sind entsprechend angepasst. Die Reaktions-Temperatur und die Reaktions-Zeit sind dermassen gewählt, dass die Reaktion erfolgreich stattfinden kann.

In einer bevorzugten Ausführungsform verläuft die Strangverdrängung durch das Aktivator-Oligonukleotid bis zur Ablösung/Dissoziation des zweiten Primer-Verlängerungsproduktes aus der Bindung mit dem ersten Primer-Verlängerungsprodukt. Eine solche Dissoziation des 3'-Segment des ersten Primer-Verlängrungsproduktes von komplementären Anteilen des zweiten Primerverlängerungsproduktes kann spontan erfolgen im Rahmen einer temperatur-abhängigen/temperatur-bedingten Trennung von beiden Primer-Verlängerungprodukten. Eine solche Dissoziation wirkt sich günstig auf Kinetik der Amplifikationsreaktion und kann durch die Wahl der Reaktionsbedingungen beeinflusst werden, z.B. mittels Temperatur-Bedingungen. Die Temperatur-Bedingungen werden deshalb dermassen gewählt, dass eine erfolgreiche Strangverdrängung durch komplementäre Bindung des Aktivator-Oligonukleotides eine Dissoziation des zweiten Primer-Verlängerungsproduktes vom 3'-Segment des ersten Primer-Verlängerungsproduktes begünstigt.

In einer weiteren bevorzugten Ausführungsform verläuft die Strangverdrängung durch das Aktivator-Oligonukleotid bis zur Ablösung / Dissoziation eines 3'-Segmentes des zweiten Primer-Verlängerungsproduktes (P2.1-Ext) aus der komplementären Bindung mit dem ersten Primer-Verlängerungsprodukt (P1.1-Ext), wobei dieses 3'-Segment des zweiten Primer-Verlängerungsproduktes (P2.1-Ext) zumindest einen komplementeren Bereich zum ersten Primer umfasst und ein komplementäres Segment zum ersten Primerverlängerungsprodukt (P1.1-Ext), welches erst bei der enzymatsichen Synthese entstanden ist. Dabei kommt es zu Ausbildung eines Komplexes (C1.1/ P1.1-Ext / P2.1.-Ext), welches sowohl das erste Primer-Verlängerungsprodukt (P1.1-Ext), das zweite Primer-Verlängerungsprodukt (P2.1-Ext) sowie das Aktivator-Oligonukleotid (C1.1) umfasst. In einem solchen Komplex liegt das 3'-Segment des zweiten Primer-Verlängerungsproduktes zumindest vorübergehend in einer einzelsträngiger Form vor, da es vom Aktivator-Oligonukleotid aus seiner Bindung mit dem ersten Primer-Verlängerungsprodukt verdrängt werden kann. Schematisch dargestellt, besteht ein Gleichgewicht zwischen Bindung und Ablösung des P2.1-Ext an das P1.1-Ext. Das 3'-Segment des ersten Primer-Verlängerungsproduktes (P1.1-Ext) liegt in einem solchen Komplex komplementär hybridisiert an das zweite Primer-Verlängerungsprodukt (P2.1-Ext).

Aufgrund einer vorübergehenden bzw. teilweise offenen Primer-Bindungsstelle für das erste Primer-Oligonukleotid (3'-Segment des zweiten Primer-Verlängerungsprodukte) kann ein neues Primer-Verlängerungsprodukt (P1.2) an eine dieses einzelsträngigers Sequenzsegment des noch im Komplex liegenden (P2.1-Ext) unter Reaktionsbedinungen binden und somit eine Synthese eines neuen ersten Primer-Verlängerungsproduktes (P1.2-Ext) durch eine Polymerase initiieren. In der Regel verläuft die Initiierung dieser Reaktion mit verminderter Effizienz, da 3'-Segment des P2.1-Ext nicht dauerhaft einzelsträngig vorliegt, sondern im kompetetiven Verhalten mit Aktivator-Oligonukleotid steht und damit abwechselnd einzelsträngige und doppelsträngige Zustände durch Bindung an das P1.1-Ext aufweist.

Fortführung dieser neugestarteten Synthese von P1.2-Ext unter Verwendung des zweiten Primer-Verlängerungsproduktes als Matrize (P2.1-Ext) kann durch Polymerase-bedingte Strandverdrängung ebenfalls bei Dissoziiereung des Komplexes (C1.1/ P1.1-Ext / P2.1.-Ext) mitwirken. Dabei wirken Aktivator-Oligonukleotid, temperatur-abhängige doppelstrangdestabilisierung und Strang-Verdrängung durch die Polymerase synnergistisch und komplementär. Es resultiert eine Dissoziation des 3'-Segment des ersten Primer-Verlängrungsproduktes (P1.1-Ext) von komplementären Anteilen des zweiten Primerverlängerungsproduktes (P2.1-Ext).

Eine solche Dissoziation wirkt sich günstig auf Kinetik der Amplifikationsreaktion und kann durch die Wahl der Reaktionsbedinungen beeinflusst werden, z.B. mittels Temperatur-Bedinungen. Das Mitwirken der Polymerase-vermittelten syntheseabhängigen Strangverdrängung an der Dissoziierung von P1.1-Ext und P2.1-Ext hat einen begünstigenden Effekt bei der Strangtrennung.

Die Temperatur in diesem Schritt umfasst beispielsweise Bereiche von 15°C bis 75°C, besser von 30°C bis 70°C, bevorzugt von 50°C bis 70°C.

Bei gegebener Länge des ersten Bereichs des Aktivator-Oligonukleotides und des zweiten Bereichs des ersten Primer-Oligonukleotides (umfassend beispielsweise Bereiche von 3 bis 25 Nukleotid-Monomere, besser von 5 bis 15 Nukleotid-Monomere) kann eine Strangverdrängungsreaktion im allgemeinen erfolgreich initiiert werden. Bei vollständiger Komplementarität des Aktivator-Oligonukleotid zu entsprechenden Anteilen des ersten Primer-Verlängerungsproduktes kann das Aktivator-Oligonukleotid an das erste Primer-Verlängerungsprodukt bis auf das 3'-Segment des ersten Primer-Verlängerungsproduktes binden und das zweite Primer-Verlängerungsprodukt verdrängen. Das zweite Primer-Verlängerungsprodukt verbleibt somit in Verbindung mit dem 3'-Segment des ersten Primer-Verlängerungsproduktes. Diese Stärke dieser Verbindung kann temperaturabhängig beeinflusst werden. Beim Erreichen einer kritischen Temperatur kann diese Verbindung zerfallen und beide Primer-Verlängerungsprodukte dissoziieren. Je kürzer die Sequenz des 3'-Segmentes ist, umso instabiler ist diese Verbindung und umso niedriger kann die Temperatur sein, welche eine spontane Dissoziation herbeiführt.

Eine spontane Dissoziation kann beispielsweise im Temperatur-Bereich erreicht werden, welcher etwa bei der Schmelztemperatur liegt. In einer Ausführungsform liegt die Temperatur der Schritte der Strangverdrängung durch das Aktivator-Oligonukleotid etwa bei der Schmelztemperatur (Tm +/- 3°C) des Komplexes umfassend das 3'-Segment des ersten Primer-Verlängerungsproduktes, welches nicht von Aktivator-Oligonukleotid gebunden wird, und dem zweiten Primer-Oligonukleotid bzw. dem zweiten Primer-Verlängerungsprodukt.

In einer Ausführungsform liegt die Temperatur der Schritte der Strangverdrängung durch das Aktivator-Oligonukleotid etwa bei der Schmelztemperatur (Tm +/- 5°C) des Komplexes umfassend das 3'-Segment des ersten Primer-Verlängerungsproduktes, welches nicht von Aktivator-Oligonukleotid gebunden wird, und dem zweiten Primer-Oligonukleotid bzw. dem zweiten Primer-Verlängerungsprodukt.

In einer Ausführungsform liegt die Temperatur der Schritte der Strangverdrängung durch das Aktivator-Oligonukleotid über der Schmelztemperatur des Komplexes umfassend das 3'-Segment des ersten Primer-Verlängerungsproduktes, welches nicht von Aktivator-Oligonukleotid gebunden wird, und dem zweiten Primer-Oligonukleotid bzw. dem zweiten Primer-Verlängerungsprodukt. Eine solche Temperatur umfasst Temperatur-Bereiche von etwa Tm + 5°C bis Tm+20°C, besser von Tm+5°C bis Tm +10°C. Durch Verwendung einer höheren Temperatur kann das Gleichgewicht in diesem Reaktionsschritt in Richtung Dissoziation verschoben werden. Dadurch kann die Kinetik der Reaktion günstig beeinflusst werden. Verwendung von zu niedrigen Temperaturen im Schritt der Strangverdrängung mittels Aktivator-Oligonukleotides kann zu einer signifikanten Verlangsamung der Amplifikation führen.

In einer Ausführungsform umfasst ein erstes Primer-Verlängerungsprodukt ein 3'-Segment, welches nicht von Aktivator-Oligonukleotid gebunden wird, und welches Sequenzlängen von 9 bis etwa 18 Nukleotiden umfasst. Bei dieser Ausführungsform kann eine spontane Dissoziation in der Regel bereits bei Temperatur-Bereichen zwischen 40°C und 65°C erreicht werden. Auch höhere Temperaturen führen zu einer Dissoziation.

In einer Ausführungsform umfasst ein erstes Primer-Verlängerungsprodukt ein 3'-Segment, welches nicht von Aktivator-Oligonukleotid gebunden wird, und welches Sequenzlängen von 15 bis etwa 25 Nukleotiden umfasst. Bei dieser Ausführungsform kann eine spontane Dissoziation in der Regel bereits bei Temperatur-Bereichen zwischen 50°C und 70°C erreicht werden. Auch höhere Temperaturen führen zu einer Dissoziation.

In einer Ausführungsform umfasst ein erstes Primer-Verlängerungsprodukt ein 3'-Segment, welches nicht von Aktivator-Oligonukleotid gebunden wird, und welches Sequenzlängen von 20 bis etwa 40 Nukleotiden umfasst. Bei dieser Ausführungsform kann eine spontane Dissoziation in der Regel bereits bei Temperatur-Bereichen zwischen 50°C und 75°C erreicht werden. Auch höhere Temperaturen führen zu einer Dissoziation.
Die Zusammensetzung des 3'-Segmentes des ersten Primer-Verlängerungsproduktes und ggf. eine Einführung von Schmelztemperatur-beeinflussenden Oligonukleotid-Modifikationen (z.B. MGB) bzw. Reaktionsbedingungen (z.B. TPAC, Betaine) kann die Wahl der Temperatur beeinflussen. Entsprechende Anpassung kann daher vorgenommen werden.

In einer Ausführungsform verlaufen alle Schritte der Amplifikation unter stringenten Bedingungen, welche Ausbildung von unspezifischen Produkten/Nebenprodukten verhindern bzw. verlangsamen. Zu solchen Bedingungen zählen beispielsweise höhere Temperaturen, beispielsweise über 50°C.

In einer Ausführungsform verlaufen die Einzelschritte der Strangverdrängung durch Aktivator-Oligonukleotide bei gleicher Temperatur wie die Synthese des ersten und des zweiten Primer-Verlängerungsproduktes. In einer weiteren Ausführungsform verläufen die Einzelschritte der Strangverdrängung durch Aktivator-Oligonukleotide bei Temperatur, welche von der Temperatur der jeweiligen Synthese des ersten und des zweiten Primer-Verlängerungsproduktes abweicht. In einer weiteren Ausführungsform verläuft die Synthese des ersten Primer-Verlängerungsproduktes und Strangverdrängung durch Aktivator-Oligonukleotid bei gleicher Temperatur. In einer weiteren Ausführungsform verläuft die Synthese des zweiten Primer-Verlängerungsproduktes und Strangverdrängung durch Aktivator-Oligonukleotid bei gleicher Temperatur.

Die Konzentration des Aktivator-Oligonukleotides umfasst Bereiche von 0,01 µmol/l bis 50 µmol/l, besser von 0,1 µmol/l bis 20 µmol/l, bevorzugt von 0,1 µmol/l bis 10 µmol/l.

### Bevorzugte Ausführungsformen des zweiten Primer-Oligonukleotides (Primer-2):

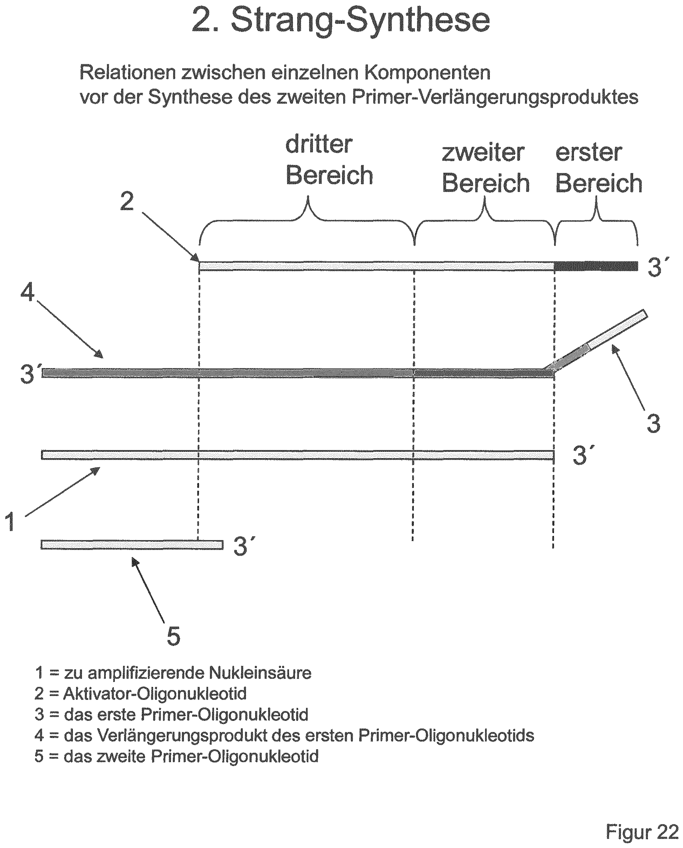

Ein Oligonukleotid, welches mit seinem 3'-Segment in der Lage ist, an eine im Wesentlichen komplementäre Sequenz innerhalb der zu amplifizierenden Nukleinsäure oder ihrer Äquivalente zu binden und eine spezifische zweite Primer-Verlängerungsreaktion zu initiieren (Fig 22 bis 25). Dieses zweite Primer-Oligonukleotid ist somit in der Lage, an das 3'-Segment eines ersten spezifischen Primer-Verlängerungsproduktes des ersten Primer-Oligonukleotids zu binden und eine polymerase-abhängige Synthese eines zweiten Primer-Verlängerungsproduktes zu initiieren. In einer Ausführungsform ist ein jedes zweites Primer-Oligonukleotid spezifisch für je eine zu amplifizierende Nukleinsäure.

Der zweite Primer-Oligonukleotid soll bei Rücksynthese kopiert werden können und dient auch als selbst Matrize für im Rahmen der Synthese des ersten Primer-Verlängerungsproduktes.

Die Länge des zweiten Primer-Oligonukleotids kann zwischen 15 und 100 Nukleotiden liegen, bevorzugt zwischen 20 und 60 Nukleotiden, besonders bevorzugt zwischen 30 und 50 Nukleotiden. Die Nukleotid-Bausteine sind vorzugsweise untereinander via übliche 5'-3' Phosphodieser-Bindung oder Phosphothioester-Bindung verknüpft. Ein solches Primer-Oligonukleotid kann in gewünscher Form chemisch synthetisiert werden.

In einer Ausführungsform kann das zweite Primer-Oligonukleotid Nukleotid-Monomere einschließen, welche Funktion der Polymerase nicht oder nur unwesentlich beeinflussen, dazu gehöhren beispielsweise:
- natürliche Nukleotide (dA, dT, dC, dG etc.) oder deren Modifikationen ohne veränderte Basen-Paarung
- Modifizierte Nukleotide, 2-Amino-dA, 2-thio-dT oder andere Nukleotid-Modifikationen mit abweichender Basen-Paarung (z.B. Universal-Basenpaaren, wie beispielsweise Inosine 5-Nitroindol).

In einer bevorzugten Ausführungsform ist das 3'-OH-Ende dieses Bereichs vorzugsweise frei von Modifikationen und hat eine funktionsfähige 3'-OH Gruppe, welche von Polymerase erkannt und matrizenabhänig verlängert werden kann. In einer weiteren bevorzugten Ausführungsform umfasst Das 3'-Segment des zweiten Primers mindestens eine Phosphorothioat-Verbindung, so dass kein Abbau von 3'-Ende des Primern durch 3'-Exonuklease Aktivität von Polymerasen erfolgen kann.

Das zweite Primer-Oligonukleotid kann in mehreren Teil-Schritten verwendet werden. In erster Linie übernimmt es eine Primer-Funktion in der Amplifikation. Dabei wird Primer-Verlängerungsreaktion unter Verwendung des ersten Primer-Verlängerungsproduktes als Matrize ausgeführt. In einer weiteren Ausführungsform kann das zweite Primer-Oligonukleotid zu Beginn der Amplifikationsreaktion die Start-Nukleinsäurekette als Matrize verwenden. In einer weiteren Ausführungsform kann das zweite Primer-Oligonukleotid bei der Erstellung/Bereitstellung einer Start-Nukleinsäurekette verwendet werden.

Im Rahmen der Amplifikation dient der zweiten Primer als Initiator der Synthese des zweiten Primer-Verlängerungsproduktes unter Verwendung des ersten Primer-Verlängerungsproduktes als Matrize. Das 3'-Segment des zweiten Primers umfasst eine Sequenz, welche vorwiegend komplementär an das erste Primer-Verlängerungsprodukt binden kann. Die enzymatische Verlängerung des zweiten Primer-Oligonukleotid unter Verwendung des ersten Primer-Verlängerungsproduktes als Matrize führt zu Ausbildung des zweiten Primer-Verlängerungsproduktes. Eine solche Synthese erfolgt typischerweise parallel zu Verdrängung des Aktivator-Oligonukleotides aus seiner Bindung mit dem ersten Primer-Verlängerungsprodukt. Diese Verdrängung erfolgt vorwiegend durch Polymerase und kann teilweise durch das zweite Primer-Oligonukleotid erfolgen. Ein solches zweites Verlängerungsprodukt umfasst die Zielsequenz oder ihre Segmente. Im Verlauf der Synthese des zweiten Primer-Verlängerungsproduktes wird die Sequenz des kopierbaren Anteils des ersten Primer-Oligonukleotid von Polymerase als Matrize erkannt und eine entsprechende komplementäre Sequenz synthetisiert wird. Diese Sequenz liegt im 3'-Segment des zweiten Primer-Verlängerungsproduktes und umfasst Primer-Bindungsstelle für das erste Primer-Oligonukleotid. Die Synthese des zweiten Primer-Verlängerungsproduktes erfolgt bis zur Stopp-Position im ersten Primer-Oligonukleotid. Unmittelbar nach der Synthese des zweiten Primer-Verlängerungsproduktes ist dieses Produkt an das erste Primer-Verlängerungsprodukt gebunden und bildet doppelsträngigen Komplex. Das zweite Primer-Verlängerungsprodukt wird aus diesem Komplex sequenzspezifisch durch das Aktivator-Oligonukleotid verdrängt. Nach einer erfolgreichen Strangverdrängung durch Aktivator-Oligonukleotid kann das zweite Primer-Verlängerungsprodukt wiederum selbst als Matrize für die Synthese des ersten Primer-Verlängerungsproduktes dienen.

Weiterhin kann das zweite Primer-Oligonukleotid als Initiator der Synthese des zweiten Primer-Verlängerungsproduktes ausgehend von Start-Nukleinsäurekette zu Begin der Amplifikation dienen. In einer Ausführungsform ist die Sequenz des zweiten Primers vollständig komplementär zum entsprechenden Sequenzsegment einer Start-Nukleinsäurekette. In einer weiteren Ausführungsform ist die Sequenz des zweiten Primer-Oligonukleotides nur teilweise komplementäre zum entsprechenden Sequenzsegment einer Start-Nukleinsäurekette. Diese abweichende Komplementarität soll das zweite Primer-Oligonukleotid allerdings nicht daran hindern, eine vorwiegend sequenzspezifische Primer-Verlängerungsreaktion zu starten. Die jeweiligen Unterschiede in Komplementarität des zweiten Primer-Oligonukleotides zur jeweiligen Position in der Start-Nukleinsäurekette liegen vorzugsweise im 5'-Segment des zweiten Primer-Oligonukleotides, so dass im 3'-Segment vorwiegend komplementäre Basenpaarung und Initiierung der Synthese möglich ist. Für die Initiierung der Synthese sollen beispielsweise besonders die ersten 4-10 Positionen im 3'-Segment vollkomplementär zu Matrize (Start-Nukleinsäurekette) sein. Die restlichen Nukleotidpositionen können von perfekten Komplementarität abweichen. Das Ausmaß einer perfekten Komplementarität im 5'-Segment kann somit Bereiche umfassen zwischen 10 % bis 100 %, besser zwischen 30 % und 100 % der Basenzzusammensetzung. Je nach Länge des zweiten Primer-Oligonukleotides, umfassen diese Abweichung von einer vollständigen Komplementarität im 5'-Segment von 1 bis 40, besser 1 bis 20 Nukleotid-Positionen. In einer weiteren Ausführungsform bindet das zweite Primer-Oligonukleotid nur mit seinem 3'-Segment an die Start-Nukleinsäurekette, aber nicht mit seinem 5'-Segment. Die Länge eines solchen zu Start-Nukleinsäurekette vollkomplementären 3'-Segment des zweiten Primer-Oligonukleotides umfasst Bereiche zwischen 6 und 40 Nukleotide, besser, zwischen 6 und 30 Nukleotide, bevorzugt zwischen 6 und 20. Die Länge eines entsprechenden, zu Start-Nukleinsäurekette nicht komplementären 5'-Segments des zweiten Primer-Oligonukleotides umfasst Bereiche zwischen 5 und 60, besser zwischen 10 und 40 Nukleotide. Das zweite Primer-Oligonukleotid kann somit eine Synthese von einer Start-Nukleinsäukette initiieren. Bei einer darauffolgenden Synthese des ersten Primer-Verlängerungsproduktes werden Sequenzabschnitte des zweiten Primer-Oligonukleotides von Polymerase kopiert, so dass wiederum in darauf folgenden Synthese-Zyklen eine vollkomplementäre Primer-Bindungsstelle innerhalb des ersten Primer-Verlängerungsprodukte für die Bindung des zweiten Primer-Oligonukleotides ausgebildet wird und in darauf folgenden Synthese-Zyklen zur Verfügung steht.

In einer weiteren Ausführungsform kann das zweite Primer-Oligonukleotid im Rahmen der Vorbereitung einer Start-Nukleinsäurekette verwendet werden. Dabei kann ein solches zweite Primer-Oligonukleotid an eine Nukleinsäure (z.B. eine einzelsträngige genomische DNA oder RNA oder ihre Äquivalente umfassend eine Zielsequenz) vorwiegend/bevorzugt sequenzspezifisch binden und in Anwesenheit einer Polymerase eine matrizenabhängige Primer-Verlängerungsreaktion initiieren. Die Bindungsposition ist dermassen gewählt, dass das Primer-Verlängerungsprodukt eine gewünschte Zielsequenz umfasst. Durch die Verlängerung des zweiten Primer-Oligonukleotides resultiert ein Strang, welche zu Matrize komplementäre Sequenz aufweist. Ein solcher Strang kann von der Matrize abgelöst werden (z.B. durch Hitze oder Alkalie) und damit in eine einzelsträngige Form überführt werden. Eine solche einzelsträngige Nukleinsäurekette kann als Start-Nukleinsäurekette zu Beginn der Amplifikation dienen. Eine solche Start-Nukleinsäurekette umfasst in ihrem 5'-Segment die Sequenzanteile des zweiten Primer-Oligonukleotides, weiterhin umfasst sie eine Zielsequenz oder ihre Äquivalente und eine Primer-Bindungsstelle für das erste Primer-Oligonukleotid. Weitere Schritte sind im Abschnitt "Start-Nukleinsäurekette" erläutert.

In einer bevorzugten Ausführungsform umfasst das zweite Primer-Oligonukleotid zumindest in seinem 3'-Segment Sequenzanteile, welche komplementär und sequenzspezifisch an ein Sequenzsegment einer Zielsequenz binden können und eine erfolgreiche Primer-Verlängerungsreaktion durch Polymerase initiieren/unterstützen. Die Länge eines solchen Sequenzsegments umfasst Bereiche von 6 und 40 Nukleotide, besser von 8 bis 30 Nukleotide, bevorzugt von 10 bis 25 Nukleotide.

In einer Ausführungsform umfasst das zweite Primer-Oligonukleotid in seinem 3'- und 5'-Segment kopierbare Sequenzabschnitte, welche bei Synthese des ersten Primer-Verlängerungsproduktes von Polymerase kopiert werden. Somit werden alle Sequenzabschnitte des zweiten Primers von Polymerase kopiert. Das führt zu Ausbildung einer Primer-Bindungsstelle im 3'-Segment des ersten Primer-Verlängerungsproduktes.

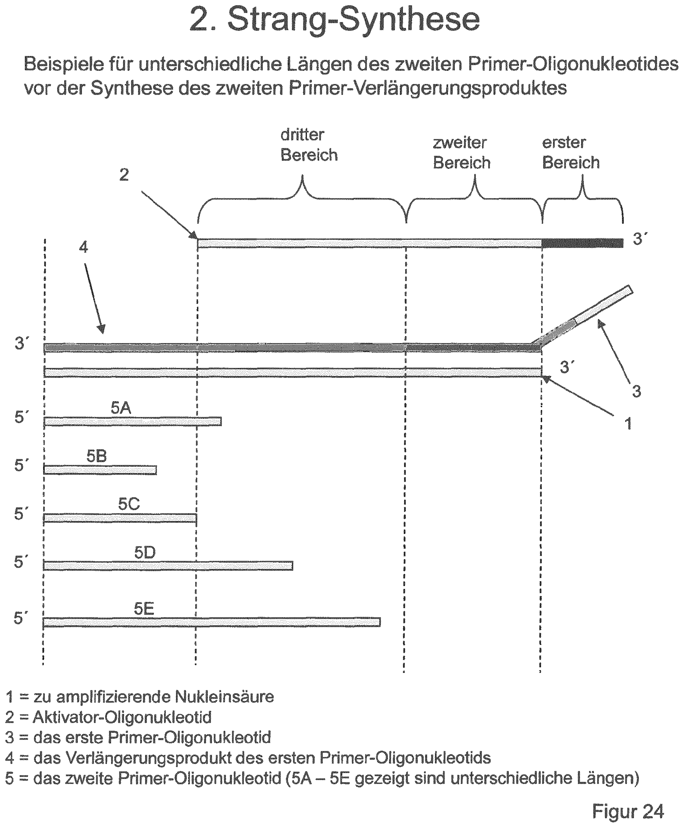

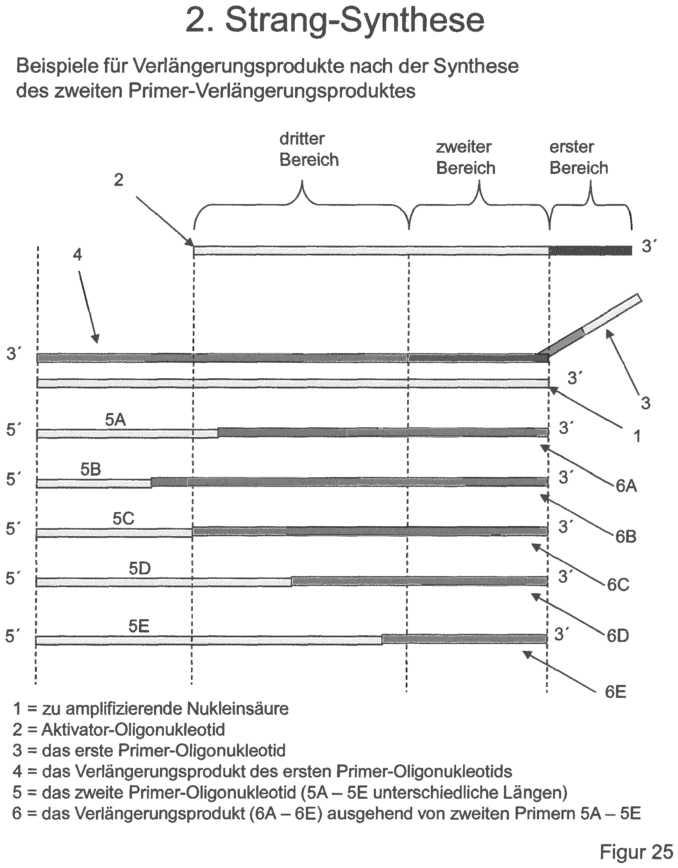

In einer Ausführungsform entspricht das zweite Primer-Oligonukleotid mit seinen kopierbaren Anteilen in ihrer Länge dem 3'-Segment des ersten Primer-Verlängerungsproduktes, welches nicht vom Aktivator-Oligonukleotid gebunden wird (Fig. 24, Primer 5C). Im Komplex umfassend das zweite Primer-Oligonukleotid und das erste Primer-Verlängerungsprodukt grenzt das 3'-Ende eines solchen zweiten Primer-Oligonukleotides an das Aktivator-Oligonukleotid, welches an das erste Primer-Verlängerungsprodukt gebunden ist. Verlängerung eines solchen Primers erfolgt unter Verwendung des ersten Primer-Verlängerungproduktes als Matrize. Bei Verlängerung eines solchen Primers erfolgt die Verdrängung des Aktivator-Oligonukleotides aus der Bindung mit dem ersten Primer-Verlängerungsprodukt mittes polymese-abhängigen Strangverdrängung. Das entsprechende zweite Primer-Verlängerungsprodukt in Fig. 25, abgebildet (Primer-Verlängerungsprodukt 6C).

In einer weiteren Ausführungsform ist das zweite Primer-Oligonukleotid mit seinen kopierbaren Sequenzanteilen kürzer als das 3'-Segment des ersten Primer-Verlängerungsproduktes, welches nicht vom Aktivator-Oligonukleotid gebunden wird (Fig. 24, Primer 5B). Im Komplex umfassend das zweite Primer-Oligonukleotid und das erste Primer-Verlängerungsprodukt liegt somit zwischen dem 3'-Ende eines solchen Primers und dem an das erste Primer-Verlängerungsprodukt gebundenen Aktivator-Oligonukleotid ein einzelsträngiger Abschnitt des ersten Primer-Verlängerungsproduktes. Verlängerung eines solchen Primers erfolgt unter Verwendung des ersten Primer-Verlängerungproduktes als Matrize. Bei Verlängerung eines solchen Primers erfolgt die Verdrängung des Aktivator-Oligonukleotides aus der Bindung mit dem ersten Primer-Verlängerungsprodukt mittels polymese-abhängigen Strangverdrängung. Das entsprechende zweite Primer-Verlängerungsprodukt in in Fig. 25, abgebildet (Primer-Verlängerungsprodukt 6B).

In einer weiteren Ausführungsform ist das zweite Primer-Oligonukleotid mit seinen kopierbaren Anteilen länger als das 3'-Segment des ersten Primer-Verlängerungsproduktes, welches nicht vom Aktivator-Oligonukleotid gebunden wird (Fig. 24, Primer 5A, 5D, 5E). Im Komplex aus dem zweiten Primer-Oligonukleotid und dem ersten Primer-Verlängerungsprodukt konkurrieren das 3'-Segment des zweiten Primers und das 5'-Segment des Aktivator-Oligonukleotides um die Bindung an das das erste Primer-Verlängerungsprodukt. Die für eine Initiierung der Synthese erforderliche Bindung des 3'-Segments des zweiten Primers an das erste Primer-Verlängerungsprodukt erfolgt unter gleichzeitiger partiellen Verdrängung des 5'-Segmentes des Aktivator-Oligonukleotides.

Nach Initiierung der Synthese durch Polymerase erfolgt die Verlängerung eines solchen Primers unter Verwendung des ersten Primer-Verlängerungproduktes als Matrize. Bei Verlängerung eines solchen Primers erfolgt die Verdrängung des Aktivator-Oligonukleotides aus der Bindung mit dem ersten Primer-Verlängerungsprodukt mittes polymese-abhängigen Strangverdrängung. Das entsprechende zweite Primer-Verlängerungsprodukt in Fig. 25, abgebildet (Primer-Verlängerungsprodukt 6A, 6D, 6E). Die Sequenzlänge des 3'-Segment des zweiten Primer-Oligonukleotides, welches das 5'-Segment des Aktivator-Oligonukleotid verdrängt, kann folgende Bereiche umfassen: 1 bis 50 Nukleotide, besser 3 bis 30 Nukleotide, bevorzugt 5 bis 20 Nukleotide. Verwendung von zweiten Primer-Oligonukleotiden mit einer größeren Länge, welche die Länge des 3'-Segment des ersten Primer-Verlängerungsproduktes übersteigt, ist beispielsweise in einigen Ausführungsformen vorteilhaft. Solche Ausführungsformen umfassen beispielsweise ein erstes Primer-Verlängerungsprodukt mit einem 3'-Segment, welches nicht vom Aktivator-Oligonukleotid gebunden wird, mit einer Länge von 5 bis 40 Nukleotiden, besser von 10 bis 30 Nukleotiden. Besonders bei kürzeren 3'-Segmenten bietet ein längeres zweites Primer-Oligonukleotid eine verbesserte Sequenzspezifität bei Initiierung Synthese.

Die Bindungsstärke des zweiten Primer-Oligonukleotides an seine Primer-Bindungsstelle hängt von der Länge des Primers ab. Im Allgemeinen können längere zweite Primer-Oligonukleotide bei höheren Reaktionstemperaturen eingesetzt werden.

Vorzugsweise sind Sequenzen des ersten, des zweiten Primer-Oligonukleotides und des Aktivator-Oligonukleotides dermassen aneinander angepasst, dass Nebenreaktionen, z.B. Primer-Dimer-Ausbildung, minimiert sind. Zu diesem Zweck sind beispielsweise die Sequenz des ersten und des zweiten Primer-Oligonukleotides aneinander demassen angepasst, daß beide Primer-Oligonukleotide nicht in der Lage sind, eine Amplifikations-Reaktion in Abwesenheit einer passenden Matrize und/oder einer Zielsequenz und/oder einer Start-Nukleinsäurekette zu starten. Das kann beispielsweise dadurch erreicht werden, dass das zweite Primer-Oligonukleotid keine Primer-Bindungsstelle für das erste Primer-Oligonukleotid umfasst und das erste Primer-Oligonukleotid keine Primer-Bindungsstelle für das zweite Primer-Oligonukleotid umfasst. Weiterhin soll vermieden werden, dass die Primer-Sequenzen ausgedenhnte eigen-komplementäre Strukturen (self-complement) umfassen.

Die Synthese des zweiten Primer-Verlängerungsproduktes ist eine Primer-Verlängerungsreaktion und bildet einen Teilschritt in der Amplifikation. Die Reaktionsbedingungen während dieses Schrittes sind entsprechend angepasst. Die Reaktions-Temperatur und die Reaktions-Zeit sind dermassen gewählt, dass die Reaktion erfolgreich stattfinden kann. Die jeweils bevorzugte Temperatur in diesem Schritt hängt von der verwendeten Polymerase ab und der Bindungsstärke des jeweiligen zweiten Primer-Oligonukleotides an seine Primer-Bindungsstelle und umfasst beispielsweise Bereiche von 15°C bis 75°C, besser von 20 bis 65°C, bevorzugt von 25°C bis 65°C. Die Konzentration des zweiten Primer-Oligonukleotides umfasst Bereiche von 0,01 µmol/l bis 50 µmol/l, besser von 0,1 µmol/l bis 20 µmol/l, bevorzugt von 0,1 µmol/l bis 10 µmol/l.

In einer Ausführungsform verlaufen alle Schritte der Amplifikation unter stringenten Bedingungen, welche Ausbildung von unspezifischen Produkten / Nebenprodukten verhindern bzw. verlangsamen. Zu solchen Bedingungen zählen beispielsweise höhere Temperaturen, beispielsweise über 50°C.

Falls mehr als eine spezifische Nukleinsäurekette in einem Ansatz amplifiziert werden müssen, werden in einer Ausführungsform bevorzugt jeweils sequenzspezifische Primer-Oligonukleotide für Amplifikation von entsprechenden jeweiligen Zielsequenzen verwendet.

In einer Ausführungsform verläuft die Synthese des ersten und des zweiten Primer-Verlängerungsproduktes bei gleicher Temperatur. In einer weiteren Ausführungsform verläuft die Synthese des ersten und des zweiten Primer-Verlängerungsproduktes bei unterschiedlichen Temperaturen. In einer weiteren Ausführungsform verläuft die Synthese des zweiten Primer-Verlängerungsproduktes und Strangverdrängung durch Aktivator-Oligonukleotid bei gleicher Temperatur. In einer weiteren Ausführungsform verläuft die Synthese des zweiten Primer-Verlängerungsproduktes und Strangverdrängung durch Aktivator-Oligonukleotid bei unterschiedlichen Temperaturen.

### Exponentielle vs. Lineare Amplifikation

Falls beide komplementäre Stränge (das erste Primer-Verlängerungs-Produkt und das zweite Primer-Verlängerungsprodukt, wobei beide Primer-Verlängerungsprodukte als Matrizen für Synthesen der komplementären Stränge auftreten können) im Wesentlichen parallel zu einander im gleichen Ansatz synthetisiert werden, kann es zu einer exponentiell ablaufenden Vermehrung beider Primer-Verlängerungsprodukte im Rahmen einer solchen Reaktion kommen.

Die während eines Synthese-Vorgangs neu synthetisierten Primer-Verlängerungsprodukte schließen die jeweils komplementäre Sequenzabschnitte zu verwendeten Primern, so dass Primer-Bindungsstellen neu generiert werden. Dadurch können neu synthetisierte Stränge in den darauf folgenden Synthese-Vorgängen ihrerseits als Matrizen dienen.

Falls im Wesentlichen nur ein Primer-Verlängerungs-Produkt als Folge zyklisch wiederholten Synthese-Vorgänge synthetisiert wird, so kommt es zu einer linear ablaufenden Amplifikation des besagten Primer-Verlängerungsproduktes.
In einer vorteilhaften Ausführungsform des Verfahrens werden beide Primer im Wesentlichen in gleich hohen Konzentrationen, oder in Konzentrationsbereichen, welche in etwas gleich hoch sind, eingesetzt.

In einer weiteren vorteilhaften Ausführungsform des Verfahrens wird mindestens eines der beiden Primer in einer höheren Konzentration eingesetzt, als sein Partnerprimer. Die Konzentrationsunterschiede können dabei in Bereichen liegen, welche zwischen 1:2 bis 1:50, vorteilhafter zwischen 1:2 bis 1:10 liegen.

Dabei kann eine asymmetrische Amplifikations-Reaktion resultieren, bei welcher Konzentration eines Primer-Verlängerungsproduktes entsprechend höher liegt als die des anderen Stranges.

Die im Folgenden aufgeführten Beispiele sollen lediglich zu Demonstrationszwecken des Verfahrens angeführt werden und nicht als Einschränkung verstanden werden.

Die in den Beispielen angeführten Strukturen, Sequenzen und Reaktiosbedingungen sollten lediglich Funktionsweise des Verfahrens darstellen und anschaulich machen, und dienen nicht als Einschränkung.

### Beispiele:

### Material und Methoden:

Reagenzien wurden von folgenden kommerziellen Anbietern bezogen:
- nicht modifizierte und modifizierte Oligonukleotide (Eurofins MWG, Eurogentec, Biomers, Trilink Technologies, IBA Solutions for Life Sciences)
- Polymerasen NEB (New England Biolabs)
- dNTP's: Jena Bioscience
- interkallierender EvaGreen Farbstoff: Jena Bioscience
- Puffer-Substanzen und andere Chemikalien: Sigma-Aldrich
- Plastikware: Sarstedt

Lösung 1 (Amplifikationsreaktion Lösung 1) :
   - Kalium Glutamat, 50 mmol/l, pH 8,0
   - Magnesium Acetat, 10 mmol/l
   - dNTP (dATP, dCTP, dTTP, dGTP), je 200 µmol/l
   - Polymerase (Bst 2.0 WarmStart, 120.000 U/ml NEB), 12 Units / 10 µl
   - Triton X-100, 0,1% (v/v)
   - EDTA, 0,1 mmol/l
   - TPAC (Tetrapropylammonium Chloride), 50 mmol/l, pH 8,0
   - EvaGreen Farbstoff (Farbstoff wurde entsprechend Anleitung des Herstellers in Verdünnung 1:50 eingesetzt).
Lösung 2 (Amplifikationsreaktion Lösung 2) :
   - 1x Isothermal Puffer (New England Biolabs); in einfacher Konzentration enthält der Puffer:
      20 mM Tris-HCl
      10 mM (NH4)2SO4
      50 mM KCl
      2 mM MgSO4
      0.1% Tween® 20
      pH 8.8@25°C)
   - dNTP (dATP, dCTP, dUTP, dGTP), je 200 µmol/l
   - EvaGreen Farbstoff (Farbstoff wurde entsprechend Anleitung des Herstellers in Verdünnung 1:50 eingesetzt)
Lösung 3 (Amplifikationsreaktion Lösung 3) :
   - 1x Isothermal Puffer (New England Biolabs); in einfacher Konzentration enthält der Puffer:
      20 mM Tris-HCI
      10 mM (NH4)2SO4
      50 mM KCl
      2 mM MgSO4
      0.1% Tween® 20
      pH 8.8@25°C)
   - dNTP (dATP, dCTP, dUTP, dGTP), je 200 µmol/l

Alle Konzentrationen sind Angaben der Endkonzentrationen in der Reaktion. Abweichungen von der Standard-Reaktion werden entsprechend angegeben.

Die Schmelztemperatur (Tm) von beteiligten Komponenten wurde bei Konzentration von 1 µmol/l von jeweiligen Komponenten in Lösung 1 bestimmt. Abweichende Parameter sind jeweils angegeben.

### Allgemeine Informationen zu Reaktionen

Primer-Verlängerungsreaktionen und Amplifikation wurden standardmäßig bei einer Reaktionstemperatur von 65°C durchgeführt. Abweichungen werden angegeben.

Der Reaktionsstart erfolgte durch Erhitzen der Reaktionslösungen auf Reaktionstemperatur, da Bst 2.0 Polymerase Warmstart bei niedrigeren Temperauren größtenteils in ihrer Funktion durch ein temperatur-sensitives Oligonukleotid gehemmt ist (laut Hersteller-Angaben). Die Polymerase wird zunehmend aktiver ab einer Temperatur von ca. 45°C, bei einer Temperatur von 65°C konnten keine Unterschiede zwischen Polymerase Bst 2.0 und Bst 2.0 Warmstart festgestellt werden. Um der extensiven Bildung von Nebenprodukten (z.B. Primer-Dimeren) während der Vorbereitungsphase einer Reaktion vorzubeugen, wurde Polymerase Bst 2.0 Warmstart verwendet. Abweichungen davon werden speziell angegeben.

Reaktionsstopp erfolgte durch Erhitzen der Reaktionslösung auf über 80°C, z.B. 10 min bei 95°C. Bei dieser Temperatur wird Polymerase Bst 2.0 irreversible denaturiert und das Ergebnis der Synthese-Reaktion kann nachträglich nicht geändert werden.

Reaktionen wurden in einem Thermostat mit einer Fluoreszenz-Messvorrichtung ausgeführt. Zu diesem Zweck wurde ein kommerzielles Real-Time PCR Gerät verwendet, StepOne Plus (Applied Biosystems, Thermofischer). Das Reaktionsvolumen betrug standardmäßig 10 µl. Abweichungen davon werden angegeben.

Sowohl Endpunkt-Bestimmung als auch kinetische Beobachtungen wurden vorgenommen. Bei Endpunktbestimmungen wurde das Signal beispielsweise von an Nukleinsäuren gebundenen Farbstoffen registriert, z.B. von TMR (Tetramethyl-Rhodamine, auch TAMRA genannt) oder von FAM (Fluorescein). Die Wellenlängen zur Anregung und Messung der Fluoreszenzsignale von FAM und TMR sind als Werkeinstellungen bei StepOne Plus Real-Time PCR Gerät gespeichert. Ebenfalls wurde ein interkallierender Farbstoff (EvaGreen) bei Endpunktmessungen vorgenommen, z.B. bei Messung einer Schmelzkurve). EvaGreen ist ein interkallierender Farbstoff und ist ein Analogon des häufig eingesetzten Farbstoff Sybrgreen, allerdings mit etwas geringerer Inhibierung von Polymerasen. Die Wellenlängen zur Anregung und Messung der Fluoreszenzsignale von SybreGreen und EvaGreen sind identisch und sind als Werkeinstellungen bei StepOne Plus Real-Time PCR Gerät gespeichert. Die Fluoreszenz kann mittels eingebauter Detektoren fortlaufend, d.h. "online" oder "Real-Time" detektiert werden. Da die Polymerase während ihrer Synthese einen Doppelstrang synthetisiert, konnte diese Technik zu kinetischen Messungen (Real-Time Monitoring) der Reaktion verwendet werden. Aufgrund von einem gewissen Cross-Talk zwischen Farb-Kanälen bei StepOne Plus Gerät wurde teilweise erhöhte basale Signal-Intensität bei Messungen beobachtet, bei welchen z.B. TMR-markierte Primer in Konzentration von über 1 µmol/l (z.B. 10 µmol/l) eingesetzt waren. Es wurde beobachtet, dass TMR-Signal in Sybre-Green-Kanal zu erhöhten Grundwerten führt. Diese erhöhten Grundwerte wurden bei Berechnungen berücksichtigt.

Die kinetischen Beobachtungen von Reaktionsverläufen wurden routinemäßig mittels Fluoreszenzsignalen von Fluoreszein (FAM-TAMRA Fret-Paar) bzw. interkallierenden Farbstoffen (EvaGreen) aufgenommen. Zeitabhängigkeit des Signal-Verlaufs wurde registriert (Real-Time Signal Erfassung bei StepOne plus PCR Gerät). Ein Anstieg des Signals während einer Reaktion verglichen mit einer Kontroll-Reaktion wurde je nach Aufbau des Ansatzes interpretiert. Beispielsweise, eine Zunahme des Signals bei Verwendung von Evagreen-Farbstoff wurde als Hinweise auf Zunahme der Menge an doppelsträngigen Nukleinsäureketten während der Reaktion interpretiert, und somit als Ergebnis einer stattfindenden Synthese durch DNA-Polymerase gewertet.

Bei einigen Reaktionen wurde im Anschluss an die Reaktion eine Schmelzkurvenbestimmung durchgeführt. Solche Messungen erlauben Rückschlüsse auf das Vorliegen von Doppelsträngen, welche beispielsweise interkallierende Farbstoffe aufnehmen können und dadurch die Signalintensität von Farbstoffen deutlich verstärken. Mit der steigenden Temperatur sinkt der Anteil von Doppelsträngen und die Signal-Intensität sinkt ebenfalls. Das Signal ist von der Länge von Nukleinsäureketten und von der Sequenzzusammensetzung abhängig. Diese Technik ist einem Fachmann hinreichend bekannt.

Bei Verwendung der Schmelzkurven-Analyse im Zusammenhang mit Reaktionen, welche signifikante Anteile von modifizierten Nukleinsäureketten (z.B. Aktivator-Oligonukleotide oder Primer) enthielten, wurde festgestellt, dass sich das Signal von Farbstoff EvaGreen beispielsweise unterschiedlich zwischen B-Form der DNA und A-Form von modifizierten Nukleinsäureketten verhalten kann. Beispielsweise wurde bei B-Form der doppelsträngigen Nukleinsäureketten (gewöhnlich angenommen für klassische DNA-Abschnitte) eine höhere Signal-Intensität beobachtet, als bei doppelsträngigen Nukleinsäureketten mit der gleichen Sequenz von Nukleobasen, welche eine A-Form ähnliche Konformation annehmen können (z.B. durch mehrere 2'-O-Me Modifikationen von Nukleotiden). Diese Beobachtung wurde beim Einsatz von interkallierenden Farbstoffen berücksichtigt.

Bei Bedarf wurde die Reaktion mittels Kapillarelektrophorese analysiert und die Länge von gebildeten Fragmenten mit einem Standard verglichen. Als Vorbereitung auf die Kapillarelektrophorese wurde die Reaktionsmischung in einem Puffer (Tris-HCI, 20 mmol/l, pH 8,0, und EDTA, 20 mmol/l, pH 8,0) dermaßen verdünnt, dass die Konzentration von markierten Nukleinsäuren ca. 20 nmol/l betrug. Die Kapillar-Elektrophorese wurde bei Firma GATC-Biotech (Konstanz, Deutschland) als Auftragsleistung durchgeführt. Nach Angaben des Anbieters wurde die Kapillarelektrophorese auf einem ABI 3730 Cappilary Sequencer unter Standard-Bedingungen für eine Sanger-Sequenzierung unter Verwendung von POP7 Gelmatrix, bei ca. 50°C durchgeführt und konstanter Spannung (ca. 10 kV) durchgeführt. Die verwendeten Bedingungen führten zu Denaturierung von Doppelsträngen, so dass in der Kapillarelektrophorese die einzelsträngige Form von Nukleinsäureketten separiert wurde. Die Elektrophorese ist eine Standardtechnik in der genetischen Analyse. Die automatisierte Kapillar-Elektrophorese wird heute routinemäßig bei Sanger-Sequenzierung eingesetzt. Das Fluoreszenz-Signal wird während der Kapillar-Elektrophorese kontinuierlich aufgezeichnet (gewöhnlich unter Verwendung von virtuellen Filtern), so dass ein Elektrophorogramm entsteht, bei welchem die Signal-Intensität mit der Dauer der Elektrophorese korreliert. Bei kürzeren Fragmenten, z.B. nicht verbrauchte Primer, beobachtet man einen frühren Signal-Peak, bei verlängerten Fragmenten kommt es zu einer zeitlichen Verschiebung der Signale proportional der Länge des extendierten Bereichs. Dank mitgeführten Kontrollen mit bekannten Längen lässt sich die Länge von extendierten Fragmenten ausmessen. Diese Technik ist einem Fachmann bekannt und wird ebenfalls standardmäßig bei Fragment-Längen-Polymorphismus eingesetzt.

### Beispiel 1

### Verwendung humaner genomischer DNA als Quelle von Zielsequenz

In diesem Beispiel wird Verwendung humaner genomischer DNA (hgDNA) als Quelle einer Zielsequenz gezeigt. Als Zielsequenz wurde ein Sequenzsegment des Faktor-V-Leiden Genes (Homo sapiens coagulation factor V (F5), mRNA, hier als FVL-Gen bezeichnet) gewählt.

### Zielsequenz:

5' GTAA GAGCAGATCC CTGGACAGGC AA GGAATACAGGTA - 3' (SEQ ID NO:1) Die Bindungssequenz für das erste Primer-Oligonukleotid ist unterstrichen. Das zweite Primer-Oligonukleotid bindet mit seinem 3'-Segment an das Reverse-Complement der doppelt unterstrichen Sequenz.

Der erste Primer, der zweite Primer, sowie Aktivator-Oligonukletid wurden für FVL-Mutation Variante des Genes designet und synthetisiert.

Das erste Primer-Oligonukleotid (SEQ ID NO:2):
P1 F5-200-AE2053

Der als Primer in der Reaktion verwendetes Segment ist unterstrichen.
A = 2'-deoxy-Adenosin
C = 2'-deoxy-Cytosin
G = 2'-deoxy-Guanosin
T = 2'-deoxy-Thymidin (Thymidin)

Dieses Oligonukleotid umfasst folgende Modifikationen:
1= C3-Linker
5 = 2'-O-Me A (2'-O-Methyl-Adenosine)
6 = 2'-O-Me G (2'-O-Methyl-Guanosine)
7 = 2'-O-Me C (2'-O-Methyl-Cytosine)
8 = 2'-O-Me U (2'-O-Methyl-Uridine)

### Primer 2:

P2G3-5270-7063

Der als Primer in der Reaktion verwendetes Segment ist unterstrichen.

Dieses Oligonukleotid umfasst folgende Modifikationen:
6 = HEG-Linker

Folgendes Aktivator-Oligonukleotid (SEQ ID NO:4) wurde verwendet:
AD-F5-1001-503

Dieses Oligonukleotid umfasst folgende Modifikaton: A = 2'-deoxy-Adenosin
C = 2'-deoxy-Cytosin
G = 2'-deoxy-Guanosin
T = 2'-deoxy-Thymidin (Thymidin)

Das 5'-Segment des Oligonukleotids [UAAUCUGUAA GAGCAGAUCC CUGGACAGGC AA GGAAUAC] umfasste 2'-O-Me-Nukleotid-Modifikationen:

### Modifikationen:

A = (2'-O-Methyl-Adenosine)
G = (2'-O-Methyl-Guanosine)
C = (2'-O-Methyl-Cytosine)
U = (2'-O-Methyl-Uridine)
X = 3'-Phosphat-Gruppe zur Blockade einer möglichen Verlängerung durch Polymerase.

Die Nukleotide und Nukleotid-Modifikationen sind untereinander mit Phosphodiesterbindungen verknüpft. Das 3'-Ende des Aktivator-Oligonukleotdies ist mit einer Phosphat-Gruppe blockiert, um eine mögliche Verlängerung durch die Polymerase zu verhindern.

Der erste Primer umfasst in seinem ersten Bereich eine Sequenz, welche spezifisch an die Sequenz des Faktor-V-Leiden Genes innerhalb der genomichen DNA binden kann, so dass eine Synthese durch eine Polymerase gestartet werden kann. Der zweite Bereich des ersten Primers umfasst eine Sequenz, welche nicht an die Sequenz von FVL-Genes spezifisch hybridisiert. Weiterhin umfasst der erste Primer ein weiteres Sequenzsegment, welches an das 5'-Ende des zweiten Bereichs anknüpft. Dieses Segment nimmt an der spezifischen Amplifikation des Faktor-5-Leiden Segments nicht teil. Die Funktion dieses Segments wird vor allem in Verzögerung von Nebenreaktionen gesehen.

Der zweite Primer umfasst ein Segment in seinem 3'-Segment, welches spezifisch an die genomiche DNA binden kann, so dass eine Synthese durch eine Polymerase gestartet werden kann. Das 5'-Segment des zweiten Primers umfasst eine Sequenz, welche nicht an die Sequenz von FVL-Genes spezifisch hybridisiert. Während der Rücksynthese kann dieses Sequenzsegment kopiert werden. Der zweite Primer umfasst ein weiteres Sequenzsegment, welches weder mit dem Aktivator-Oligonukleotid, noch mit dem erten Primer, noch mit dem zweiten Primer spezifisch hybridisieren kann. Dieses Segment wurde an das 5'-Ende des zweiten Primer lokalisiert und vom 5'-Ende des Primers durch eine HEG-Linker getrennt ist. Dieses Segment nimmt nicht an der spezifischen Amplifikation teil. Die Funktion dieses Segments wird vor allem Verzögerung von Nebenreaktionen gesehen.

Aktivator-Oligonukleotid wurde dermassen konstruiert, dass eine Perfekt-Match-Situation zur Sequenz der Faktor-V-Leiden Mutation des FVL-Gens resultiert. Das Aktivator-Oligonukleotid umfasst einen ersten, zweiten und dritten Bereich.

Als genomische DNA wurde WHO-Standard für FVL-Mutation verwendet. Vor der Verwendung in der Reaktion wurde DNA durch Erhitzung denaturiert (5 min bei 95°C) und damit aus dem doppelsträngigen Zustand in einzelsträngigen Zustand überführt. Anhang dieser einzelsträngiger hgDNA wurde zunächst eine Start-Nukleinsäurekette durch eine Primer-Verlängerng erstellt. Anschließend erfolgte eine exponentielle Amplifikation ausgehend von dieser Start-Nukleinsäurekette. Der Nachweis der Spezifität der Amplifikation erfolgte mittels Schmelzkurven-Analyse und Sanger-Sequenzierung mit einem Sequenzierungprimer.

Alle Reaktionen wurden in Amplifikations-Lösung 1 durchgeführt. Die verwendeten dNTPs umfassten: dATP, dGTP, dCTP, dUTP (anstatt von dTTP). Als Polymerase wurde Bst 2.0 Warm-Start Polymerase von NEB verwendet.

Die Start-Nukleinsäurekette wurde wie folgt erstellt:
Etwa 50000 haploider genom Äquivalente (HGE), 150 ng hgDNA, wurden in Kontakt mit dem zweiten Primer (0,5 µmol/l) und Bst-2.0 Warm Start Polymerase (etwa 1 unit), sowie dNTPs (ca. 250 µmol/l) unter Hybridierungsbedingungen (Amplifikationslösung 1, Temperatur von etwa 60°C) in 50 µl Reaktionsvolumen gebracht und ca. 10 min inkubiert. Während dieser Phase erfolgt eine Verlängerung des zweiten Primers, wobei die genomische DNA als Matrize dient. Es resultiert ein Primer-Verlängerungsprodukt, welches als Start-Nukleinsäure dienen kann. Nach Abschluss dieser Reaktion wurde Reaktionsgemisch erhitzt auf 95°C für ca. 10 min, um diese Start-Nukleinsäurekette Matrize zu trennen. Dieses Reaktionsgemisch wurde eingefroren und als Quelle der Start-Nukleinsäurekette nach Bedarf verwendet.

Die spezifische Amplifikation der Zielsequenz des FVL-Gens erfolgte unter Verwendung von 5 µl des Reaktionsgemisches mit der Start-Nukleinsäurekette (entspricht ca. 5000 HGE).

Die anderen Reaktionskomponenten (erster Primer, zweiter Primer, Aktivator-Oligonukleotid, Eva-Green Farbstoff, Polymerase Bst.2.0 Warm Start, dNTPs) wurden dazugegeben, so dass ein Reaktionsendvolumen von ca. 10 µl resultierte. Die Endkonzentrationen der Komponenten betrugen: erster Primer: 5µmol/l, zweiter Primer: 2 µmol/l, Aktivator-Oligonukleotid: 1 µmol/l, Eva-Green Farbstoff (1:50), Polymerase Bst.2.0 Warm Start (ca. 8 Units), dNTPs: ca. 250 µmol/l.

Im Kontroll-Ansatz wurde keine hgDNA zugegeben.

Die Reaktion wurde in einem Step-One Plus Gerät (Thermofisher Scientific) durchgeführt.

Die Reaktionstemperatur wurde initial durch zyklische Änderungen (30 Zyklen) geändert zwischen 65°C (5 min, einschließlich Detektionsschritt) und 55°C (1 min) und anschließend für 1 hr bei 65°C konstant gehalten (Detektionsschritt alle 2 min). Der Verlauf der Reaktion wurde durch Signal-Erfassung des EvaGreen Farbstoffs verfolgt. Nach Abschluss der Reaktion wurde das Reaktionsgemisch zunächst auf 95°C für 10 min gebracht und anschließend wurde eine Schmelzkurve der gebildeten Produkte gemessen.

Ein schematischer Ablauf der Amplifikation ist in Fig. 7 bis Fig. 10 dargestellt.

Zunächst erfolgte eine Erstellung einer Start-Nukleinsäurekette (Fig.7, Schritte A bis D). Anschließend erfolgte eine exponentielle Amplifikation unter Verlängerung des ersten Primers und des zweiten Primers und des Aktivator-Oligonukleotids (Fig. 7E bis Fig. 10).

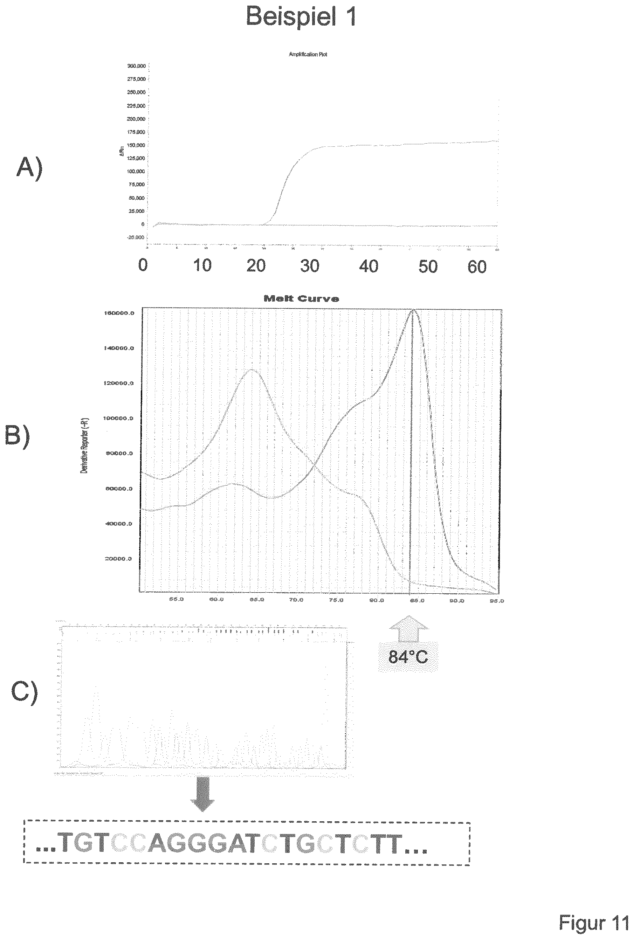

Als Ergebnis der Amplifikation kommt es zu Akkumulierung von Amplifikationsfragmenten. Das Ergebnis der Detektion der Amplifikation ist in Fig. 11 zu sehen. Man sieht, dass ca. nach 2 hr der Reaktion eine sichtbare Zunahme der Fluoreszenz erfolgt (Fig. 11 A). Anschließende Schmelzkurven-Analyse zeigte eine spezifische Schmelzkurve mit Tm von 84°C (Fig. 11 B).

### Sequenzüberprüfung (Fig.11C):

Die Sequenzüberprüfung erfolgte mittels eines Sequenzierungsprimers:
SEQP1 F5-35-X03

Zur Sequenzüberprüfung wurde das Reaktionsgemisch (nach Messung der Schmelzkurve) mit Wasser verdünnt (von ca. 1:10 bis ca. 1:100) und jeweils erhaltenen Aliquoten mit einem Sequenzierungsprimer (zugegeben in Konzentration von 2 µmol/l) gemischt. Dieses Gemisch wurde durch einen kommerziellen Sequenzierungs-Anbieter versendet (GATC-Biotec) und mittels Sanger-Sequenzierung als Auftrags-Sequenzierung sequenziert. Die erhaltenen Elektropherogramme wurden auf Übereinstimmung mit der FVL-Sequenz Gens geprüft. Als Ergebnis der Reaktion wurde Sequenz des FVL-Gens identifiziert.

### Beispiel 2

### Real-Time Monitoring der Amplifikations-Reaktion mit einer Oligonukleotid-Sonde

In diesem Ausführungsbeispiel werden Oligonukleotid-Sonden zur Detektion von synthetisierten Amplifikationsprodukten während Amplifikationsreaktion eingesetzt.

Folgende Matrize wurde als Start-Nukleinsäurekette verwendet:
Matrize (SEQ ID NO 1) mit Sequenzzusammensetzung, welche zu einem ersten Primer-Verlängerungsprodukt mit einer Perfekt-Match-Übereinstimmung mit dem Aktivator-Oligonukleotid führt:
M2SF5-M001-200

Die Bindungssequenz für das erste Primer-Oligonukleotid ist unterstrichen. Das zweite Primer-Oligonukleotid bindet an das Reverse-Complement der doppelt unterstrichenen Sequenz.

Folgende Primer wurden verwendet:
Das erste Primer-Oligonukleotid (SEQ ID NO 2):
P1 F5-200-AE2053 (wie im Beispiel 1)

Das als Primer in der Reaktion verwendete Segment ist unterstrichen.
A = 2'-deoxy-Adenosin
C = 2'-deoxy-Cytosin G = 2'-deoxy-Guanosin
T = 2'-deoxy-Thymidin (Thymidin)

Dieses Oligonukleotid umfasst folgende Modifikationen:
1= C3-Linker
5 = 2'-O-Me A (2'-O-Methyl-Adenosine)
6 = 2'-O-Me G (2'-O-Methyl-Guanosine)
7 = 2'-O-Me C (2'-O-Methyl-Cytosine)
8 = 2'-O-Me U (2'-O-Methyl-Uridine)

### Primer 2:

P2G3-5270-7063 (wie im Beispiel 1)

Das als Primer in der Reaktion verwendete Segment ist unterstrichen.

Dieses Oligonukleotid umfasst folgende Modifikationen:
6 = HEG-Linker

Folgendes Aktivator-Oligonukleotid (SEQ ID NO 4) wurde verwendet:
AD-F5-1001-503 (wie im Beispiel 1)

Dieses Oligonukleotid umfasst folgende Modifikation: A = 2'-deoxy-Adenosin
C = 2'-deoxy-Cytosin
G = 2'-deoxy-Guanosin
T = 2'-deoxy-Thymidin (Thymidin)

Das 5'-Segment des Oligonukleotids [UAAUCUGUAA GAGCAGAUCC CUGGACAGGC AA GGAAUAC] umfasste 2'-O-Me-Nukleotid-Modifikationen:

### Modifikationen:

A = (2'-O-Methyl-Adenosine)
G = (2'-O-Methyl-Guanosine)
C = (2'-O-Methyl-Cytosine)
U = (2'-O-Methyl-Uridine)
X = 3'-Phosphat-Gruppe zur Blockade einer möglichen Verlängerung durch Polymerase.

Die Nukleotide und Nukleotid-Modifikationen sind untereinander mit Phosphodiesterbindungen verknüpft. Das 3'-Ende des Aktivator-Oligonukleotdies ist mit einer Phosphat-Gruppe blockiert, um eine mögliche Verlängerung durch die Polymerase zu verhindern.

Als Oligonukleotid-Sonde wurde folgendes Oligonukleotid eingesetzt (SEQ ID NO 10):
P2D-Lux-1000-101 5'- FAM-TAGTATGAGCTTTT 1 ***GCTCATAC 2 ACAATGTCACTTACTGTAAGAGCAGA*** 3' (SEQ ID NO:10)

### Modifikationen:

am 5' Ende mit FAM markiertes Oligonukleotid
1 = HEG-Linker
2 = dT-BHQ1

Ein schematischer Aufbau der Oligonukleotid-Sonde ist Fig. 3B dargestellt (S4.1). Selbstkomplementäre Segmente der Oligonukleotid-Probe sind unterstrichen (Stem-Segment 1 und Stem-Segment 2). Die Positionen werden vom 5'-Ende des Oligonukleotides angegeben:
Stem-Segment 2 (Positionen 1-10), Stem-Segment 1 (Positionen 15-25). Das zwischen beiden Stem-Segmenten liegende Loop-Segment 3 (11-14) umfasst einen HEG-Linker. Die Oligonukleotid-Sonde umfasst weiterhin ein Segment-4 (Position 26-51), welches mit dem Stem-Segment 1 einen Nukleinsäurestrang bildet. Stem-Segment 1 und Segment 4 sind in der Lage, an das 3'-Segment des während der Reaktion gebildeten ersten Primer-Verlängerungsproduktes komplementär zu binden. Weiterhin kann das 3'-Ende der gebundenen Oligonukleotid sonde als Primer in der Reaktion von Polymerase verlängert werden.

Fluoreszenzreporter (FAM) ist an das 5'-Ende des Oligonukleotides gebunden. Quencher (dT-BHQ1) ist in der Position 23 positioniert. Im nicht an eine komplementäre Sequenz gebundenen Zustand können selbstkomplementäre Bereiche des Oligonukleotides aneinander binden. Die Schmelztemperatur beträgt ca. 70°C (gemessen in ReaktionsLösung 2 bei 0,5 µmol/l Sonde). Damit werden Fluoreszenzreporter und Quencher in räumliche Nähe gebracht, so dass Fluoreszenzsignal vom Reporter vermindert wird. Die Reduktion des Fluoreszenzsignals betrug ca. 80 % des FAM-Signals.

Bei einer komplementären Bindung an das 3'-Segment des Primer-Verlängerungsproduktes erfolgt Ausbildung des Doppelstranges zwischen dem 3'-Segment des ersten Primer-Verlängerungsproduktes und Positionen 15-51 der Sonde, was zu Hinderung der Bindung von selbstkomplementären Segmenten führt. Der Reporter und der Quencher werden somit von einander entfernt und die Intensität des Fluoreszenzsignals nimmt zu. Daraus wurde angenommen, dass die Intensität des Fluoreszenzsignals mit steigendem Ausmaß der Bindung der Sonde an das 3'-Segment ebenfalls ansteigen soll.

Die Matrize wurde in Konzentrationen von 1 pmol/l, 1 fmol/l. In der Kontroll-Reaktion wurde keine Matrize eingesetzt (= Negativkontrolle). Primer 1 wurde mit 5 µmol/l, das Aktivator-Oligonukleotid mit 2 µmol/l und Primer 2 mit 0,5 µmol/l und die Oligonukleotid-Sonde ebenfalls mit 0,5 µmol/l eingesetzt.

Die Reaktionen wurden mit der Reaktionslösung 2 oder 3 durchgeführt, je nach dem ob die Oligonukleotid-Sonde oder Eva-Green (interakallierender Farbstoff) in der Reaktion das Signal generiert. Die Reaktion wurde entweder mit einer Oligonukleotid-Sonde ohne EvaGreen durchgeführt oder ohne Oligonukleotid-Sonde aber mit EvaGreen.

Die thermischen Reaktionsbedingungen waren zyklisch, alternierende Temperaturänderungen, wobei jeweils auf ein 2 min Zeitintervall bei 55 °C ein 2 min Zeitintervall bei 65 °C folgte. Die Amplifikation wurde über 150 Zyklen verfolgt. Detektion erfolgte bei 65°C sowohl für EvaGreen-Fluoreszenzsignal als auch bei FAM-Signal.

Die erfolgreiche Amplifikation konnte durch einen Anstieg des FAM-Fluoreszenzsignals oder EvaGreen Signals im Laufe der Zeit festgestellt werden.

Die Temperatur-Änderungen und Real-Time Monitoring wurde mit StepPne Plus Real-Time PCR Gerät von Thermofisher durchgeführt.

### Analyse der Amplifikationsreaktion

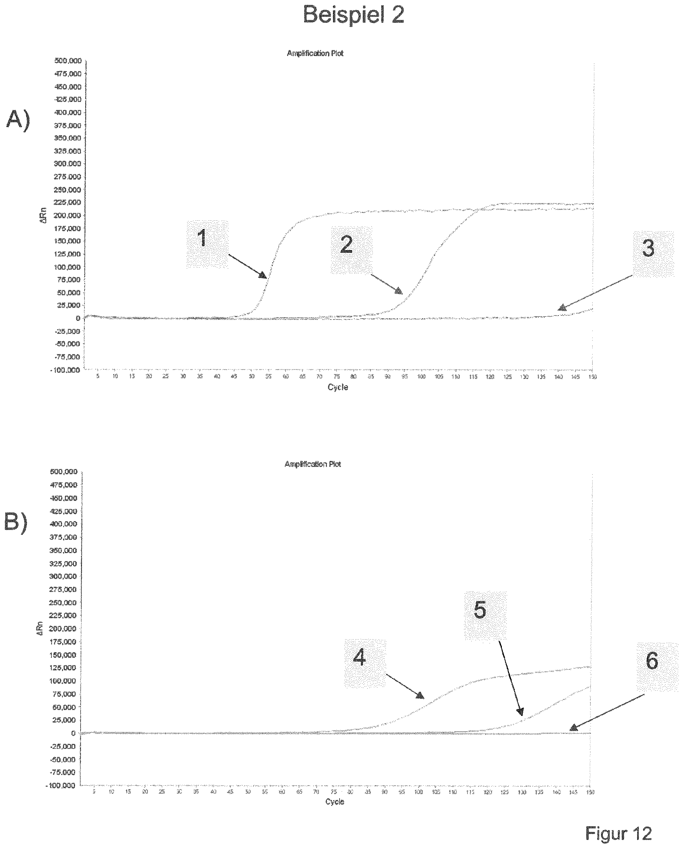

Fig. 12 A zeigt einen typischen Verlauf des EvaGreen-Signals. Fig. 12 B zeigt das FAM-Fluoreszenzsignals im Laufe der Amplifikationsreaktion. Auf der Y-Achse ist die Zunahme des Fluoreszenzsignal (Delta Rn) und auf der X-Achse ist die Reaktionszeit (als Zyklusnummer) aufgetragen. Pfeile markieren einzelne Reaktionsansätze. Dabei gehören die markierten Positionen zu folgenden Ansätzen:
Pfeil 1: 1 pmol/l Matrize
Pfeil 2: 1 fmol/l Matrize
Pfeil 3: keine Matrize
Pfeil 4: 1 pmol/l Matrize
Pfeil 5: 1 fmol/l Matrize
Pfeil 6: keine Matrize

Man sieht den Anstieg des Fluoreszenz-Signals sowohl bei EvaGreen als auch bei Sonde, wobei das Signal der Sonde zeitlich später erscheint. Die Zykluszahl bei der ein Fluoreszenzanstieg erreicht wird, korreliert dabei mit der Menge an eingesetzter Matrize.

### Beispiel 3:

### Spezifität der Amplifikations-Reaktion

In diesem Beispiel wurde Einfluss einer Sequenzänderung in der Matrize auf die Amplifikation untersucht. Bei Verlängerung des ersten Primer-Oligonukleotides wird ein komplementärer Strang gebildet, welcher eine komplementäre Sequenz zur Matrize aufweist und somit diese Abweichungen in der Sequenz umfasst. Auf diese Weise sollte überprüft werden, welche Auswirkung ein solcher Mismatch zwischen einem dabei entstehenden ersten Primer-Verlängerungsprodukt und einem Aktivator-Oligonukleotid auf die Amplifikation hat. Die Position des Mismatches liegt in 3'-Richtung vom ersten Primer und wird somit nicht vom Primer, sonder durch das Aktivator-Oligonukleotid überprüft.

Folgende Matrizen wurden verwendet:
Matrize (SEQ ID NO 1) mit Sequenzzusammensetzung, welche zu einem ersten Primer-Verlängerungsprodukt mit einem Perfekt-Match Übereinstimmung mit Aktivator-Oligonukleotid führt:
M2SF5-M001-200 (wie im Beispiel 2)

Die Bindungssequenz für das erste Primer-Oligonukleotid ist unterstrichen. Das zweite Primer-Oligonukleotid bindet an das Reverse-Complement der doppelt unterstrichen Sequenz.

Matrize (SEQ ID NO 1) mit Sequenzzusammensetzung, welche zu einem ersten Primer-Verlängerungsprodukt führt, das an einer einzelnen Basenposition (fett gedruckt) ein Mismatch mit dem Aktivator-Oligonukleotid bildet:
M2SF5-WT01-200

Die Bindungssequenz für das erste Primer-Oligonukleotid ist unterstrichen. Das zweite Primer-Oligonukleotid bindet an das Reverse-Complement der doppelt unterstrichenen Sequenz.

Folgende Primer wurden verwendet:

Das erste Primer-Oligonukleotid (SEQ ID NO 2):
P1F5-200-AE2053 (wie im Beispiel 1)

Der als Primer in der Reaktion verwendetes Segment ist unterstrichen.
A = 2'-deoxy-Adenosin
C = 2'-deoxy-Cytosin
G = 2'-deoxy-Guanosin
T = 2'-deoxy-Thymidin (Thymidin)

Dieses Oligonukleotid umfasst folgende Modifikationen:
1= C3-Linker
5 = 2'-O-Me A (2'-O-Methyl-Adenosine)
6 = 2'-O-Me G (2'-O-Methyl-Guanosine)
7 = 2'-O-Me C (2'-O-Methyl-Cytosine)
8 = 2'-O-Me U (2'-O-Methyl-Uridine)

### Primer 2:

P2G3-5270-7063 (wie im Beispiel 1)

Der als Primer in der Reaktion verwendetes Segment ist unterstrichen.

Dieses Oligonukleotid umfasst folgende Modifikationen:
6 = HEG-Linker

Folgendes Aktivator-Oligonukleotid (SEQ ID NO 4) wurde verwendet:
AD-F5-1001-503 (wie im Beispiel 1)

Dieses Oligonukleotid umfasst folgende Modifikaton:
A = 2'-deoxy-Adenosin
C = 2'-deoxy-Cytosin
G = 2'-deoxy-Guanosin
T = 2'-deoxy-Thymidin (Thymidin)

Das 5'-Segment des Oligonukleotids [UAAUCUGUAA GAGCAGAUCC CUGGACAGGC AA GGAAUAC] umfasste 2'-O-Me-Nukleotid-Modifikationen:

### Modifikationen:

A = (2'-O-Methyl-Adenosine)
G = (2'-O-Methyl-Guanosine)
C = (2'-O-Methyl-Cytosine)
U = (2'-O-Methyl-Uridine)
X = 3'-Phosphat-Gruppe zur Blockade einer möglichen Verlängerung durch Polymerase.

Die Nukleotide und Nukleotid-Modifikationen sind untereinander mit Phosphodiesterbindungen verknüpft. Das 3'-Ende des Aktivator-Oligonukleotdies ist mit einer Phosphat-Gruppe blockiert, um eine mögliche Verlängerung durch die Polymerase zu verhindern.

Es wurden vier Ansätze vorbereitet:
Ansatz 1 enthält als Start-Nukleinsäurekette die Matrize M2SF5-M001-200 (Perfect Match Situation) in Konzentration von 300 fmol/l (entspricht ca. 2x10^6 Kopien/ Ansatz).
Ansatz 2 enthält als Start-Nukleinsäurekette die Matrize M2SF5-M001-200 (Perfect Match Situation) in Konzentration von 300 amol/l (entspricht ca. 2x10^3 Kopien/ Ansatz).
Ansatz 3 enthält keine Matrize und bildet somit eine Kontrolle.
Ansatz 4 enthält als Start-Nukleinsäurekette die Matrize M2SF5-WT01-200 (single Mismatch Situation) in 300 pmol/l Konzentration (ca. 2x 10^9 Kopien pro Ansatz).

Primer 1 wurde mit 5 µmol/l, das Aktivator-Oligonukleotid mit 2 µmol/l und Primer 2 mit 1 µmol/l eingesetzt.

Die weiteren Reaktionsbedingungen waren: Amplifikations-Lösung 2.

Um die Anwesenheit von genomischer DNA im Assay nachzustellen wurden pro Reaktion 100 ng frisch denaturierte Fisch-DNA (Lachs-DNA) zugegeben.

Die thermischen Reaktionsbedingungen waren zyklisch, alternierende Temperaturänderungen, wobei jeweils auf ein 2 min Zeitintervall bei 55 °C ein 5 min Zeitintervall bei 65 °C folgte. Die Amplifikation wurde über 100 Zyklen verfolgt. Detektion erfolgte bei 65°C für EvaGreen-Fluoreszenzsignal.

Die erfolgreiche Amplifikation konnte durch einen Anstieg des EvaGreen-Fluoreszenzsignals im Laufe der Zeit festgestellt werden.

Die Temperatur-Änderungen und Real-Time Monitoring wurde mit StepPne Plus Real-Time PCR Gerät von Thermofisher durchgeführt.

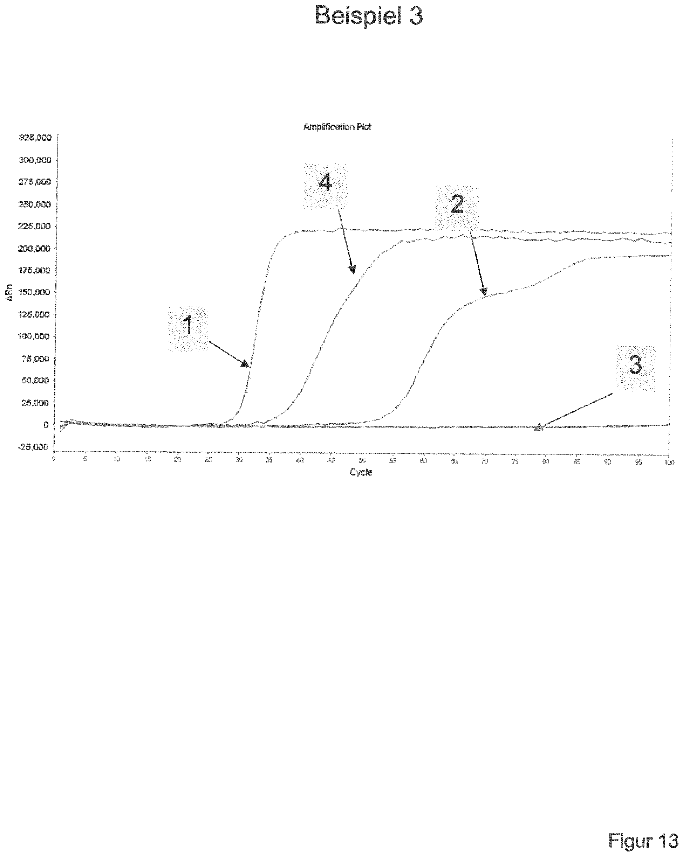

Fig. 13A zeigt einen typischen Verlauf des EvaGreen-Signals. Auf der Y-Achse ist die Zunahme des Fluoreszenzsignal (Delta Rn) und auf der X-Achse ist die Reaktionszeit (als Zyklusnummer) aufgetragen. Pfeile markieren einzelne Reaktionsansätze. Dabei gehören die markierten Positionen zu folgenden Ansätzen:

| | |
|---|---|
| Pfeil 1: 300 fmol/l perfekt-Match Matrize | (ca. 2x10^6 Kopien/Ansatz) |
| Pfeil 2: 300 amol/l perfekt-Match Matrize | (ca. 2x10^3 Kopien/Ansatz) |
| Pfeil 3: keine Matrize | |
| Pfeil 4: 300 pmol/l Mismatch Matrize | (ca. 2x 10^9 Kopien pro Ansatz) |

Man sieht den Anstieg des Fluoreszenz-Signals sowohl bei perfekt-Match als auch bei Mismatch-Variante der Matrize, wobei das Signal der Mismatch-Variante (4) trotz 100fachen Überschußes später erscheint. Es ist erkennbar, dass die Mismatch-Amplifikationssignale gegenüber dem Perfekt-Match Amplifikationssignal deutlich verzögert sind. Beim Single Mismatch wird eine Verzögerung von ca. 15 Zyklen beobachtet. Die zeitliche Verzögerung (= □Zyklusanzahl) ist ein unmittelbares Maß für die Diskriminierung in der Amplifikation. Eine weitere Quantifizierung der Diskriminierung in der Amplifikation kann über den Vergleich mit einer Matrizen-Konzentrationsreihe unter Perfekt-Match Amplifikation erzielt werden.

Bei Verwendung einer Perfect-Match Matrize kommt es zur Synthese eines komplementären Stranges eines Primer-Verlängerungsproduktes. Dieses Verlängerungsprodukt ist sowohl zu Perfect-Match-Matrize komplementär als auch zum verwendeten Aktivator-Oligonukleotid. Diese Konstellation wurde im Beispiel 2 ausführlich dargestellt. Sie dient als Grundlage für eine erfolgreiche Amplifikation.

Im Gegensatz dazu, unter Verwendung einer Mismatch-Sequenz kommt es bei der Synthese des ersten Primer-Verlängerungsproduktes zu Generierung eines komplementären Stranges des Verlängerungsproduktes, welches zwar eine vollständige Komplementarität zu Mismatch-Matrize aufweist, aber dadurch von der Komplementarität mit dem dritten Bereich des Aktivator-Oligonukleotid abweicht. Diese Abweichung findet im 5'-ständigen Segment des Verlängerungsproduktes statt, welches mit Aktivator-Oligonukleotid reagieren sollte, damit Strangverdrängungsvorgang voranschreiten kann. Wie im vorliegenden Beispiel gezeigt wird, stört das Mismatch eine Strangverdrängung durch das Aktivator-Oligonukleotid.

Die Kontrollreaktionen mit einer Perfect Match Matrize (Pfeile 1 und 2) zeigten eine Konzentrationsabhängigkeit der Amplifikation. Mit sinkender Konzentration brauchte Reaktion längere Zeit, um eine ausreichende Menge der zu amplifizierenden Nukleinsäure zu synthetisieren, damit das Signal über Niveau der Baseline ansteigt.

Dieses Ergebnis veranschaulicht die Bedeutung der Basenzusammensetzung im Aktivator-Oligonukleotid: Abweichungen von der Komplementarität zwischen Aktivator-Oligonukleotid und dem Primer-Verlängerungsprodukt können zu Verlangsamung oder sogar Unterbrechung der Amplifikation führen.

In diesem Beispiel wurde gezeigt, dass obwohl Sequenz-Enden von Perfect-Match-Matrize und Mismatch-Matrize komplett übereinstimmten und somit das Potenzial zu Bindung von beiden Primer-Oligonukleotide gleich war, sind beide Reaktionen vollkommen unterschiedlich gelaufen: bei einer vollständigen Komplementarität zwischen dem Aktivator-Oligonukleotid und dem 5'-Segment des Verlängerungsproduktes des ersten Primer-Oligonukleotides verlief die Amplifikation planmäßig. Eine Unterbrechung der Strangverdrängung durch eine Sequenzabweichung (in diesem Fall durch ein Mismatch) führte zur Unterdrückung der Amplifikation.

## Patentansprüche

1. Verfahren zur Amplifikation einer Nukleinsäure, das folgende Schritte umfasst:
a) Hybridisierung eines ersten Primer-Oligonukleotids an das 3'-Segment eines Stranges einer zur amplifizierenden Nukleinsäurekette, wobei die zu amplifizierende Nukleinsäurekette eine Zielsequenz umfasst,
wobei das erste Primer-Oligonukleotid umfasst:
• Einen ersten Primer-Bereich im 3'-Segment des ersten Primer-Oligonukleotides, der an einen Strang einer zu amplifizierenden Nukleinsäurekette sequenzspezifisch binden kann,
• einen zweiten Bereich, gekoppelt direkt oder via einen Linker an das 5'-Ende des ersten Primer-Bereichs des ersten Primer-Oligonukleotides, welcher einen Polynukleotid-Schwanz umfasst, welcher zur Bindung eines Aktivator-Oligonukleotids und Unterstützung der Strangverdrängung (Schritt c) durch Aktivator-Oligonukleotids geeignet ist, wobei der Polynukleotid-Schwanz von Polymerase unter den gewählten Reaktionsbedingungen im Wesentlichen unkopiert bleibt.
b) Extension des ersten Primer-Oligonukleotids mit Hilfe einer Polymerase zur Bildung eines ersten Primer-Verlängerungsproduktes, das eine zur Zielsequenz der zu amplifizierenden Nukleinsäurekette (a) komplementäre Sequenz umfasst,
c) Bindung des Aktivator-Oligonukleotides an den Polynukleotid-Schwanz des zweiten Bereichs des ersten verlängerten Primer-Oligonukleotids, wobei das Aktivator-Oligonukleotid umfasst:
• Einen ersten einzelsträngigen Bereich, welcher an den Polynukleotid-Schwanz des zweiten Bereichs des ersten Primer-Oligonukleotides binden kann,
• einen zweiten einzelsträngigen Bereich, welcher zu dem ersten Bereich des ersten Primer-Oligonukleotides im Wesentlichen komplementär ist und an diesen binden kann,
• einen dritten einzelsträngigen Bereich, welcher zumindest zu einem Segment des von Polymerase synthetisierten Verlängerungsproduktes des ersten Primer-Verlängerungsprodukt im Wesentlichen komplementär ist,
wobei das Aktivator-Oligonukleotid nicht als Matrize für eine Primerverlängerung des ersten Primer-Oligonukleotid dient,
d) Bindung des Aktivator-Oligonukleotides an den ersten Primer-Bereich des ersten verlängerten Primer-Oligonukleotids, unter Verdrängung des zu diesem ersten Primer-Bereich komplementären Stranges der zur amplifizierenden Nukleinsäurekette
e) Bindung des Aktivator-Oligonukleotides an das komplementäre Segment des Verlängerungsproduktes des ersten verlängerten Primer-Oligonukleotids, unter Verdrängung des zu diesem Verlängerungsprodukt komplementären Stranges der zur amplifizierenden Nukleinsäurekette, wobei das 3'-Segment des ersten Primer-Verlängerungsproduktes einzelsträngig wird,
f) Hybridisierung eines zweiten Oligonukleotid-Primers an das erste Primer-Verlängerungsprodukt, wobei das 3'-Segment des zweiten Oligonukleotid-Primers eine Sequenz umfasst, die an das erste Primer-Verlängerungsprodukt hybridisieren kann, und
g) Verlängerung des zweiten Oligonukleotid-Primers mit Polymerase zur Bildung eines zweiten Primer-Verlängerungsproduktes, wobei Verlängerung bis einschließlich des ersten Primer-Bereichs des ersten Primer-Oligonukleotids erfolgt und dieser erste Primer-Bereich von der Polymerase kopiert wird, wobei der Polynukleotid-Schwanz des zweiten Bereichs unkopiert bleibt,
h) Wiederholung der Schritte a)-g) bis das gewünschte Ausmaß der Amplifikation erreicht ist, **dadurch gekennzeichnet, dass** der Reaktionsmischung ein Detektionssystem zugegeben wird.

2. Verfahren nach Anspruch 1, **dadurch gekennzeichnet, dass** das Detektionssystem ein Fluoreszenzreportermolekül und ein damit über eine Nukleotidsequenz verbundenes Signal-Quenchermolekül aufweist, wobei die Nukleotidsequenz komplementär ist zu einem Teil der Zielsequenz.

3. Verfahren nach Anspruch 1, **dadurch gekennzeichnet, dass** das Detektionssystem ein Fluoreszenzreportermolekül aufweist, das gebunden ist an eine Komponente, die im Verfahren eingesetzt wird, und dass ein Fluoreszenz-Donor oder ein Fluoreszenz-Quencher eingesetzt wird, verbunden mit einer anderen, in dem Verfahren verwendeten Komponente, wobei eine räumliche Nähe oder räumliche Abwesenheit in Abwesenheit bzw. Anwesenheit der nachzuweisenden Zielsequenz bewirkt wird.

4. Verfahren nach einem der Ansprüche 1-3, **dadurch gekennzeichnet, dass** bei dem Detektionssystem ein Fluoreszenz-Resonanz-Energie-Transfer vorliegt.

5. Verfahren nach Anspruch 1, **dadurch gekennzeichnet, dass** das Verfahren im Wesentlichen isothermal durchgeführt wird.

6. Verfahren nach einem der Ansprüche 1 oder 2, **dadurch gekennzeichnet, dass** der dritte einzelsträngige Bereich des Aktivator-Oligonukleotids zum Segment des von der Polymerase synthetisierten Verlängerungsproduktes des ersten Primer-Verlängerungsprodukts, welches sich unmittelbar an den ersten Primer-Bereich anschließt, im Wesentlichen komplementär ist.

7. Verfahren nach Anspruch 1, **dadurch gekennzeichnet, dass** Schritt (e) des Verfahrens dahingehend modifiziert wird, dass es umfasst die Bindung des Aktivator-Oligonukleotides an das komplementäre Segment des Verlängerungsproduktes des ersten verlängerten Primer-Oligonukleotids, unter Verdrängung des zu diesem Verlängerungsprodukt komplementären Stranges der zur amplifizierenden Nukleinsäurekette, bis dieser komplementäre Strang der zu amplifizierenden Nukleinsäure vom ersten Primer-Verlängerungsprodukt abgelöst wird, wobei das 3'-Segment des ersten Primer-Verlängerungsproduktes einzelsträngig wird.

8. Verfahren nach Anspruch 1, **dadurch gekennzeichnet, dass** Schritt (f) des Verfahrens dahingehend modifiziert wird, dass es umfasst die Hybridisierung eines zweiten Oligonukleotid-Primers an das erste Primer-Verlängerungsprodukt, wobei gleichzeitig zumindest eine partielle Verdrängung des Aktivator-Oligonukleotides aus der Bindung mit dem ersten Verlängerungsprodukt durch Strangverdrängung erfolgt.

9. Verfahren nach Anspruch 1, **dadurch gekennzeichnet, dass** Schritt (g) des Verfahrens so modifiziert wird, dass er eine Verdrängung des Aktivator-Oligonukleotides aus der Bindung mit dem ersten Primer-Verlängerungsprodukt unter Mitwirkung der Polymerase umfasst.

10. Verfahren nach Anspruch 1, **dadurch gekennzeichnet, dass** Schritt (h) des Verfahrens dahingehend modifiziert wird, dass es die Bindung des Aktivator-Oligonukleotides an den unkopierten Polynukleotid-Schwanz des ersten verlängerten Primer-Oligonukleotids und eine Verdrängung des zweiten Primer-Verlängerungsprodukts aus der Bindung an das erste Primer-Verlängerungsprodukt unter gleichzeitiger Ausbildung eines komplementären Doppelstranges mit einem Segment des ersten spezifischen Verlängerungsproduktes des ersten Primer-Oligonukleotides umfasst.

11. Verfahren nach Anspruch 1, **dadurch gekennzeichnet, dass** die Wiederholung der Schritte unter solchen Bedingungen durchgeführt wird, die die Wiederholung der Schritte (a) bis (g) zulassen.

12. Verfahren nach einem der vorhergehenden Ansprüche, **dadurch gekennzeichnet, dass** es die gleichzeitige Amplifikation des ersten und zweiten Primer-Verlängerungsproduktes in einer exponentiellen Reaktion unter Verwendung der ersten und zweiten Primer-Oligonukleotide und des Aktivator-Oligonukleotides umfasst, wobei die gebildeten Primer-Verlängerungsprodukte als Matrizen für die gegenseitige Synthese fungieren.

13. Verwendung eines Kits zur Durchführung eines Verfahrens nach einem der Ansprüche 1 bis 11, **dadurch gekennzeichnet, dass** der Kit wenigstens ein erstes Primer-Oligonukleotid, wenigstens ein Aktivator-Oligonukleotid und wenigstens eine Polymerase zur Amplifikation und ein Detektionssystem enthält.

14. Verwendung eines Kits nach Anspruch 13, **dadurch gekennzeichnet, dass** es weiterhin einen zweiten Oligonukleotid-Primer enthält.

## Claims

1. A method for amplification of a nucleic acid comprising the following steps:
a) hybridizing a first primer oligonucleotide to the 3' segment of a strand of a nucleic acid chain to be amplified, wherein the nucleic acid chain to be amplified comprises a target sequence,
wherein the first primer oligonucleotide comprises:
• a first primer region in the 3' segment of the first primer oligonucleotide that can sequence-specifically bind to a strand of a nucleic acid chain to be amplified,
• a second region that is directly or via a linker linked to the 5' end of the first primer region of the first primer oligonucleotide and that comprises a polynucleotide tail which is suitable for binding an activator oligonucleotide and supporting strand displacement (step c) by the activator oligonucleotide, wherein the polynucleotide tail remains substantially uncopied by polymerase under the selected reaction conditions,
b) extending the first primer oligonucleotide by means of a polymerase to form a first primer extension product comprising a sequence that is complementary to the target sequence of the nucleic acid chain (a) to be amplified,
c) binding the activator oligonucleotide to the polynucleotide tail of the second region of the first extended primer oligonucleotide, wherein the activator oligonucleotide comprises:
• a first single-stranded region that can bind to the polynucleotide tail of the second region of the first primer oligonucleotide,
• a second single-stranded region that is substantially complementary and can bind to the first region of the first primer oligonucleotide,
• a third single-stranded region that is substantially complementary to at least a segment of the extension product, which has been synthesized by polymerase, of the first primer extension product,
wherein the activator oligonucleotide does not serve as template for a primer extension of the first primer oligonucleotide,
d) binding the activator oligonucleotide to the first primer region of the first extended primer oligonucleotide by displacing the strand of the nucleic acid chain to be amplified that is complementary to said first primer region,
e) binding the activator oligonucleotide to the complementary segment of the extension product of the first extended primer oligonucleotide by displacing the strand of the nucleic acid chain to be amplified that is complementary to said extension product, wherein the 3' segment of the first primer extension product becomes single-stranded,
f) hybridizing a second oligonucleotide primer to the first primer extension product, wherein the 3' segment of the second oligonucleotide primer comprises a sequence that can hybridize to the first primer extension product; and
g) extending the second oligonucleotide primer with polymerase to form a second primer extension product, wherein the extension takes place up to and including the first primer region of the first primer oligonucleotide and said first primer region is copied by the polymerase, wherein the polynucleotide tail of the second region remains uncopied,
h) repeating steps a)-g) until the desired degree of amplification has been achieved, **characterized in that** a detection system is added to the reaction mixture.

2. The method according to claim 1, **characterized in that** the detection system has a fluorescence reporter molecule and a signal quencher molecule linked therewith via a nucleotide sequence, wherein the nucleotide sequence is complementary to a part of the target sequence.

3. The method according to claim 1, **characterized in that** the detection system has a fluorescence reporter molecule which is linked to a component used in the method, and that a fluorescence donor or a fluorescence quencher is used, linked to another component used in the method, wherein a spatial proximity or spatial absence is effected in the presence or absence, respectively, of the target sequence to be detected.

4. The method according to any one of claims 1-3, **characterized in that** a fluorescence resonance energy transfer is present in the detection system.

5. The method according to claim 1, **characterized in that** the method is carried out substantially isothermal.

6. The method according to any of claims 1 or 2, **characterized in that** the third single-stranded region of the activator oligonucleotide is substantially complementary to the segment of the extension product, which has been synthesized by the polymerase, of the first primer extension product, which immediately follows the first primer region.

7. The method according to claim 1, **characterized in that** step (e) of the method is modified **in that** it comprises the binding of the activator oligonucleotide to the complementary segment of the extension product of the first extended primer oligonucleotide by displacing the strand of the nucleic acid chain to be amplified that is complementary to said extension product until said complementary strand of the nucleic acid to be amplified is detached from the first primer extension product, wherein the 3' segment of the first primer extension product becomes single-stranded.

8. The method according to claim 1, **characterized in that** step (f) of the method is modified **in that** it comprises the hybridization of a second oligonucleotide primer to the first primer extension product, wherein at the same time there is at least a partial displacement of the activator oligonucleotide from the binding with the first extension product by strand displacement.

9. The method according to claim 1, **characterized in that** step (g) of the method is modified such that it comprises a displacement of the activator oligonucleotide from the binding with the first primer extension product with the participation of the polymerase.

10. The method according to claim 1, **characterized in that** step (h) of the method is modified **in that** it comprises the binding of the activator oligonucleotide to the uncopied polynucleotide tail of the first extended primer oligonucleotide and a displacement of the second primer extension product from the binding to the first primer extension product with the simultaneous formation of a complementary double strand with a segment of the first specific extension product of the first primer oligonucleotide.

11. The method according to claim 1, **characterized in that** the repetition of the steps is performed under such conditions that allow the repetition of the steps (a) to (g).

12. The method according to any of the preceding claims, **characterized in that** it comprises the simultaneous amplification of the first and second primer extension products in an exponential reaction by using the first and second primer oligonucleotides and the activator oligonucleotide, wherein the formed primer extension products function as a template for the mutual synthesis.

13. Use of a kit for carrying out a method according to any of claims 1 to 11, **characterized in that** the kit contains at least one first primer oligonucleotide, at least one activator oligonucleotide, and at least one polymerase for amplification and a detection system.

14. Use of a kit according to claim 13, **characterized in that** it further contains a second oligonucleotide primer.

## Revendications

1. Procédé d'amplification d'un acide nucléique, qui comprend des étapes suivantes :
a) hybridation d'un premier oligonucléotide d'amorce au niveau du segment 3' d'un brin d'une chaîne d'acide nucléique à amplifier, dans lequel la chaîne d'acide nucléique à amplifier comprend une séquence cible,
dans lequel le premier oligonucléotide d'amorce comprend :
• une première région d'amorce dans le segment 3' du premier oligonucléotide d'amorce, qui peut se lier de façon spécifique à une séquence, à un brin d'une chaîne d'acide nucléique à amplifier,
• une deuxième région, couplée directement ou via un segment de liaison à l'extrémité 5' de la première région d'amorce du premier oligonucléotide d'amorce, qui comprend une queue de polynucléotide qui est adaptée pour se lier à un oligonucléotide activateur et pour soutenir le déplacement de brin (étape c) par l'oligonucléotide activateur, dans lequel la queue de polynucléotide de la polymérase reste sensiblement non copiée dans les conditions réactionnelles sélectionnées,
b) extension du premier oligonucléotide d'amorce à l'aide d'une polymérase pour former un premier produit de prolongement d'amorce qui comprend une séquence complémentaire à la séquence cible de la chaîne d'acide nucléique à amplifier (a),
c) liaison de l'oligonucléotide activateur à la queue de polynucléotide de la deuxième région du premier oligonucléotide d'amorce prolongé, dans lequel l'oligonucléotide activateur comprend :
• une première région à brin simple, qui peut se lier à la queue de polynucléotide de la deuxième région du premier oligonucléotide d'amorce,
• une deuxième région à brin simple, qui est sensiblement complémentaire à la première région du premier oligonucléotide d'amorce et peut se lier à celle-ci,
• une troisième région à brin simple, qui est au moins sensiblement complémentaire à un segment du produit de prolongement synthétisé par la polymérase, du premier produit de prolongement d'amorce,
dans lequel l'oligonucléotide activateur ne sert pas de matrice pour un prolongement d'amorce du premier oligonucléotide d'amorce,
d) liaison de l'oligonucléotide activateur à la première région d'amorce du premier oligonucléotide d'amorce prolongé, en déplaçant le brin complémentaire de cette première région d'amorce de la chaîne d'acide nucléique à amplifier
e) liaison de l'oligonucléotide activateur au segment complémentaire du produit de prolongement du premier oligonucléotide d'amorce prolongé, en déplaçant le brin complémentaire de ce produit de prolongement de la chaîne d'acide nucléique à amplifier, dans lequel le segment 3' du premier produit de prolongement d'amorce est à brin simple,
f) hybridation d'une seconde amorce d'oligonucléotide au premier produit de prolongement d'amorce, dans lequel le segment 3' de la seconde amorce d'oligonucléotide comprend une séquence qui peut s'hybrider au premier produit de prolongement d'amorce, et
g) prolongement de la seconde amorce d'oligonucléotide avec une polymérase pour former un second produit de prolongement d'amorce, dans lequel un prolongement s'effectue de façon inclusive jusqu'à la première région d'amorce du premier oligonucléotide d'amorce et cette première région d'amorce est copiée par la polymérase, dans lequel la queue de polynucléotide de la deuxième région reste non copiée,
h) répétition des étapes a) à g) jusqu'à l'obtention de l'ampleur de l'amplification souhaitée, **caractérisé en ce qu'**un système de détection est ajouté au mélange réactionnel.

2. Procédé selon la revendication 1, **caractérisé en ce que** le système de détection présente une molécule rapporteur de fluorescence et une molécule d'inactivation de signal liée par une séquence nucléotidique, dans lequel la séquence nucléotidique est complémentaire à une partie de la séquence cible.

3. Procédé selon la revendication 1, **caractérisé en ce que** le système de détection présente une molécule rapporteur de fluorescence qui est liée à un composant qui est utilisé dans le procédé, et **en ce qu'**un donneur de fluorescence ou un inactivateur de fluorescence est utilisé, lié à un autre composant utilisé dans le procédé, dans lequel une proximité spatiale ou une absence spatiale est provoquée en l'absence ou en présence de la séquence cible à détecter.

4. Procédé selon l'une quelconque des revendications 1 à 3, **caractérisé en ce qu'**il existe un transfert de fluorescence-résonance-énergie dans le système de détection.

5. Procédé selon la revendication 1, **caractérisé en ce que** le procédé est exécuté de façon sensiblement isothermique.

6. Procédé selon l'une quelconque des revendications 1 ou 2, **caractérisé en ce que** la troisième région à brin simple de l'oligonucléotide activateur est sensiblement complémentaire au segment du produit de prolongement synthétisé par la polymérase, du premier produit de prolongement d'amorce, lequel se raccorde directement à la première région d'amorce.

7. Procédé selon la revendication 1, **caractérisé en ce que** l'étape (e) du procédé est modifiée de sorte qu'il comprend la liaison de l'oligonucléotide activateur au segment complémentaire du produit de prolongement du premier oligonucléotide d'amorce prolongé, en déplaçant le brin complémentaire de ce produit de prolongement de la chaîne d'acide nucléique à amplifier, jusqu'à ce que ce brin complémentaire de la chaîne d'acide nucléique à amplifier se détache du premier produit de prolongement d'amorce, dans lequel le segment 3' du premier produit de prolongement d'amorce est à brin simple.

8. Procédé selon la revendication 1, **caractérisé en ce que** l'étape (f) du procédé est modifiée de sorte qu'il comprend l'hybridation d'une seconde amorce d'oligonucléotide au premier produit de prolongement d'amorce, dans lequel s'effectue en même temps au moins un déplacement partiel de l'oligonucléotide activateur hors de la liaison avec le premier produit de prolongement par déplacement de brin.

9. Procédé selon la revendication 1, **caractérisé en ce que** l'étape (g) du procédé est modifiée de sorte qu'elle comprend un déplacement de l'oligonucléotide activateur hors de la liaison avec le premier produit de prolongement d'amorce sous l'effet de la polymérase.

10. Procédé selon la revendication 1, **caractérisé en ce que** l'étape (h) du procédé est modifiée de sorte qu'il comprend la liaison de l'oligonucléotide d'amorce à la queue de polynucléotide non copiée du premier oligonucléotide d'amorce prolongé et un déplacement du second produit de prolongement d'amorce hors de la liaison au premier produit de prolongement d'amorce en réalisant en même temps un brin double complémentaire avec un segment du premier produit de prolongement spécifique du premier oligonucléotide d'amorce.

11. Procédé selon la revendication 1, **caractérisé en ce que** la répétition des étapes est exécutée dans des conditions telles que la répétition des étapes (a) à (g) est autorisée.

12. Procédé selon l'une quelconque des revendications précédentes, **caractérisé en ce qu'**il comprend l'amplification concomitante des premier et second produits de prolongement d'amorce dans une réaction exponentielle en utilisant les premier et second oligonucléotides d'amorce et l'oligonucléotide activateur, dans lequel les produits de prolongement d'amorce formés font office de matrices pour la synthèse réciproque.

13. Utilisation d'un kit pour l'exécutiond'un procédé selon l'une quelconque des revendications 1 à 11, **caractérisée en ce que** le kit contient au moins un premier oligonucléotide d'amorce, au moins un oligonucléotide activateur et au moins une polymérase pour l'amplification et un système de détection.

14. Utilisation d'un kit selon la revendication 13, **caractérisé en ce qu'**il contient en outre une seconde amorce d'oligonucléotide.
